(19) Europäisches Patentamt
European Patent Office
Office européen des brevets

(11) **EP 2 978 748 B1**

(12) # EUROPEAN PATENT SPECIFICATION

(45) Date of publication and mention of the grant of the patent:
**10.05.2017 Bulletin 2017/19**

(21) Application number: **14717080.7**

(22) Date of filing: **25.03.2014**

(51) Int Cl.:
***C07D 307/60*** $^{(2006.01)}$ ***C07B 53/00*** $^{(2006.01)}$
***C07C 59/74*** $^{(2006.01)}$ ***C07C 229/04*** $^{(2006.01)}$

(86) International application number:
**PCT/IB2014/060140**

(87) International publication number:
**WO 2014/155291 (02.10.2014 Gazette 2014/40)**

(54) **PROCESS AND INTERMEDIATES FOR THE PREPARATION OF PREGABALIN**

VERFAHREN UND ZWISCHENPRODUKTE FÜR DIE HERSTELLUNG VON PREGABALIN

PROCESSUS ET INTERMÉDIAIRES POUR LA PRÉPARATION DE PRÉGABALINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.03.2013 US 201361805786 P**

(43) Date of publication of application:
**03.02.2016 Bulletin 2016/05**

(60) Divisional application:
**17162642.7**

(73) Proprietor: **PFIZER IRELAND PHARMACEUTICALS**
**Ringaskiddy**
**Cork (IE)**

(72) Inventors:
- **DEBARGE, Sebastien**
**Ringaskiddy**
**County Cork (IE)**
- **ERDMAN, David Thomas**
**Kalamazoo, Michigan 49001 (US)**
- **O'NEILL, Padraig Mary**
**Ringaskiddy**
**County Cork (IE)**
- **KUMAR, Rajesh**
**Groton, Connecticut 06340 (US)**
- **KARMILOWICZ, Michael John**
**Groton, Connecticut 06340 (US)**

(74) Representative: **Pfizer**
**European Patent Department**
**23-25 avenue du Docteur Lannelongue**
**75668 Paris Cedex 14 (FR)**

(56) References cited:
**WO-A1-03/093220** **WO-A1-2011/086565**
**WO-A2-2008/127646** **WO-A2-2010/081053**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the Invention

The present invention relates to the manufacture of 5-hydroxy-4-(2-methylpropyl)-3,4-dihydro-5*H*-2-furanone, and derivatives thereof, for use in the manufacture of (S)-(+)-3-aminomethyl-5-methylhexanoic acid (Pregabalin). The invention further relates to an improved process for the conversion of 5-hydroxy-4-(2-methylpropyl)-3,4-dihydro-5*H*-2-furanone into Pregabalin. Pregabalin is a γ-amino acid that exhibits binding affinity to the human $\alpha_2\delta$ calcium channel subunit.

### Background to the Invention

Pregabalin, or (*S*)-(+)-3-aminomethyl-5-methyl-hexanoic acid ((*S*)-**II**),

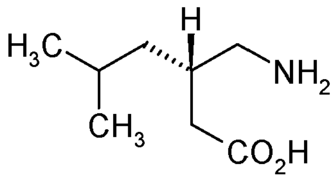

Pregabalin ((*S*)-**II**)

is the active agent in Lyrica®, which is approved for the treatment of epilepsy, neuropathic pain, fibromyalgia and generalized anxiety disorder. It exhibits anti-seizure activity, as discussed in U.S. Patent US 5,563,175, and anti-nociceptive activity, as discussed in U.S. Patent US 6,001,876. It is hypothesised that the pharmacological activity of Pregabalin (**II**) is the result of binding to the alpha-2-delta ($\alpha_2\delta$) subunit of a calcium channel. Pregabalin (**II**) is also described as having utility in other conditions, such as physiological conditions associated with psychomotor stimulants, inflammation, gastrointestinal damage, alcoholism, insomnia, and various psychiatric disorders, including anxiety, depression, mania, and bipolar disorder.

A number of methods for manufacturing Pregabalin have been disclosed. 5-Hydroxy-4-(2-methylpropyl)-3,4-dihydro-5*H*-2-furanone (**I**[A]) has been identified as a useful precursor.

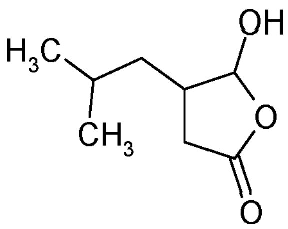

(**I**[A])

It will be appreciated that compound (**I**[A]), in common with a number of the other compounds discussed herein, can be considered to be the cyclized isomer of a 4-oxobutanoic acid derivative. Derivatives of 4-oxobutanoic acid can exist as either the open-chain form, or as the cyclic form.

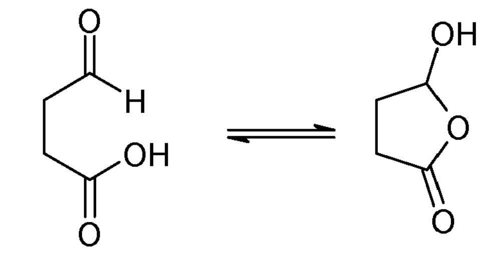

"Open chain" isomer

"Cyclic" isomer

These two isomeric forms may co-exist in equilibrium, and the relative contributions of the two forms will depend on the precise chemical nature of the species.WO03/093220 discloses a synthesis for the manufacture of Pregabalin starting from mucochloric or mucobromic acid. International Patent Application PCT/US2008/004699 (published as WO2008/127646A2) proposes the conversion of 5-hydroxy-4-(2-methylpropyl)-3,4-dihydro-5*H*-2-furanone (**I$^A$**) or an ester of the ring-opened isomeric form (**XIII**, wherein $R^A$ is alkyl) to (**II**) using a chemical or an enzyme-mediated reductive amination. It is suggested that the use of a transaminase enzyme will provide selectively the preferred ((*S*)-II) enantiomer.

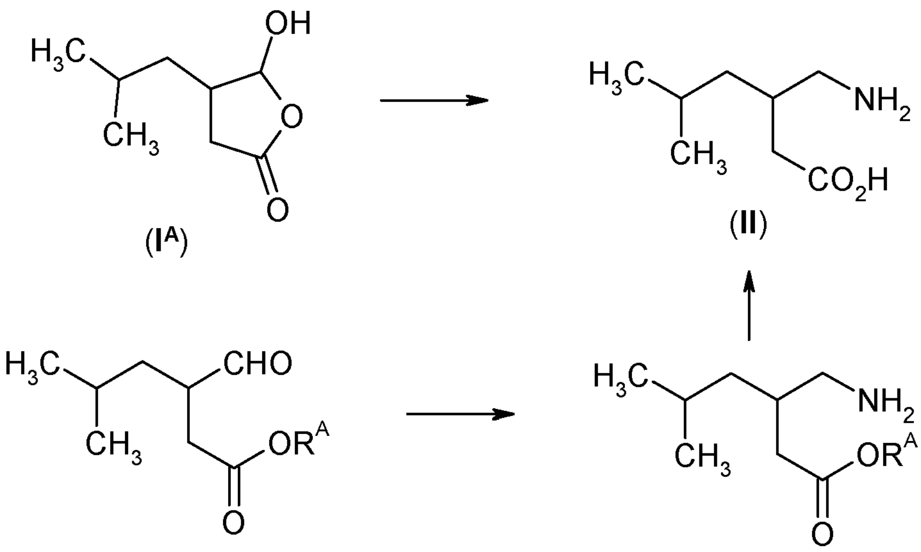

(**I$^A$**)

(**II**)

(**XIII**)

The ester (**XIII**) is prepared from 4-methylpentanal (**III**) by alkylation with an appropriate haloacetate in the presence of diisobutylamine. The precursor (**I$^A$**) is made either by ester hydrolysis of ester (**XIII**) or by condensation of 4-methylpentanal (**III**) with glyoxylic acid (**IV**) and subsequent reduction of the double bond. The use of an enzyme-mediated reduction is also suggested as a way of introducing the desired stereochemistry.

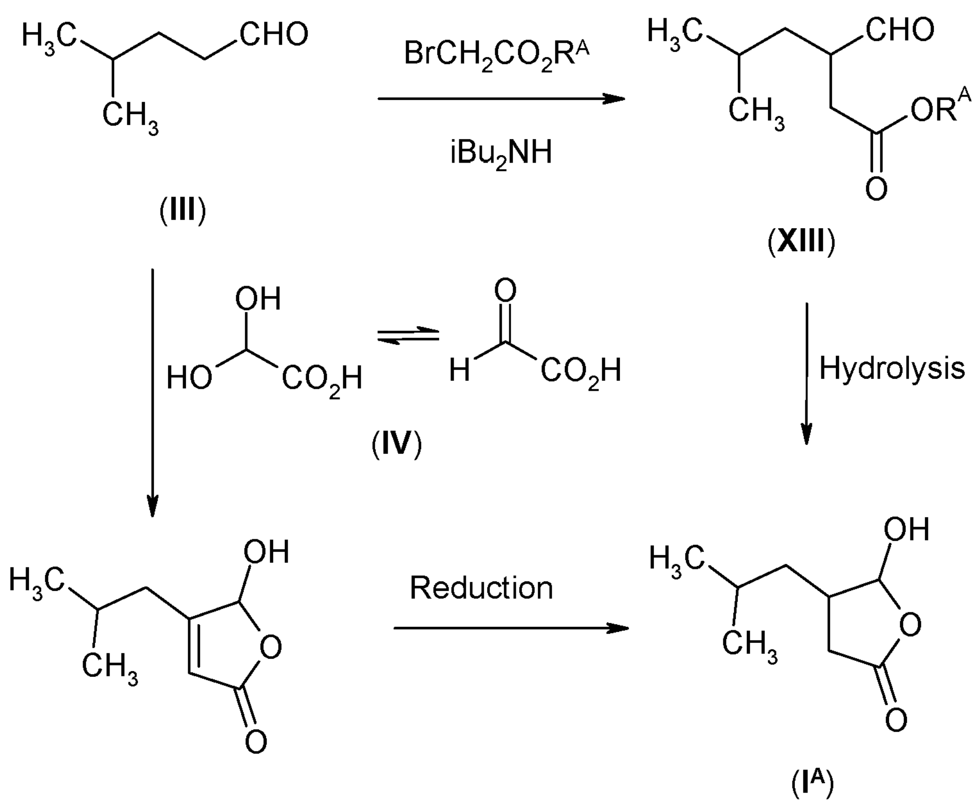


(III) (XIII) (IV) (I^A)

International Patent Application PCT/IN2010/000140 (published as WO2011/086565) discloses a related process. The condensation product of 4-methylpentanal (**III**) and glyoxylic acid **(IV)** is reacted with a chiral amine such as α-methylbenzylamine to give a pyrrolone (V). Hydrogenation gives some degree of stereoselectivity, and deprotection gives Pregabalin (**II**) in chiral form.

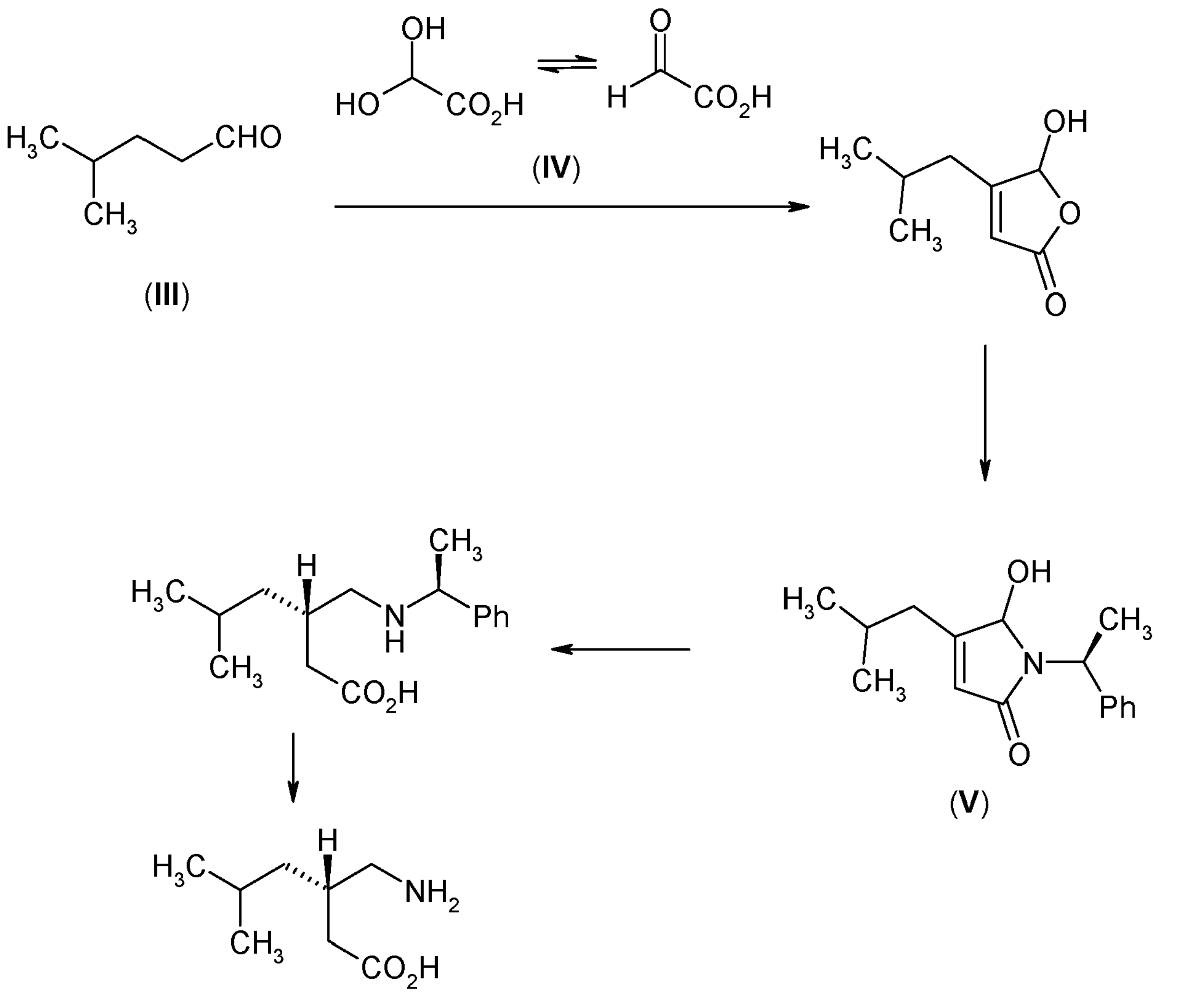
(**III**) (**IV**) (**V**)

((*S*)-**II**)

Still further improved syntheses of Pregabalin (**II**) are sought. It is especially desirable to provide a process which is cost effective and safe. In particular, it is important to provide a synthesis of Pregabalin (II) which can be carried out on a commercial scale, which uses readily available, inexpensive and safe starting materials and reagents, and which avoids the need for difficult separations.

<u>Summary of the Invention</u>

The present invention provides improved methods for the manufacture of 5-hydroxy-4-(2-methylpropyl)-3,4-dihydro-5*H*-2-furanone ($\mathbf{I}^{\mathbf{A}}$), and intermediates that are useful in these improved methods.

In a first aspect **A1,** the invention provides a compound according to formula **(VI)**

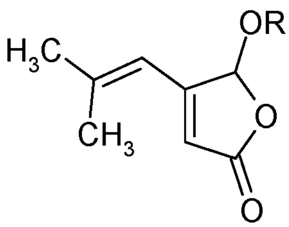

(**VI**)

In a first embodiment **A1 E1,** the invention provides a compound according to formula (**VI**) wherein R is selected from:

hydrogen,
$(C_1-C_6)$alkyl,
$(C_1-C_6)$haloalkyl,
$(C_1-C_3)$alkoxy$(C_2-C_6)$alkyl,
$(C_2-C_6)$alkenyl,
$(C_3-C_{10})$cycloalkyl, which may optionally be substituted with 1, 2 or 3 groups independently selected from halo, $(C_1-C_3)$alkyl, and $(C_1-C_3)$alkyloxy,
$(C_3-C_{10})$cycloalkyl$(C_1-C_6)$alkyl, wherein the cycloalkyl group may optionally be substituted with 1, 2 or 3 groups independently selected from halo, $(C_1-C_3)$alkyl, and $(C_1-C_3)$alkyloxy,
aryl, which may optionally be substituted with 1, 2 or 3 groups independently selected from halo, $(C_1-C_3)$alkyl, and $(C_1-C_3)$alkyloxy,
aryl$(C_1-C_6)$alkyl, wherein the aryl group may optionally be substituted with 1, 2 or 3 groups independently selected from halo, $(C_1-C_3)$alkyl, and $(C_1-C_3)$alkyloxy,
$R^1$-C(O)-, and
$R^2$-$SO_2$-;

$R^1$ is selected from:

hydrogen,
$(C_1-C_6)$alkyl,
$(C_1-C_6)$haloalkyl,
$(C_1-C_3)$alkoxy$(C_2-C_6)$alkyl,
$(C_2-C_6)$alkenyl,
$(C_3-C_{10})$cycloalkyl, which may optionally be substituted with 1, 2 or 3 groups independently selected from halo, $(C_1-C_3)$alkyl, and $(C_1-C_3)$alkyloxy,
$(C_3-C_{10})$cycloalkyl$(C_1-C_6)$alkyl, wherein the cycloalkyl group may optionally be substituted with 1, 2 or 3 groups independently selected from halo, $(C_1-C_3)$alkyl, and $(C_1-C_3)$alkyloxy,
aryl, which may optionally be substituted with 1, 2 or 3 groups independently selected from halo, $(C_1-C_3)$alkyl, and $(C_1-C_3)$alkyloxy, and
aryl$(C_1-C_6)$alkyl, wherein the aryl group may optionally be substituted with 1, 2 or 3 groups independently selected from halo, $(C_1-C_3)$alkyl, and $(C_1-C_3)$alkyloxy,

and $R^2$ is selected from:

$(C_1-C_6)$alkyl,
$(C_1-C_6)$haloalkyl,
$(C_1-C_3)$alkoxy$(C_2-C_6)$alkyl,
$(C_2-C_6)$alkenyl,
$(C_3-C_{10})$cycloalkyl, which may optionally be substituted with 1, 2 or 3 groups independently selected from halo, $(C_1-C_3)$alkyl, and $(C_1-C_3)$alkyloxy,
$(C_3-C_{10})$cycloalkyl$(C_1-C_6)$alkyl, wherein the cycloalkyl group may optionally be substituted with 1, 2 or 3 groups independently selected from halo, $(C_1-C_3)$alkyl, and $(C_1-C_3)$alkyloxy,
aryl, which may optionally be substituted with 1, 2 or 3 groups independently selected from halo, $(C_1-C_3)$alkyl, and $(C_1-C_3)$alkyloxy, and
aryl$(C_1-C_6)$alkyl, wherein the aryl group may optionally be substituted with 1, 2 or 3 groups independently selected from halo, $(C_1-C_3)$alkyl, and $(C_1-C_3)$alkyloxy.

In a further embodiment **A1E2**, the invention provides a compound according to embodiment **A1 E1** wherein R is hydrogen such that the compound of formula **(VI)** is 5-hydroxy-4-(2-methyl-1-propenyl)-5*H*-2-furanone according to formula **(VIA).**

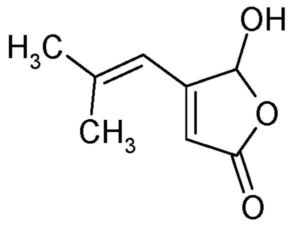

**(VI^A)**

In a further embodiment **A1E3,** the invention provides a compound according to embodiment **A1 E1** of formula **(VI^B)**

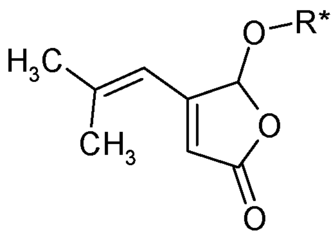

**(VI^B)**

wherein R* is a chiral ($C_5$-$C_{15}$) hydrocarbon group.

In a further embodiment **A1E4,** the invention provides a compound according to embodiment **A1E3** wherein R* is selected from (*R*)- or (*S*)-α-methylbenzyl, (*R*)- or (*S*)-1-(1-naphthyl)ethyl, (*R*)- or (*S*)-1-(2-naphthyl)ethyl, menthyl and bornyl, such that the compound of formula (**VI**) is the compound of formula (**VI^C**) - (**VI^K**).

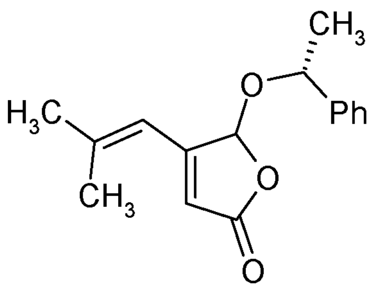

(**VI^C**)

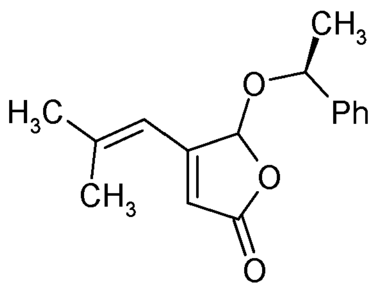

(**VI^D**)

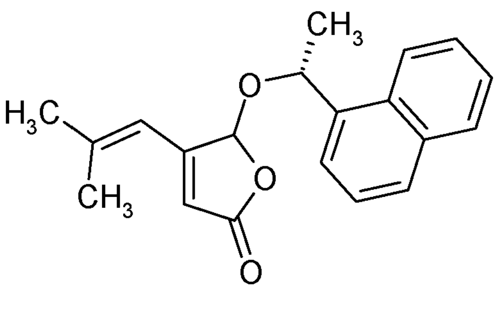

(**VI^E**)

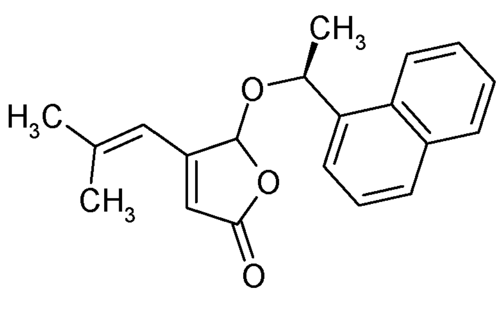

(**VI^F**)

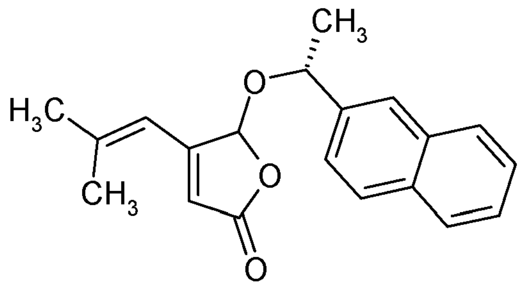

($VI^G$)

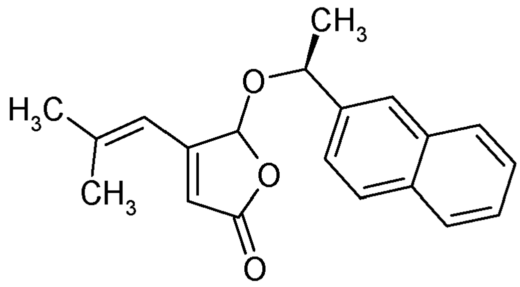

($VI^H$)

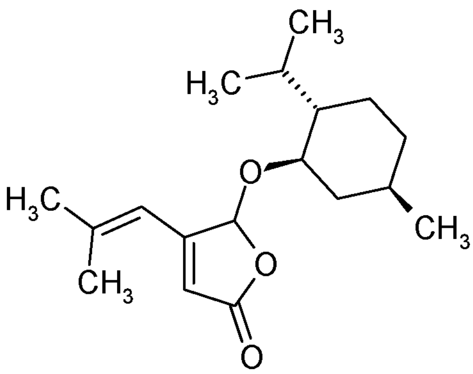

($VI^J$)

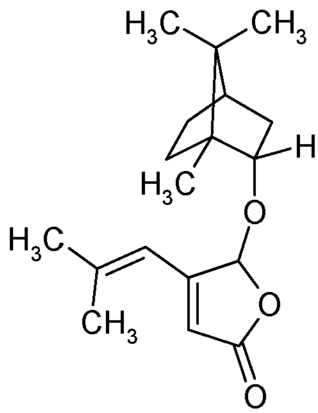

($VI^K$)

In a further embodiment **A1E5,** the invention provides a compound according to embodiment **A1 E1** wherein R is $R^1$-C(O)- or $R^2$-$SO_2$- and $R^1$and $R^2$ are chiral ($C_5$-$C_{15}$) hydrocarbon groups.

In another aspect A2, the invention provides a compound of formula **(IX)**

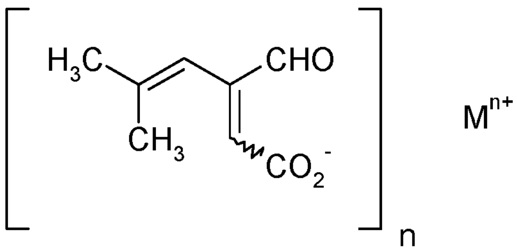

(**IX**)

The compound of formula (**IX**) may exist as either the (*E*)- or (*Z*)-geometric isomer, or as a mixture of the two geometric isomers.

In a first embodiment **A2E1,** the invention provides a compound of formula (**IX**) wherein: n is 1 and $M^+$ is selected from $Li^+$, $Na^+$, $K^+$, $Rb^+$, $NH_4^+$, (($C_1$-$C_3$)alkyl)$NH_3^+$, (($C_1$-$C_3$)alkyl)$_2NH_2^+$, (($C_1$-$C_3$)alkyl)$_3NH^+$ and (($C_1$-$C_3$)alkyl)$_4N^+$; or n is 2 and $M^{2+}$ is selected from $Mg^{2+}$, $Ca^{2+}$ and $Zn^{2+}$.

In a further embodiment **A2E2,** the invention provides a compound according to embodiment **A2E1** wherein n is 1 and $M^+$ is selected from $NH_4^+$ and (($C_1$-$C_3$)alkyl)$NH_3^+$. In a further embodiment **A2E3,** the invention provides a compound according to embodiment **A2E1** wherein n is 1 and $M^+$ is selected from $Li^+$, $Na^+$ and $K^+$.

In another aspect **A3,** the invention provides a compound of formula (**VII**).

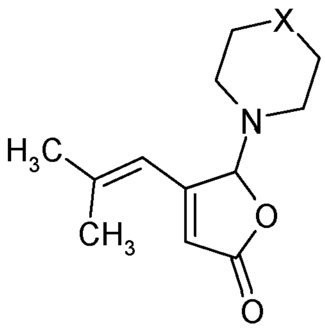

(**VII**)

In a first embodiment **A3E1,** the invention provides a compound of formula (**VII**) wherein -X- represents a single bond, $-CH_2-$, -O-; -NH-,$-N((C_1-C_3)alkyl)-$, -N(benzyl)-, or

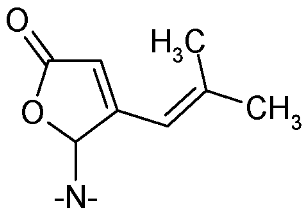

In a further embodiment **A3E2,** the invention provides a compound according to embodiment **A3E1** selected from:

4-(2-methylpropenyl)-5-pyrrolidin-1-yl-5*H*-furan-2-one;
4-(2-methylpropenyl)-5-piperidin-1-yl-5*H*-furan-2-one;
4-(2-methylpropenyl)-5-morpholin-4-yl-5*H*-furan-2-one; and
1,4-*bis*-(4-(2-methylpropenyl)-5*H*-furan-2-on-5-yl)piperazine.

In another aspect **A4,** also disclosed herein is a compound of formula (**VIII**).

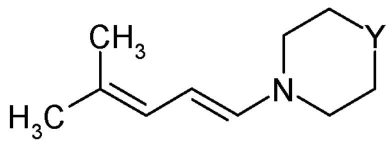

(**VIII**)

9

In a first embodiment **A4E1,** a compound of formula (**VIII**) wherein -Y- represents a single bond, $-CH_2-$, -O-; -NH-, $-N((C_1-C_3)alkyl)-$, -N(benzyl)-, or

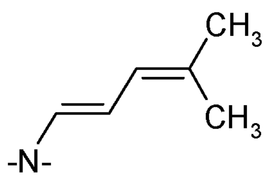

is disclosed. In a further embodiment **A4E2,** disclosed herein is a compound according to embodiment **A4E1** selected from:

4-(4-methyl-1,3-pentadien-1-yl)morpholine;

1-(4-methyl-1,3-pentadien-1-yl)-piperazine,
1-(4-methyl-1,3-pentadien-1-yl)-4-methylpiperazine,
4-ethyl-1-(4-methyl-1,3-pentadien-1-yl)-piperazine,
4-benzyl-1-(4-methyl-1,3-pentadien-1-yl)-piperazine, and
1,4-*bis*-(4-methyl-1,3-pentadien-1-yl)piperazine.

In another aspect **A5,** the invention provides a process for the manufacture of the compound of formula **(VIA)** comprising the step of treating a compound of formula (**VII**) with water in the presence of an acid catalyst.

In a first embodiment **A5E1,** the invention provides a process for the manufacture of the compound of formula **(VIA),** comprising the steps of:

(a) preparing a compound of formula (**VII**)

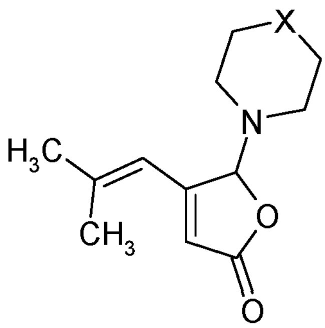

(**VII**)

wherein -X- represents a single bond, $-CH_2-$, -O-, -NH-, $-N((C_1-C_3)alkyl)-$, -N(benzyl)-, or

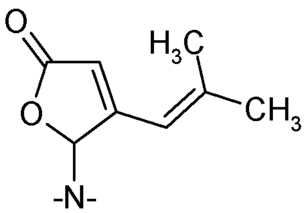

and

(b) treating the compound of formula (**VII**) with water in the presence of an acid catalyst.

In a further embodiment **A5E2,** the invention provides a process according to embodiment **A5E1** wherein the compound of formula (**VII**) from step (a) is isolated prior to hydrolysis step (b).

In a further embodiment **A5E3,** the invention provides a process according to embodiment **A5E1** wherein hydrolysis step (b) is carried out directly following step (a) such that the compound of formula (**VII**) or (**VII$^B$**) is not isolated prior to hydrolysis step (b).

In a further embodiment **A5E4,** the invention provides a process according to embodiments **A5E1, A5E2** or **A5E3** wherein the compound of formula (**VII**) is prepared by treating a compound of formula (**VIII**)

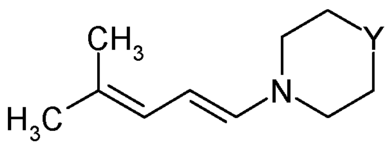

(**VIII**)

wherein -Y- represents a single bond, $-CH_2-$, -O-; -NH-, $-N((C_1-C_3)alkyl)-$, -N(benzyl)-, or

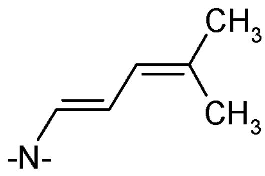

with glyoxylic acid or its hydrate.

In another aspect **A6,** the invention provides a process for the manufacture of a compound of formula (**VI**) wherein R is other than hydrogen, $R^1$-C(O)-, and $R^2$-$SO_2$-, comprising the step of treating a compound of formula **(VIA)** with an alcohol R-OH in the presence of an acid catalyst.

In an embodiment **A6E1,** the invention provides a process for the manufacture of a compound of formula (**VI**) wherein R is other than hydrogen, $R^1$-C(O)-, and $R^2$-$SO_2$-, comprising the steps of:

(a) preparing a compound of formula **(VIA)** using a process according to any of embodiments **A5E1, A5E2, A5E3** and **A5E4** as defined above; and
(b) treating the compound of formula **(VIA)** with an alcohol R-OH in the presence of an acid catalyst.

In another aspect **A7,** the invention provides a process for the manufacture of a compound of formula (**VI**) wherein R is other than hydrogen, $R^1$-C(O)-, and $R^2$-$SO_2$-, comprising the step of treating acompound of formula (**VII**) with an alcohol R-OH in the presence of stoichiometric acid.

In an embodiment **A7E1,** the invention provides a process for the manufacture of a compound of formula (**VI**) wherein R is other than hydrogen, $R^1$-C(O)-, and $R^2$-$SO_2$-, comprising the steps of:

(a) preparing a compound of formula (**VII**); and
(b) treating the compound of formula (**VII**) with an alcohol R-OH in the presence of stoichiometric acid.

In another aspect **A8,** the invention provides a process for the manufacture of a compound of formula (**VI**) wherein R is $R^1$-C(O)-, comprising the step of treating a compound of formula **(VIA)** with an acid chloride $R^1$-C(O)-Cl or acid anhydride $(R^1\text{-C(O)})_2O$.

In an embodiment **A8E1,** the invention provides a process for the manufacture of a compound of formula (**VI**) wherein R is $R^1$-C(O)-, comprising the steps of:

(a) preparing a compound of formula **(VIA)** using a process according to any of embodiments **A5E1, A5E2, A5E3** and **A5E4** as defined above; and
(b) treating the compound of formula **(VIA)** with an acid chloride $R^1$-C(O)-Cl or acid anhydride $(R^1\text{-C(O)})_2O$.

In another aspect **A9,** the invention provides a process for the manufacture of a compound of formula (**VI**) wherein R is $R^2$-$SO_2$-, comprising the step of treating a compound of formula **(VIA)** with a sulfonyl chloride $R^2$-$SO_2$-Cl.

In an embodiment **A9E1,** the invention provides a process for the manufacture of a compound of formula (**VI**) wherein R is $R^2$-$SO_2$-, comprising the steps of:

(a) preparing a compound of formula **(VIA)** using a process according to any of embodiments **A5E1, A5E2, A5E3** and **A5E4** as defined above; and
(b) treating the compound of formula **(VIA)** with a sulfonyl chloride $R^2$-$SO_2$-Cl.

A process for the manufacture of an enamine derivative of 4-methyl-2-pentanal is also disclosed herein:

In a first embodiment **A10E1,** the process for the manufacture of an enamine derivative of 4-methyl-2-pentenal comprises reacting acetaldehyde with isobutyraldehyde in the presence of a suitable amine.

In a further embodiment **A10E2** of embodiment **A10E1** the suitable amine is a secondary amine.

In a further embodiment **A10E3** of embodiment **A10E2** the secondary amine is selected from: $((C_1\text{-}C_4)\text{alkyl})_2NH$, pyrrolidine, piperidine, morpholine, piperazine, N-methylpiperazine, N-ethylpiperazine and N-benzylpiperazine.

In a further embodiment **A10E4** of embodiment **A10E3** the secondary amine is selected from pyrrolidine, piperidine, morpholine, and piperazine.

In a further embodiment **A10E5** of a process according to any of embodiments **A10E1, A10E2, A10E3** and **A10E4** the reaction is performed in the presence of an acid catalyst.

In a further embodiment **A10E6** of a process according to any of embodiments **A10E1, A10E2, A10E3, A10E4** and **A10E5** the isobutyraldehyde is combined with the suitable amine before addition of the acetaldehyde.

In a further embodiment **A10E6** of a process according to any of embodiments **A10E1, A10E2, A10E3, A10E4** and **A10E5** the isobutyraldehyde and acetaldehyde are added to the reaction vessel simultaneously.

In another aspect **A11,** the invention provides a process for the manufacture of 3-aminomethyl-5-methylhexanoic acid (**II**).

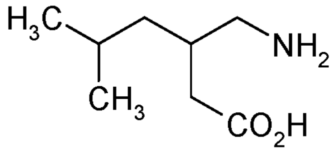

(**II**)

In a first embodiment **A11E1,** the invention provides a process for the manufacture of 3-aminomethyl-5-methylhexanoic acid (**II**) or a pharmaceutically acceptable salt thereof, comprising the steps:

(a) preparing 5-hydroxy-4-(2-methyl-1-propenyl)-5*H*-2-furanone (VIA)

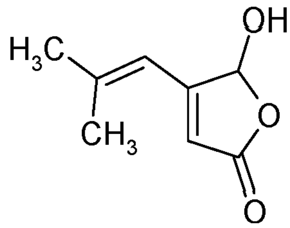

(**VI**$^{A}$)

(b) converting said 5-hydroxy-4-(2-methyl-1-propenyl)-5*H*-2-furanone **(VIA)** into 5-hydroxy-4-(2-methylpropyl)-3,4-dihydro-5*H*-2-furanone (**I**$^{A}$)

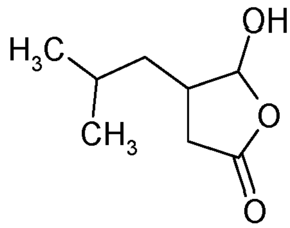

(**I**$^{A}$)

and

(c) converting said 5-hydroxy-4-(2-methylpropyl)-3,4-dihydro-5*H*-2-furanone (**I**$^{A}$) into 3-aminomethyl-5-methylhexanoic acid (**II**).

In a further embodiment **A11 E2,** the invention provides a process according to embodiment **A11E1** wherein the 3-aminomethyl-5-methylhexanoic acid (**II**) is (S)-3-aminomethyl-5-methylhexanoic acid ((*S*)-**II**)

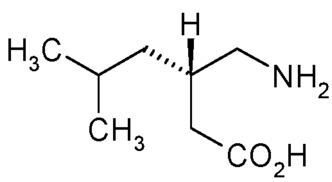

((*S*)-**II**)

wherein said (S)-3-aminomethyl-5-methylhexanoic acid has an enantiomeric excess of at least 80%.

In a further embodiment **A11 E3,** the invention provides a process according to embodiment **A11 E1** or **A11 E2** wherein step (a) comprises a process according to embodiments **A5E1, A5E2, A5E3** or **A5E4** as described above.

In a further embodiment **A11 E4,** the invention provides a process according to embodiment **A11 E1, A11 E2** or **A11 E3** wherein step (b) comprises the steps:

(b1) treating the 5-hydroxy-4-(2-methyl-1-propenyl)-5*H*-2-furanone **(VIA)** with a metal oxide, hydroxide, carbonate or bicarbonate, ammonia, a mono- di- or tri-($C_1$-$C_3$)alkylamine, or a tetra-($C_1$-$C_3$)alkylammonium hydroxide to form a salt of formula **(IX)**

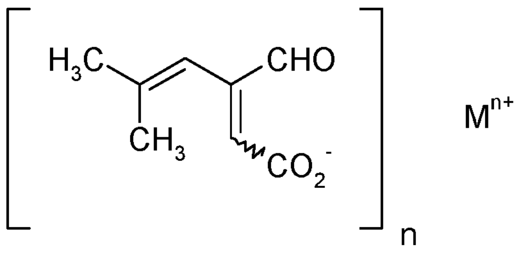

(**IX**)

wherein:

n is 1 and $M^+$ is selected from $Li^+$, $Na^+$, $K^+$, $Rb^+$, $NH_4^+$, (($C_1$-$C_3$)alkyl)$NH_3^+$, (($C_1$-$C_3$)alkyl)$_2NH_2^+$, (($C_1$-$C_3$)alkyl)$_3NH^+$ and (($C_1$-$C_3$)alkyl)$_4N^+$; or
n is 2 and $M^{2+}$ is selected from $Mg^{2+}$, $Ca^{2+}$ and $Zn^{2+}$;

(b2) hydrogenating the salt of formula (**IX**) to obtain a salt of formula (X)

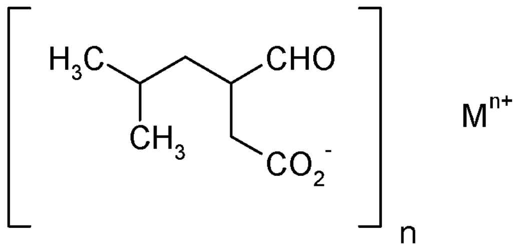

(**X**) ;

and
(b3) treating the salt of formula **(X)** with an acid.

In a further embodiment **A11 E5,** the invention provides a process according to embodiment **A11 E1, A11 E2** or **A11 E3** wherein step (b) comprises the steps:

(b1) converting the 5-hydroxy-4-(2-methyl-1-propenyl)-5*H*-2-furanone **(VIA)** to a compound of formula (**VI**) as defined in embodiment **A1E3,** wherein R is a chiral ($C_5$-$C_{15}$)hydrocarbon group;
(b2) hydrogenating the compound of formula (**VI**) to obtain a compound of formula (**XI**)

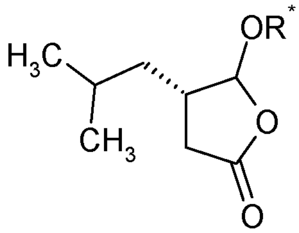

(**XI**)

wherein R* is a chiral ($C_5$-$C_{15}$)hydrocarbon group; and
(b3) treating the compound of formula (**XI**) with an acid to give ((*S*)-**I$^A$**).

In another aspect **A12,** the invention provides a further process for the manufacture of 3-aminomethyl-5-methylhexanoic acid (**II**).

In a first embodiment **A12E1,** the invention provides a process for the manufacture of 3-aminomethyl-5-methylhexanoic acid (**II**) or a pharmaceutically acceptable salt thereof, comprising the steps:

(a) preparing 5-hydroxy-4-(2-methyl-1-propenyl)-5*H*-2-furanone **(VIA)**

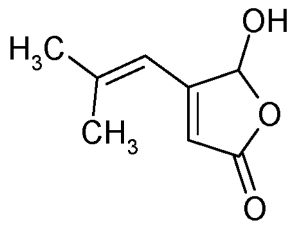

(**VI$^A$**)

(b) treating the 5-hydroxy-4-(2-methyl-1-propenyl)-5*H*-2-furanone **(VIA)** with ammonia or a mono-($C_1$-$C_3$)alkylamine to form a salt of formula (**IX$^A$**)

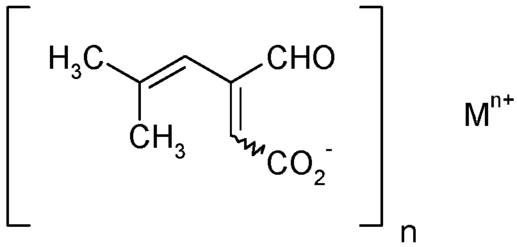

(**IX$^A$**)

wherein n is 1 and $M^+$ is selected from $NH_4^+$ and (($C_1$-$C_3$)alkyl)$NH_3^+$

(c) hydrogenating the salt of formula (**IX$^A$**) to obtain a salt of formula (**X$^A$**)

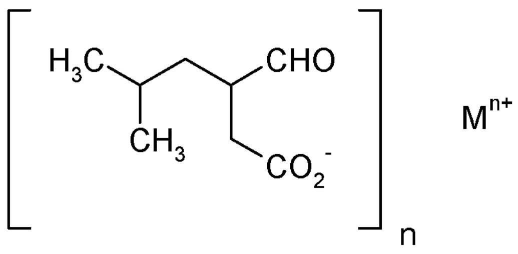

($X^A$) ;

and

(d) treating the salt of formula ($X^A$) with a transaminase or an amine oxidase/imine reductase enzyme to provide 3-aminomethyl-5-methylhexanoic acid (**II**).

In a further embodiment **A12E2,** the invention provides a process according to embodiment **A12E1** wherein the 3-aminomethyl-5-methylhexanoic acid (**II**) is (S)-3-aminomethyl-5-methylhexanoic acid ((*S*)-**II**)

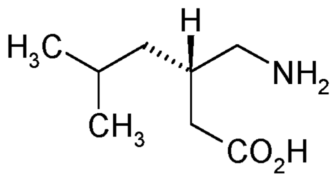

((*S*)-**II**)

wherein said (*S*)-3-aminomethyl-5-methylhexanoic acid has an enantiomeric excess of at least 80%.

In a further embodiment **A12E3,** the invention provides a process according to embodiment **A12E1** or **A12E2** wherein step (a) comprises a process according to embodiments **A5E1, A5E2, A5E3** or **A5E4** as described above.

Detailed Description of Preferred Embodiments

The term "alkyl" means a straight-chain or branched-chain saturated aliphatic hydrocarbon radical containing the specified number of carbon atoms. Examples of alkyl radicals include methyl, ethyl, *n*-propyl, isopropyl, *n*-butyl, isobutyl, *sec*-butyl, *tert*-butyl, neopentyl and *n*-hexyl.

The term "alkoxy" means a group made up of an alkyl group as defined above connected to an oxygen atom. Examples of alkoxy groups include methoxy, ethoxy and isopropoxy.

The term "alkoxy-alkyl" means a straight-chain or branched-chain saturated aliphatic hydrocarbon radical in which an alkoxy group is substituted for an alkyl hydrogen atom. An example of an alkoxy-alkyl group is 2-methoxyethyl.

The term "haloalkyl" means an alkyl group as defined above wherein one or more hydrogen atoms are replaced by fluorine, chlorine, bromine or iodine. When more than one hydrogen atom is replaced, the replacing halogen atoms may be the same or different. Examples of haloalkyl groups include fluoromethyl, difluoromethyl, trifluoromethyl, chlorodifluoromethyl, 2,2,2-trifluoroethyl and 3-bromopropyl.

The term "aryl" means a phenyl or naphthyl group.

The term "aryl-alkyl" means a straight-chain or branched-chain saturated aliphatic hydrocarbon radical in which an aryl group is substituted for an alkyl hydrogen atom. An example of an aryl-alkyl group is benzyl.

The term "cycloalkyl" means a saturated monocyclic or polycyclic carbocyclic ring containing the specified number of carbon atoms. Examples of monocyclic cycloalkyl groups include cyclopropyl, cyclobutyl, cyclopentyl and cyclohexyl. Examples of polycyclic cycloalkyl groups include bicyclo[2,2,1]heptyl and bicyclo[3,2,1]octyl.

The term "optionally substituted" with reference to an alkyl or aryl group means that a hydrogen atom of the alkyl or aryl group may be replaced by one of the groups listed. The substitution may be made at any position within the alkyl or aryl group. When the optional substitution is with "one or more groups" then any number of hydrogen atoms of the alkyl or aryl group, up to a maximum equal to the number of hydrogens present in the alkyl or aryl group, may be replaced, and each replacement is independent of the others.

The term "enantiomeric excess", sometimes abbreviated as "e.e.", is a measure, for a given sample, of the excess of one enantiomer in excess of its antipode and is expressed as a percentage. Enantiomeric excess is defined as:

$$100 \times (er-1) / (er+1)$$

where "er" is the ratio of the more abundant enantiomer to the less abundant enantiomer.

5-Hydroxy-4-(2-methylpropyl)-3,4-dihydro-5*H*-2-furanone (**I**^A^) is a convenient intermediate in the manufacture of 3-aminomethyl-5-methylhexanoic acid (**II**). Treatment of racemic (**I**^A^) with ammonia in the presence of a chemical reducing agent provides racemic 3-aminomethyl-5-methylhexanoic acid ((*R*/*S*)-**II**) (see WO2008/127646A2). The reaction is presumed to involve the ring-opened isomer (**I**^B^).

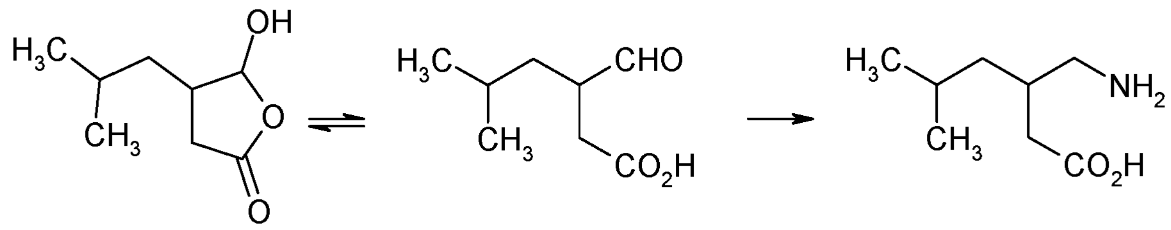

($I^A$) ($I^B$) (**II**)

Reductive amination with an amine donor in the presence of a transaminase enzyme can provide enantiomerically enriched 3-aminomethyl-5-methylhexanoic acid. Suitable amine donors are primary amines such as mono-alkylamines, particularly isopropylamine, and α-amino acids.

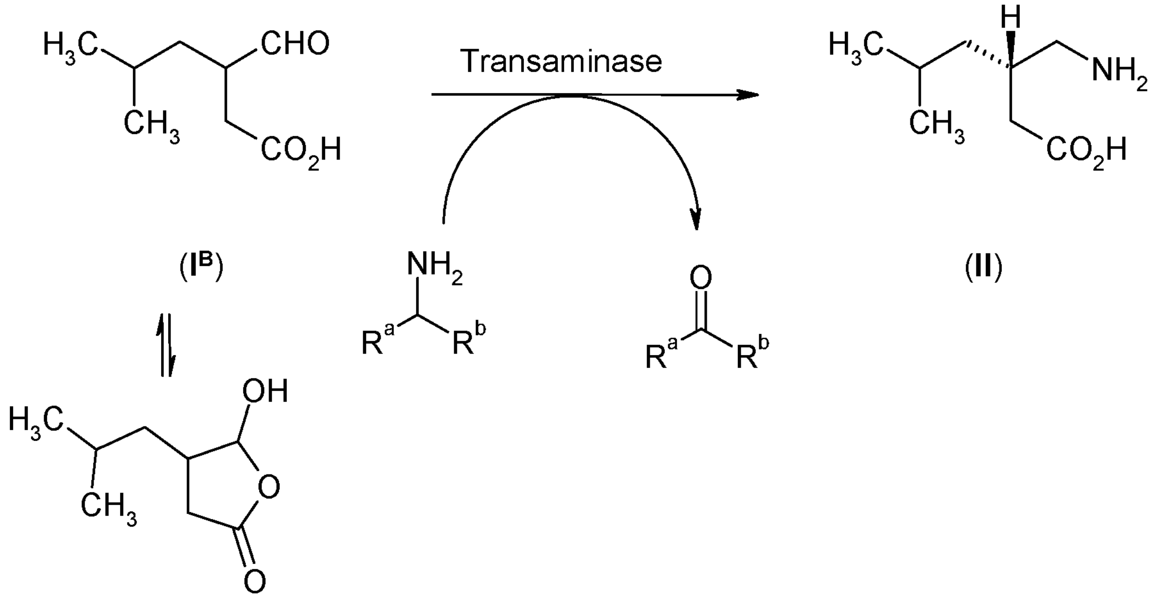


($I^B$) (**II**) ($I^A$)

The reaction of ($I^A$) with a suitable amine dehydrogenase/ imine reductase in the presence of ammonia can also be a suitable route to pregabalin. A co-factor such as NADH or NADPH may be needed in a stoichiometric amount, or a second oxidoreductase, such as formate dehydrogenase, may be included to recycle the co-factor.

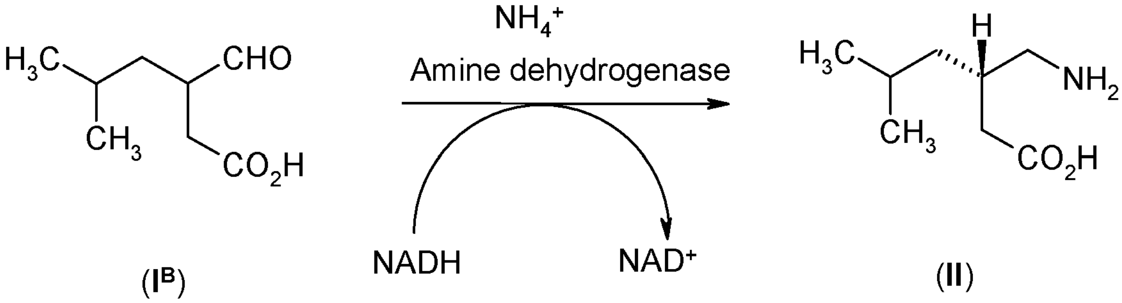


($I^B$) (II)

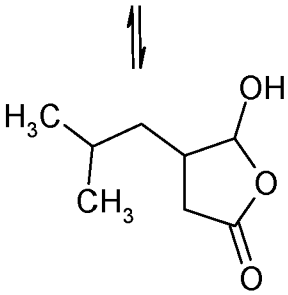

($I^A$)

Where the dihydrofuranone ($I^A$) has a defined stereochemistry at the C-4 position, this stereochemistry may be preserved during the reductive amination reaction. Since Pregabalin has the (S)-stereochemistry it may be preferred to have this stereochemistry already present in the dihydrofuranone.

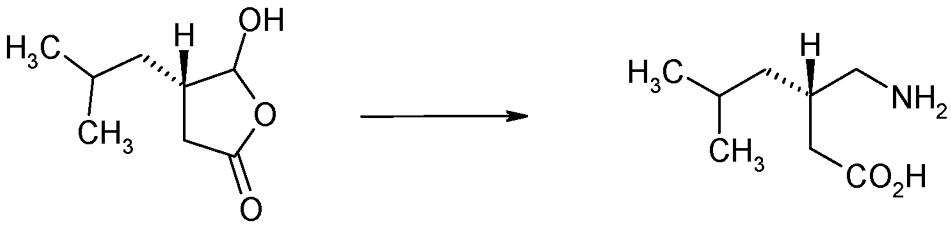

((*S*)-$I^A$) ((*S*)-**II**)

Alternatively, the dihydrofuranone ($I^A$) may be used as in racemic form. The desired stereoisomer of the product may then be obtained either by carrying out the reductive amination reaction under conditions that allow for the stereoselective formation of a single enantiomer, such as by carrying out the transformation in the presence of a transaminase enzyme, or by subjecting the product to a separate resolution step, such as by crystallization with a chiral acid or base.

The dihydrofuranone ($I^A$) may be conveniently prepared in racemic or enantiomerically enriched form using the methods set out below.

In a first method, the dihydrofuranone ($I^A$) is prepared by reduction of 5-hydroxy-4-(2-methyl-1-propenyl)-5H-2-furanone **(VIA).**

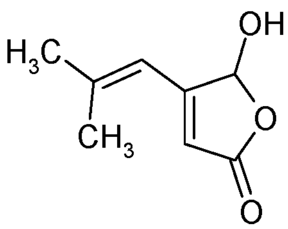

($VI^A$)

The reduction may conveniently be accomplished by hydrogenation in the presence of a suitable catalyst. Suitable catalysts include homogeneous and heterogeneous catalysts. The catalyst typically comprises a transition metal such as palladium, platinum, rhodium, ruthenium or nickel, or a salt or oxide thereof. Heterogeneous catalysts include finely divided metals and substrate-supported metals and metal oxides, where the substrate may be carbon, silica, alumina or any other suitable inert material. Homogeneous catalysts include phosphine ligand complexes of transition metals. When the phosphine ligand is chiral then the catalyst is chiral. When an achiral catalyst is used, then the product is the racemic dihydrofuranone ($I^A$). The use of a chiral catalyst may provide the dihydrofuranone ($I^A$) in an enantioselective manner.

The selectivity of the hydrogenation reaction, and the overall yield, maybe improved when the reaction is carried out in an alkaline medium. Without being bound by theory, it is thought that in the presence of a base the furanone exists predominantly as the ring-opened salt **(IX).**

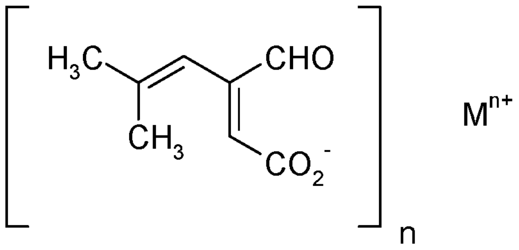

(**IX**)

Any suitable base may be used provided that it does not interfere with the hydrogenation process, such as by poisoning the catalyst. Examples of suitable bases include alkali (such as Li, Na, K and Rb) and alkaline earth metal (such as Ca and Mg) oxides, hydroxides, carbonates and bicarbonates. Other metal salts, such as zinc salts, may also be used. Alkali metal salts may be preferred due to their good solubility and/or low toxicity. Amine bases such as ammonia, and primary, secondary and tertiary amines may be used to prepare ammonium salts. Tetra-alkylammonium hydroxide may also be used, leading to the formation of tetra-alkylammonium salts.

Hydrogenation of the salt of formula (**IX**) provides a salt of formula **(X).**

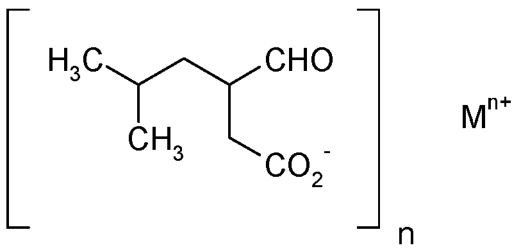

(**X**)

The dihydrofuranone ($I^A$) is recovered, after the hydrogenation reaction, by treatment of this salt with a suitable acid. Alternatively, the salt may be converted directly to 3-aminomethyl-5-methylhexanoic acid (**II**) by treatment with a transaminase or amine oxidase/imine reductase enzyme. In this case it may be preferable to use the ammonium salt ($M^+ = NH_4^+$) or a primary alkylammonium salt ($M^+$ = alkyl-$NH_3^+$) since the ammonium or alkylammonium ion provides the co-substrate for the enzyme. The use of the isopropylammonium salt in combination with a transaminase enzyme is a preferred example.

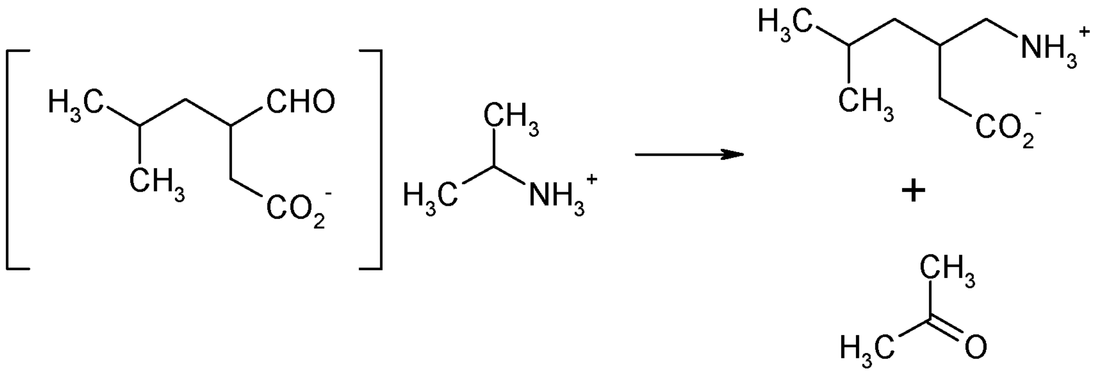

Furanones of formula **(VI)** wherein R is other than hydrogen may also be reduced by hydrogenation. Where R is an alkyl, haloalkyl, alkoxyalkyl alkenyl, cycloalkyl, cycloalkyl-alkyl, aryl or aryl-alkyl group, these furanones may be prepared from the compound of formula **(VIA)** by reaction with an alcohol R-OH in the presence of an acid catalyst. Where R is $R^1$-C(O)- the furanones may be prepared from the compound of formula **(VIA)** by reaction with an acid anhydride $(R^1\text{-C(O)})_2O$ or acid chloride $R^1$-C(O)-Cl, optionally in the presence of a base, for example a tertiary amine. Where R is $R^2$-$SO_2$- the furanones may be prepared from the compound of formula **(VIA)** by reaction with a sulfonyl chloride $R^2$-$SO_2$-Cl, optionally in the presence of a base, for example a tertiary am ine.

Following the hydrogenation step the dihydrofuranone of formula (**I**$^A$) can be generated from the reduction product by treatment with acid (where R is an alkyl, haloalkyl, alkoxyalkyl alkenyl, cycloalkyl, cycloalkyl-alkyl, aryl or aryl-alkyl group) or base (where R is $R^1$-C(O)- or $R^2$-$SO_2$-).

The use of a chiral R group may provide a chiral hydrogenation product without the need for a chiral catalyst.

For example, the furanone **(VIA)** is reacted with a chiral alcohol R*-OH to afford a chiral ether derivative (**VI**$^B$).

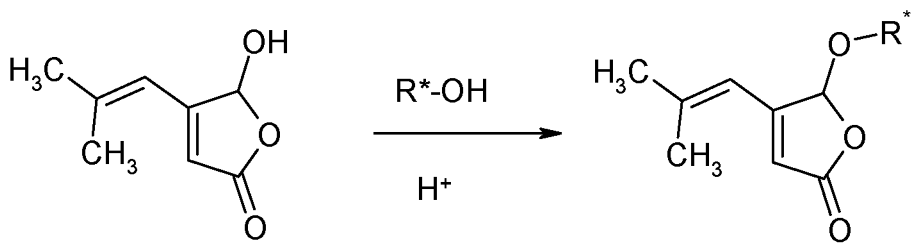


(**VI**$^A$) (**VI**$^B$)

Suitable chiral alcohols may include α-aryl alcohols such as 1-phenylethanol and 1-naphthylethanol, as well as terpene alcohols such as menthol and borneol. Hydrogenation of derivative (**VI**$^B$) may proceed in an enantioselective manner, and treatment of the resulting product with a suitable acid and in the presence of water then provides the dihydrofuranone (**I**$^A$) in chiral form.

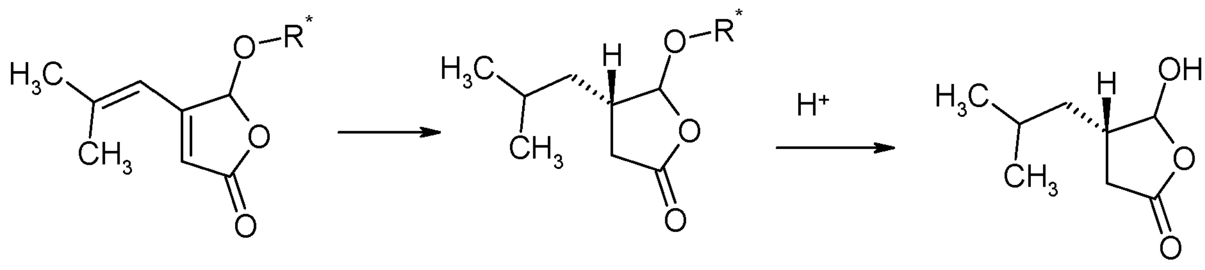


(**VI**$^B$) ((S)-**I**$^A$)

The furanone (VIA) may be prepared from a compound of formula (**VII**)

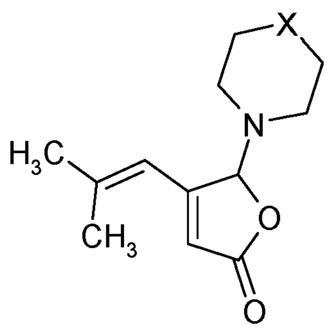

(**VII**)

wherein -X- represents a single bond, $-CH_2-$, -O-, -NH-, $-N((C_1-C_3)alkyl)-$, -N(benzyl)-, or

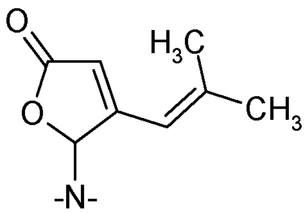

by treating the compound of formula (**VII**) with water in the presence of an acid catalyst. Suitable acids include mineral acids such as sulphuric acid. Alternatively, the compound of formula (**VII**) can be treated with an alcohol R-OH to provide directly a compound of formula **(VI)** wherein R is an alkyl, haloalkyl, alkoxyalkyl alkenyl, cycloalkyl, cycloalkyl-alkyl, aryl or aryl-alkyl group.

The compounds of formula (**VII**) can be prepared by the reaction of a dienamine of formula (**VIII**).

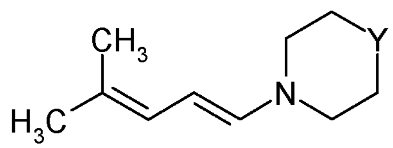

(**VIII**)

wherein -Y- represents a single bond, $-CH_2-$, -O-; -NH-, $-N((C_1-C_3)alkyl)-$, -N(benzyl)-, or

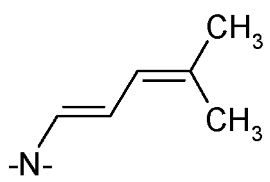

with glyoxylic acid or its hydrate.

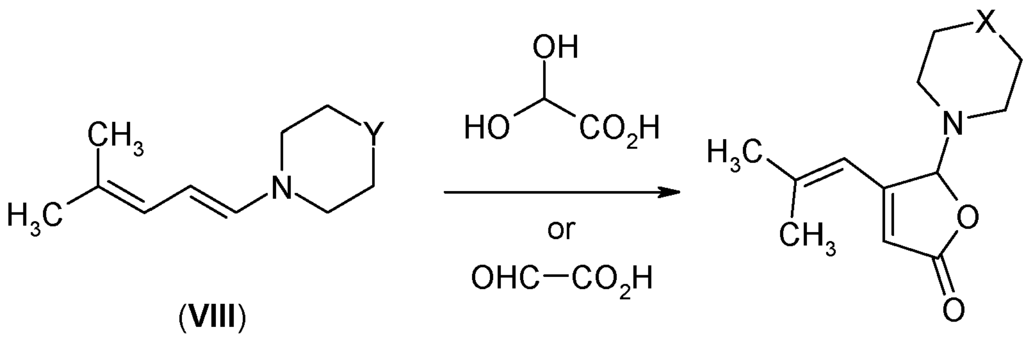


(VIII)

(VII)

It will be understood that -X- in formula (**VII**) corresponds to -Y- in the starting material of formula (VIII), except that when -Y- is

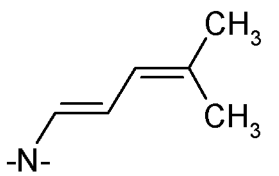

then –X- is

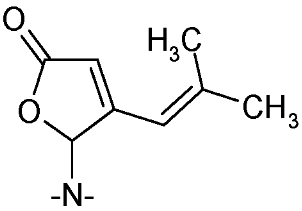

The compounds of formula (**VIII**) wherein -Y- represents a single bond or $-CH_2-$ have been prepared by the reaction of 4-methyl-2-pentenal with pyrrolidine or piperidine (Kienzle, F. et al., Helv. Chim. Acta 1985, 68(5), 1133-39). Other compounds of formula (**VIII**) may be prepared analogously.

Alternatively, condensation of isobutyraldehyde and acetaldehyde in the presence of a suitable amine such as pyrrolidine, piperidine or morpholine with catalytic acid in a solvent such as acetonitrile provides the dienamine derivatives (**VIII**).

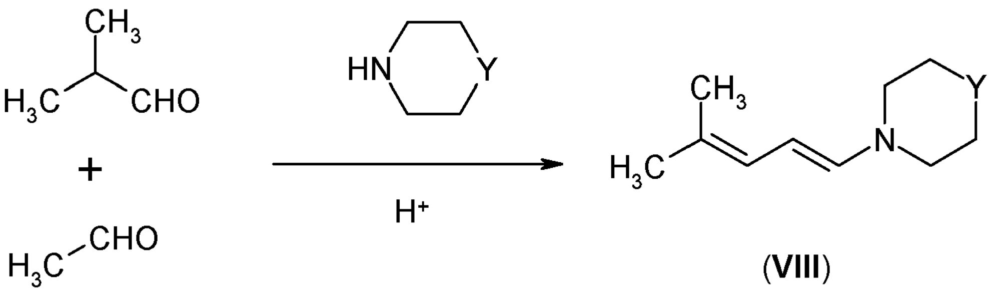


(VIII)

Y = single bond, $CH_2$, O, N(alkyl), N(benzyl)

If piperazine is used as the amine then a *bis*-dienamine is obtained.

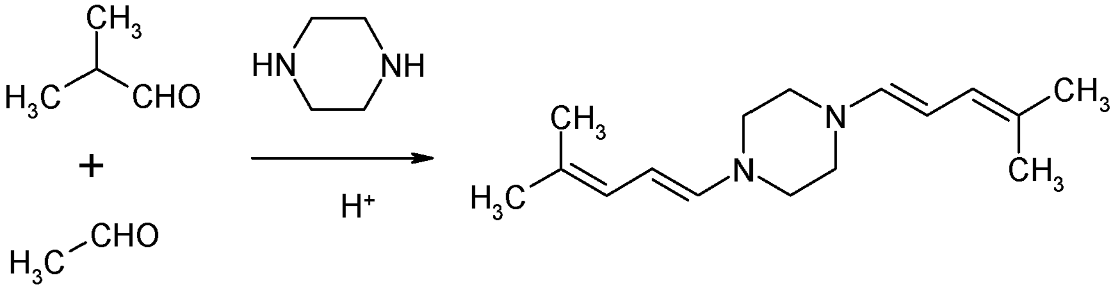

The compound of formula (VIII) wherein Y is NH may be obtained by using mono-protected piperazine as the amine, followed by a deprotection step.

Acyclic secondary amines such as diethylamine and di-isopropylamine may also be used, but cyclic secondary amines are preferred.

This mode of reaction of isobutyraldehyde and acetaldehyde differs from the direct base catalysed reaction (eg using potassium carbonate as base: UK patent GB834100) which only gives the adduct 2,2-dimethyl-3-hydroxybutanal where isobutyraldehyde acts as a nucleophile.

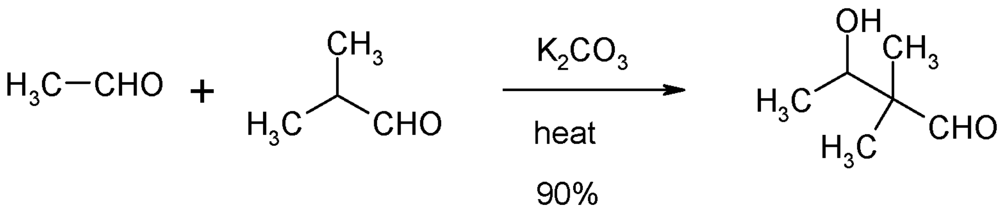


Without wishing to be bound by any particular theory, it is postulated that both the acetaldehyde and the isobutyraldehyde are initially converted to their enamine derivatives. In the presence of the acid catalyst, the more basic isobutyraldehyde enamine is converted to its iminium ion. This electrophilic species reacts preferentially with the less sterically hindered nucleophile, which is the acetaldehyde enamine - this ensures reaction the desired way around.

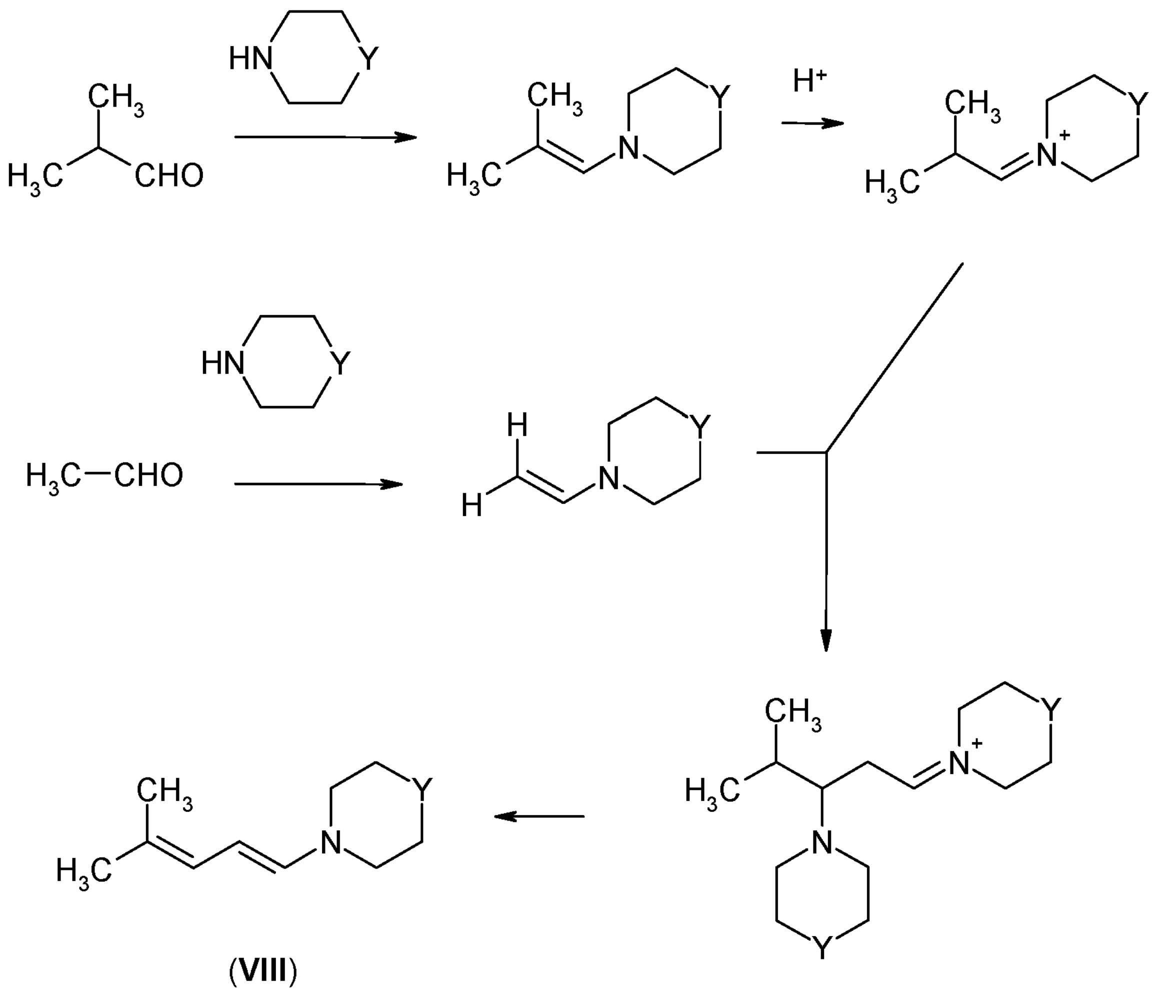


(VIII)

The method of the present invention is more economical and more amenable to scale-up than the literature methods of effecting this 'umpolung' of normal acetaldehyde reactivity, in which acetaldehyde is converted to an O-silylated enol derivative and coupled with the isobutyraldehyde under Mukiyama aldol conditions, In the present invention, both coupling partners are activated simultaneously - electronic and steric effects directing the observed reactivity pattern.

The other advantage is that the product dienamine is the desired 'activated' form of 4-methyl-2-pentenal for reaction with glyoxylic acid to form the desired 5-aminofuranones (**VII**).

These dienamine derivatives of formula (**VIII**) may be isolated and purified, or alternatively they can be treated directly with glyoxylic acid (or its hydrate), which provides furanone derivative (**VII**) directly.

The furanone derivatives (**VII**) may be isolated and purified. Treatment with aqueous acid then provides furanone **(VIA).**

Overall, the transformations set out above provide a short route for the manufacture of pregabalin using inexpensive and safe starting materials.

Examples

The invention is illustrated by the following non-limiting examples in which the following abbreviations and definitions are used:

| | |
|---|---|
| bp | Boiling point |
| CPME | Cyclopentyl methyl ether |
| d | Doublet |
| DIW | De-ionised water |
| dd | Doublet of doublets |
| eq or eq. | Equivalent |
| e.e. or ee | Enantiomeric excess |
| $ES^+$ | Positive mode electrospray ionization |
| EtOAc | Ethyl Acetate |
| EtOH | Ethanol |
| GC | Gas chromatography |
| GC/MS | Gas chromatography / Mass spectroscopy |
| HOAc | Acetic acid |
| HPLC | High Performance Liquid Chromatography |
| Hr or h | Hour |
| $^1H$ NMR | Proton Nuclear Magnetic Resonance Spectroscopy |
| L | Litre |
| LCMS | Liquid chromatography / Mass spectroscopy |
| m | Multiplet |
| mbar | Millibar |
| MeOH | Methanol |
| min | Minute |
| mL | Millilitre |
| mmol | Millimole |
| mol | Mole |
| Mp | Melting point |
| MTBE | Methyl tert-butyl Ether |
| $NAD^+$ | Nicotinamide adenine dinucleotide (oxidised) |
| $NADP^+$ | Nicotinamide adenine dinucleotide phosphate (oxidised) |
| NADH | Nicotinamide adenine dinucleotide (reduced) |
| NADPH | Nicotinamide adenine dinucleotide phosphate (reduced) |
| PLP | Pyridoxal phosphate |
| ppm | Parts per million |
| pTsOH | Para-toluenesulfonic acid |
| q | Quartet |
| qNMR | Quantitative nuclear magnetic resonance spectroscopy |
| $R_f$ | Retention factor |
| RT | Room temperature |
| s | Singlet |
| t | Triplet |
| ThDP | Thiamine diphosphate |
| TLC | Thin layer chromatography |
| TsOH | Toluenesulfonic acid (= pTsOH) |
| UPLC | Ultra Performance Liquid Chromatography |

| | |
|---|---|
| XRD | X-ray diffraction crystallography |
| δ | Chemical shift |

Commercial chemicals were used as received unless stated otherwise. Thin layer chromatography was performed on pre-coated plastic plates (Merck silica 60F254), and visualised using UV light and $KMnO_4$ dip. Proton ($^1H$) and carbon ($^{13}C$) NMR spectra were recorded on a Varian INOVA 300 MHz spectrometer. Chemical shifts are quoted relative to tetramethylsilane and referenced to residual solvent peaks as appropriate. Unless otherwise indicated, chiral HPLC analysis was performed using an Agilent 1200 HPLC system and data was processed using the Chemstation software or with a Varian semiprep/analytical HPLC using Galaxie software.

**Example 1**

**Preparation of 4-(2-methyl-1-propenyl)-5-morpholino-5*H*-2-furanone from 4-methyl-2-pentenal**

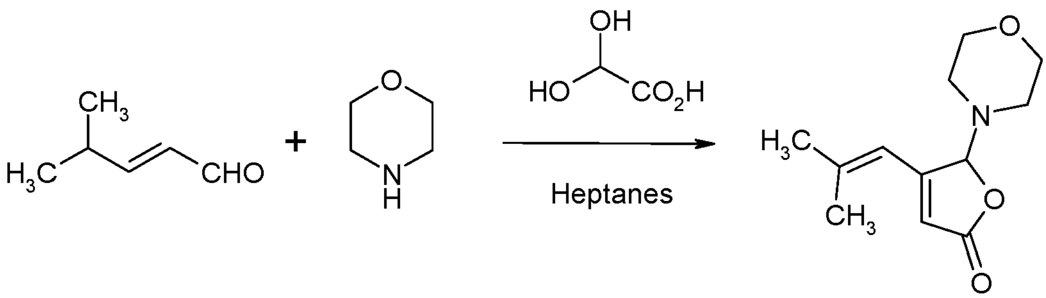


A 50% solution of glyoxylic acid in water (29.6g, 0.2mol) was added to a biphasic stirred mixture of morpholine (17.8g, 0.2mol) and heptanes (75mL), which had been pre-cooled to 0-10°C. The temperature was kept less than 10°C. The mixture was warmed to 20°C and 4-methyl-2-pentenal (19.6g, 0.2mol) was added. The mixture was stirred at 45°C for 20h. A large quantity of solid was formed. Water (100mL) was added at ambient temperature and the mixture stirred for 2h. The mixture was extracted with cyclopentyl methyl ether (100mL) and the organic solution washed twice with water (100mL) and concentrated to leave 30.4g of crude product. This solid was purified by recrystallisation from methanol (100mL) to afford 17.7g (40%) of pure 4-(2-methyl-1-propenyl)-5-morpholino-5*H*-2-furanone.

GC/MS: m/z = 223
$^1H$ NMR: δ 6.0 (s, 1 H); 5.9 (s, 1 H); 5.5 (s, 1 H), 3.7 (d, 4H), 2.7 (d, 4H), 2.00 (s, 3H), 1.95 (s, 3H).

**Example 2**

**Preparation of 4-(4-methyl-1,3-pentadien-1-yl)morpholine**

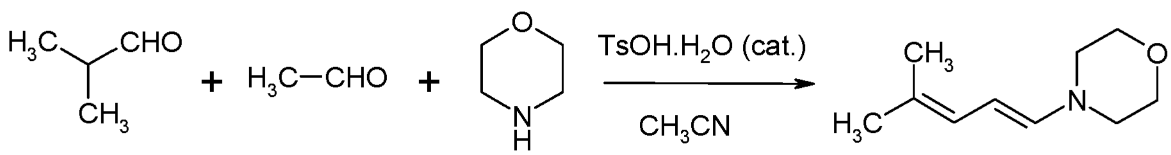


Isobutyraldehyde (46.90g, 0.65mol, 1.43eq.) was stirred in acetonitrile (300mL). Morpholine (56.63g, 0.65mol, 1.43eq.) followed by pTsOH (8.63g, 0.1 equiv) were slowly added at room temperature to the isobutyraldehyde solution. A solution of acetaldehyde (20g, 0.454mol) in acetonitrile (100mL) was added drop-wise over 1hr with internal temperature monitoring at 50°C. After complete addition the mixture was stirred for 30min at 50°C and then cooled to room temperature before evaporation of the solvent *in vacuo* to give an orange oil (123.3g). Assay of the crude by quantitative NMR using benzyl benzoate as the internal standard was 40% which give 65% yield of the desired dienamine.

GC/MS: m/z = 167

$^1H$ NMR: δ 6.01 (d, 1 H); 5.69 (d, 1 H); 5.31 (dd, 1 H), 3.70 (m, 4H), 2.89 (m, 4H), 1.71 (s, 3H), 1. 63 (s, 3H).

**Example 3**

**Preparation of 4-(2-methyl-1-propenyl)-5-morpholino-5*H*-2-furanone from crude 4-(4-methyl-1,3-pentadien-1-yl)morpholine**

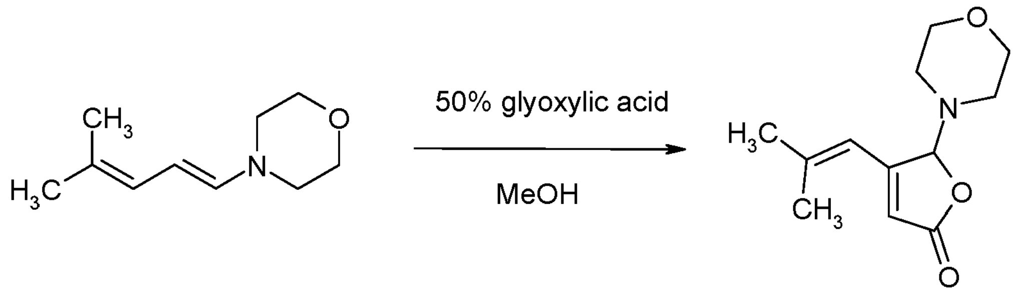


The crude 4-(4-methyl-1,3-pentadien-1-yl)morpholine dienamine of example 2 (123.3g, 40% assay) was dissolved in methanol (300mL) at room temperature. On complete dissolution, glyoxylic acid (50wt%, 60g, 1.1eq) was added and the resultant biphasic mixture stirred at 50°C for 18h. The reaction mixture was cooled to room temperature and the solvent removed by rotary evaporation. The residue was partitioned between ethyl acetate (200mL) and saturated sodium carbonate solution (200mL). The aqueous phase was extracted with ethyl acetate (100mL) and combined organics washed with brine and evaporated to a thick oil which solidified on standing (89.0g, 67% assay by qNMR, 60% yield).

GC/MS: m/z = 223
NMR: as example 1.

**Example 4**

**Preparation of 4-(2-methyl-1-propenyl)-5-morpholino-5*H*-2-furanone in one pot from isobutyraldehyde and acetaldehyde.**

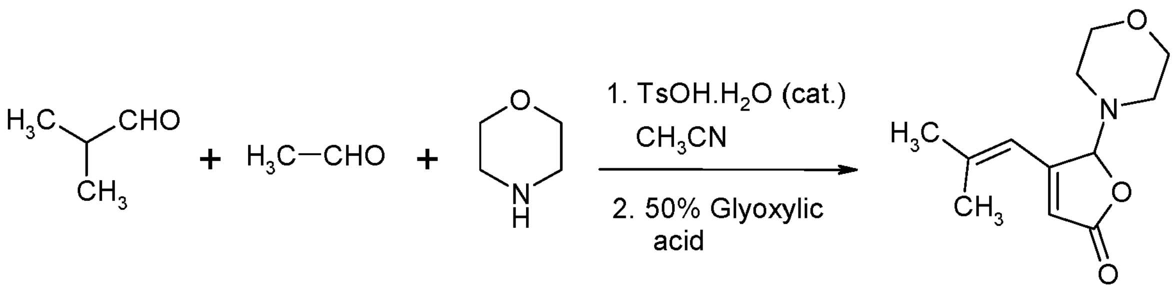


Isobutyraldehyde (102.90g, 1.43mol) was stirred in acetonitrile (600mL). Morpholine (124.3g, 1.43mol) followed by pTsOH (19.0g, 0.1 equiv) were slowly added at room temperature to the isobutyraldehyde solution. A solution of acetaldehyde (44.05g, 1.0mol) in acetonitrile (150mL) was added drop-wise over 1hr with internal temperature monitoring at 50°C. After complete addition the mixture was stirred for 30min at 50°C and then cooled to <10°C. Glyoxylic acid (50wt%, 211.3g, 1.43mol) was added and the resultant biphasic mixture stirred at 50°C for 18h. The reaction mixture was cooled to room temperature and the solvent removed by rotary evaporation. The residue was partitioned between ethyl acetate (1L) and saturated sodium carbonate solution (1L). The aqueous was extracted with ethyl acetate and combined organics washed with brine and evaporated to a thick oil which solidified on standing (166g). The crude product was triturated with MTBE at room temperature. The product is collected by filtration and washed with MTBE to give pure

4-(2-methyl-1-propenyl)-5-morpholino-5*H*-2-furanone (84g, 37% yield) identical to the material prepared in Example 1. Analysis of the crude solid (166g) indicated ca 50% yield.

**Example 5**

**Preparation of 1, 4-bis-(4-methyl-1,3-pentadien-1-yl)piperazine**

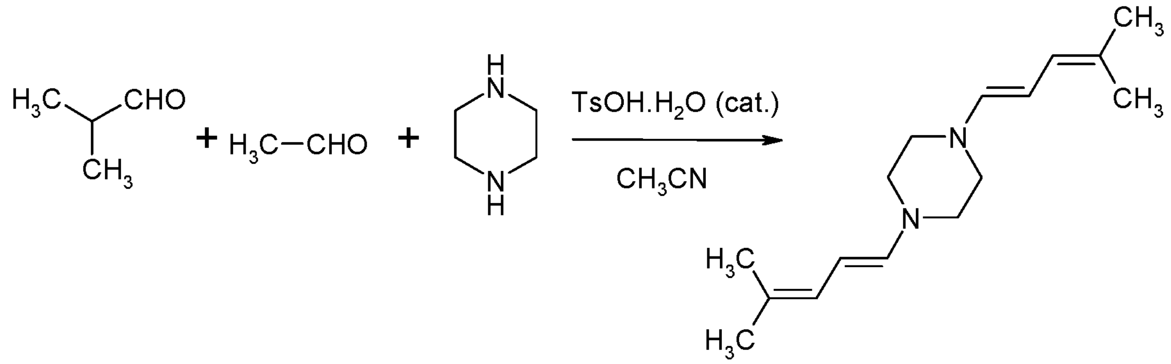


A solution of piperazine (43.0g, 0.5mol) and 4-toluenesulfonic acid monohydrate (4.4g, 0.023mol) in acetonitrile (600mL) was prepared. This solution was heated to 50°C and isobutyraldehyde (100mL, 1.10mol) added over 10 minutes. The solution went red-orange in colour and a transient white precipitate occurred. A solution of acetaldehyde (30.8g, 0.70mol) in acetonitrile (30 mL) was then added via syringe pump over 3h at 50°C. A suspension was formed which was stirred at 50°C for 0.5h. The solvent was removed and the residual solid isolated from methanol (500mL) at -5°C. It was filtered and washed with chilled methanol and dried to afford 52.9g (60%) of 1,4-bis-(4-methyl-1,3-pentadien-1-yl)piperazine.

GC/MS: m/z = 246.

$^1$H NMR: $\delta$ 6.00 (d, 2H); 5.70 (d, 2H); 5.28 (dd, 2H); 2.92 (s, 8H); 1.73 (s,6H); 1.68 (s, 6H). $^{13}$C NMR ($CDCl_3$)- 140.6 (C), 126.3 (CH), 123.4 (CH), 100.2 (CH), 48.2 ($CH_2$), 25.8 ($CH_3$), 18.1 ($CH_3$).

**Example 6**

**Preparation of 5,5'-(piperazine-1,4-diyl)bis(4-(2-methylprop-1-en-1-yl)furan-2(5*H*)-one) from 1, 4-bis-(4-methyl-1,3-pentadien-1-yl)piperazine.**

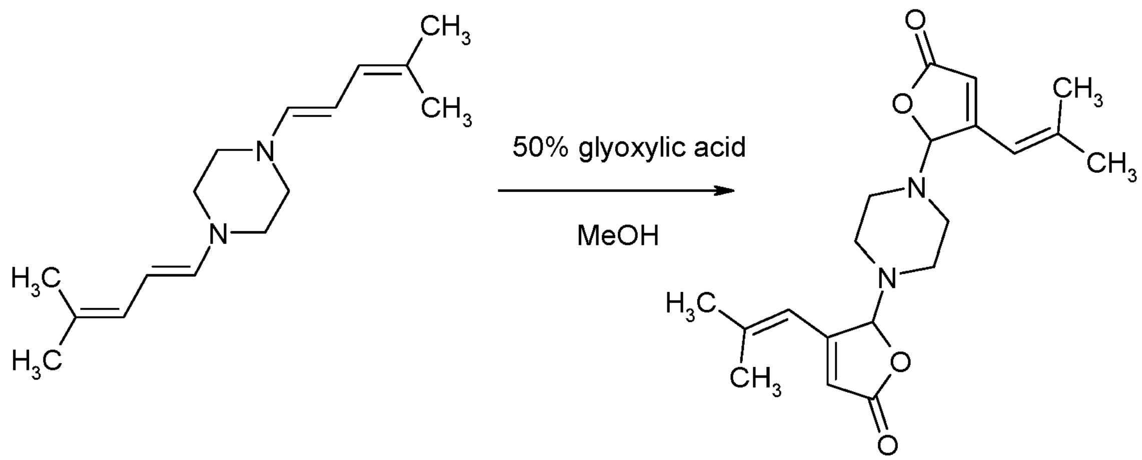

1,4-bis-(4-Methyl-1,3-pentadien-1-yl)piperazine (37.3g, 0.151mol); see Example 5) was charged to methanol (300mL). The temperature was adjusted to 34°C and a 50% solution of glyoxylic acid in water (44.9g, 0.302 mol) was added rapidly (over 5 minutes). The resulting suspension was stirred at 45°C for 15h; cooled to 0-10°C for 2h and filtered. The solid was washed with methanol (100mL) and dried to afford pure 5,5'-(piperazine-1,4-diyl)bis(4-(2-methylprop-1-en-1-yl)furan-2(5H)-one) (32.3g, 60%). An extra 5% could be obtained from the mother-liquor by concentration.

$^1$H NMR: $\delta$ 5.96 (d, 2H), 5.87 (d, 2H), 5.56 (m, 2H), 2.71 (s, 8H), 2.07-1.90 (m,12H). $^{13}$C NMR ($CDCl_3$): 172.3 (C), 159.2 (C), 150.9 (C), 116.6 (CH), 115.4 (CH), 99.2 (CH), 46.7 ($CH_2$), 28.2 ($CH_3$), 21.4 ($CH_3$).

This reaction can also be conducted in isopropanol, acetonitrile-water, toluene-water or in heptane water with similar yields.

## Example 7

## Preparation of 5,5'-(piperazine-1,4-diyl)bis(4-(2-methylprop-1-en-1-yl)furan-2(5H)-one) in one pot from isobutyraldehyde and acetaldehyde.

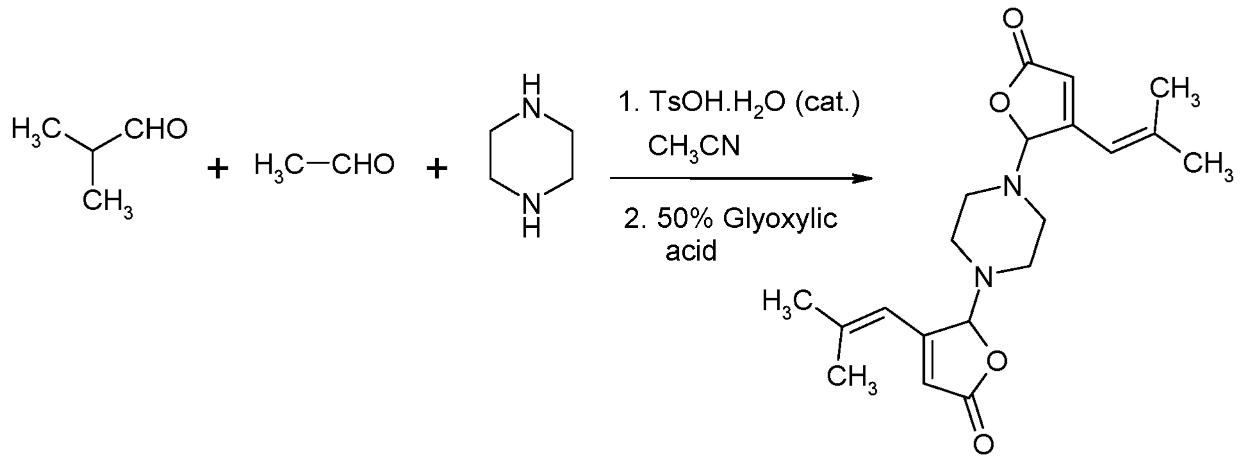


Tosic acid (6.5g, 0.03mol) was charged to a solution of piperazine (59g, 0.69 mol) in acetonitrile (240mL). The reaction was heated to 50°C and agitated until dissolution of solids was observed, before addition of isobutyraldehyde (138mL, 1.51mol). The reaction was held at 50°C before a solution of acetaldehyde (60mL; 1.07mol) in acetonitrile (30mL) was charged to the vessel over 3hrs via syringe pump. On complete addition, the reaction was stirred for a further 30 minutes before a 50% w/w solution of glyoxylic acid in water (148g, 1.0 mol) was added over 5min to the reaction mixture followed by water (50ml). The reaction was then heated to 70°C for 2h, then to 50°C overnight. The reaction was then cooled to 5°C and held for 30 minutes. 5,5'-(Piperazine-1,4-diyl)bis(4-(2-methylprop-1-en-1-yl)furan-2(5H)-one) precipitated and was isolated by filtration and washed with acetonitrile (2 x 100mL) to give the desired product (126 g, 93.5% assay, 66% yield from acetaldehyde).

## Example 8

## Solvent free preparation of 5,5'-(piperazine-1,4-diyl)bis(4-(2-methylprop-1-en-1-yl)furan-2(*5H*)-one)

Isobutyraldehyde (79g, 100mL, 1.1mol) was charged to a 3-necked round bottom flask and argon was flushed through the system. Piperazine (43g, 0.5 mol) and TsOH-$H_2O$ (4.4g; 0.023 mol) were divided into 4 equal portions containing piperazine (10.75 g) and TsOH-$H_2O$ (1.1 g). Addition of the first portion of piperazine (10.75g) resulted in exotherm from 24°C to 35°C. This was followed by the first portion of TsOH (1.1 g). The reaction was agitated until all the piperazine had dissolved (t=35°C), after which the second portion of piperazine (10.75g) was added, followed by TsOH (1.1g) (t=41°C). Stirring was continued until all of piperazine had dissolved (t=41°C), then the third portion of piperazine (10.75g) was added, followed by TsOH (1.1 g). After the 3rd charge a clear solution was generated and then the fourth portion of piperazine (10.75g) & TsOH-$H_2O$ were added, followed by TsOH (1.1 g) (t=52°C). On complete addition the reaction was stirred at 50°C for 30 min.

Another flask (50mL) was charged with acetaldehyde (30.8g, 39mL, 0.7mol) and placed in an ice-water bath

(0-2°C). The acetaldehyde flask was connected to the 3-necked flask by cannula via septa. A stream of argon was then passed through the acetaldehyde flask at the rate which ensures that the addition of the acetaldehyde was complete within 3 h. After all acetaldehyde was added, the reaction was stirred for 30 min at 50°C, then cooled to 40°C and 50% glyoxylic acid (104g, 78mL) was added dropwise over 45 min at a rate to keep temperature below 50°C. Water (100mL) was then added and the reaction was heated at 70°C for 5 h. The reaction was cooled to 4°C in an ice-water bath. The precipitated product was filtered and filter cake was washed with cold water (100 mL). After drying in vacuo at 50°C 88.9g (71%) of desired product was obtained. Precipitate contains 78.5% of the title compound (HPLC assay).

**Example 9**

**Alternative preparation of 5,5'-(piperazine-1,4-diyl)bis(4-(2-methylprop-1-en-1-yl)furan-2(5*H*)-one) with simultaneous addition of both aldehydes**

Piperazine (236.6g) was charged to a clean 3L dry vessel fitted with thermometer, addition funnel (100mL) and reflux condenser. pTsOH (25.3g) was charged to vessel followed by acetonitrile (550mL). The agitator was started and the vessel was inerted with nitrogen. Isobutyraldehyde (150mL, 27% of the total charge) was charged to the agitated piperazine / pTsOH slurry and a temperature rise to ca 43°C was observed. The white suspension was then heated to 50°C (+/- 5°C). A pre-mixed chilled solution of isobutyraldehyde (400mL, 73% of total charge) and chilled acetaldehyde (260mL) were charged to a clean, dry 1 L vessel and this mixture was maintained in an ice bath. The isobutyraldehyde / acetaldehyde mixture was charged to the contents of the 3L vessel in 100mL aliquots over 5-6h at 50°C. The contents of the reaction flask changed from a white suspension to a wine red solution and finally an orange suspension during the addition of the acetaldehyde. After complete addition the suspension was agitated at 50°C for ca. 0.5 to 1.0h.

Aqueous glyoxylic acid (50% wt/wt) solution was charged to the suspension over 10-15 min and the temperature rose to ca 75°C. 5,5'-(Piperazine-1,4-diyl)bis(4-(2-methylprop-1-en-1-yl)furan-2(5H)-one) was observed to crystallise from solution. The suspension was stirred at 70°C (+/ 5°C) for 6h, then cooled with stirring to ambient. The batch was then cooled to -5 to 0°C for and held for 3-4h before the suspension was filtered and the cake washed with chilled methanol (1 x 500mL) then 2 x 500mL of methanol at ambient temperature. The washed product was dried in a vacuum oven at 40-50°C to constant weight to afford 474g of 98% pure 5,5'-(piperazine-1,4-diyl)bis(4-(2-methylprop-1-en-1-yl)furan-2(5*H*)-one) (70%).

**Example 10**

**Preparation of 5-hydroxy-4-(2-methylprop-1-en-1-yl)furan-2(5*H*)-one from 4-(2-methyl-1-propenyl)-5-morpholino-2(5*H*)-furanone or 5,5'-(piperazine-1,4-diyl)bis(4-(2-methylprop-1-en-1-yl)furan-2(5*H*)-one).**

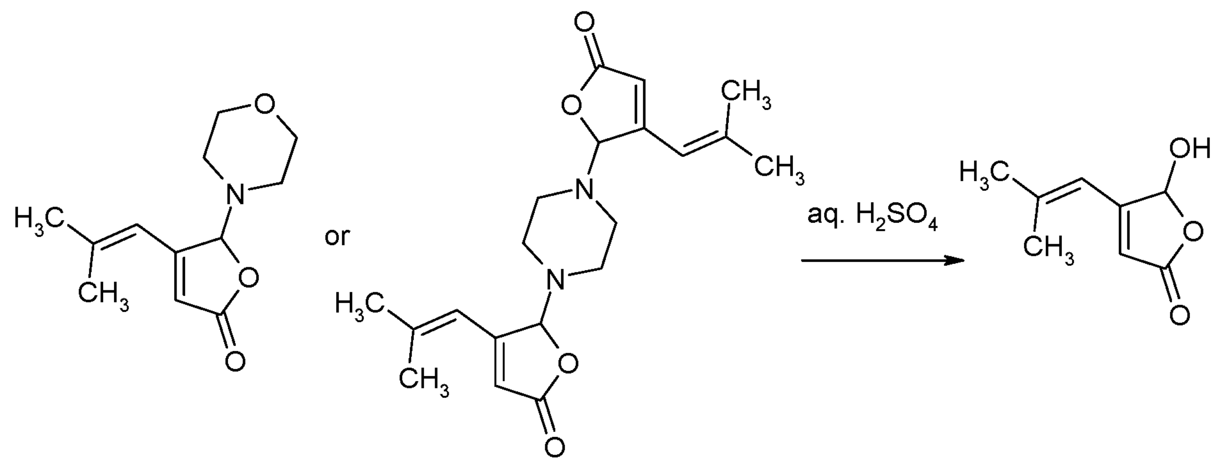


A 10% w/w aqueous sulphuric acid solution (110g) was charged to 4-(2-methyl-1-propenyl)-5-morpholino-2(5H)-furanone (20.0g, 0.896mol) and the mixture was stirred at reflux for 4h or until TLC (100:3 CPME: HOAc) indicated reaction completion. The mixture remained a suspension at all times. It was then cooled to 5°C, held for 2h, and filtered. The white solid was washed with water and dried to afford 5-hydroxy-4-(2-methylprop-1-en-1-yl)furan-2(5*H*)-one (12.9g, 93%).

Alternatively, a 10% w/w aqueous solution of sulphuric acid (412g) was charged to a vessel containing 5,5'-(piperazine-1,4-diyl)bis(4-(2-methylprop-1-en-1-yl)furan-2(5*H*)-one) (60g, 0.167mol). The contents were agitated and then heated to reflux. The reaction was held at reflux until the starting material was consumed, which was determined by dissolution. On completion of reaction the batch was cooled to 35°C at which point the target compound began to crystallise. The slurry was further cooled to 0-5°C and then transferred to a filter. The product was filtered, washed with water (2 x 100mL) and then dried under vacuo at <50°C, to give 5-hydroxy-4-(2-methylprop-1-en-1-yl)furan-2(5*H*)-one (42.0g, 81%).

GC/MS: m/z = 154

$^1$H NMR ($CDCl_3$): δ 6.10 (s, 1 H); 5.92 (s, 1 H); 5.88 (s, 1 H); 5.35 (bs, 1 H, O-H); 1.98 (s,3H); 1.93 (s, 3H). $^{13}$C NMR ($CDCl_3$): 172.8 (C), 161.4 (C), 152.3 (C), 115.3 (CH), 114.9 (CH), 99.5 (CH), 28.2 ($CH_3$), 21.4 ($CH_3$).

**Example 11**

**Preparation of 5-hydroxy-4-(2-methylprop-1-en-1-yl)furan-2(5H)-one in one pot from isobutyraldehyde and acetaldehyde.**

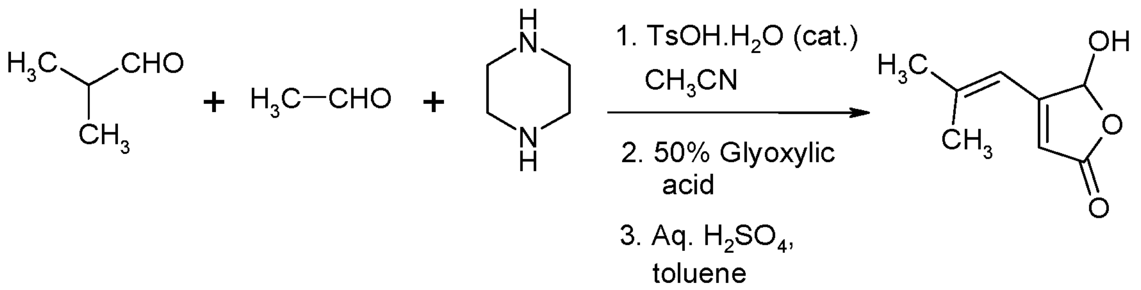


A mixture of piperazine (43.0g, 0.5 mol) and isobutyraldehyde (200mL) were refluxed under Dean-Stark until the theoretical amount of water (18mL) collected. The excess isobutyraldehyde was distilled off to leave a crystalline mass of 1,4 bis(2-methylpropen-1-yl)piperazine. This was dissolved in acetonitrile (1.2L) and extra isobutyraldehyde (100 mL, 1.0mol) added. Extra piperazine (4.4g) was added followed by p-toluenesulfonic acid (4.4g, 23.2mmol). The temperature was adjusted to 40°C and a solution of acetaldehyde (44g, 1.0mol) in acetonitrile (40mL) was added over 4h. The reaction mixture was stirred at 40°C for 1h and at ambient temperature for 4h. The acetonitrile was distilled off and the residue suspended in toluene (400mL). A mixture of 50% glyoxylic acid (148g) and water (150mL) was added over 0.5h. The mixture was then stirred at 45°C for 15h. There was a solid precipitate. Dilute sulphuric acid (10%, 1L) was added and the mixture refluxed for 3h. A biphasic mixture resulted. The toluene phase was separated. It contained ca 50% yield (based on acetaldehyde) of 5-hydroxy-4-(2-methylpropen-1-yl)-2-furanone by analysis

**Example 12**

**Preparation of 5-methoxy-4-(2-methylprop-1-en-1-yl)furan-2(5H)-one**

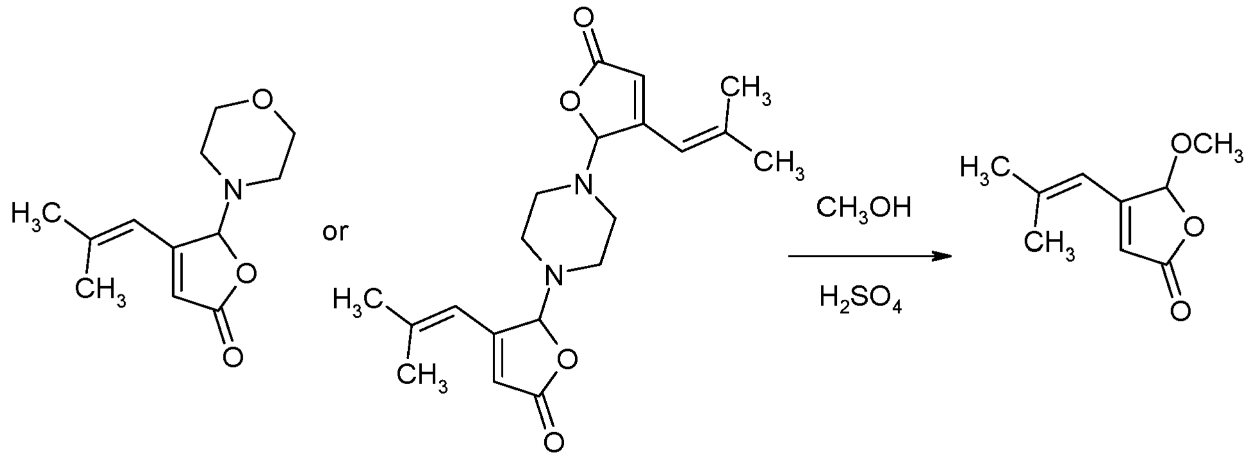


4-(2-Methyl-1-propenyl)-5-morpholino-2(5*H*)-furanone (7.70g, 34.5mmol) was stirred in MeOH (50mL) with $H_2SO_4$ (4mL, 2.2eq.) at reflux for 3h until disappearance of the starting material by GC. The reaction mixture was cooled to room temperature. The solvent was then evaporated *in vacuo* and the residue was diluted with EtOAc (50mL). The organic phase was washed with $H_2O$ (3x 50mL). Solvent was then evaporated in *vacuo* to give 5-methoxy-4-(2-methylprop-1-en-1-yl)furan-2(5H)-one as an orange oil (5.50g, 95%) which can be used without purification.

Alternatively, 5,5'-(piperazine-1,4-diyl)bis(4-(2-methylprop-1-en-1-yl)furan-2(5*H*)-one) (130g, 363mmol) and MeOH (690mL) were charged in 2L round bottom flask with mechanical agitation to form a suspension. Concentrated $H_2SO_4$ (43mL, 762mmol) was added dropwise with stirring at 20-25°C - a slight exotherm was observed, and nature of the solids present changed from dispersed and easily mixed to a thicker slurry. The reaction mixture was refluxed for 5h. After 0.5h complete dissolution (orange liquid) was observed. After 5h LCMS showed ~99% of the desired product. The mixture was cooled to ambient and left for crystallization of piperazine sulfate. The sediments were filtered, and filter cake was washed with ice cold MeOH (400mL). The filtrate was concentrated *in vacuo* (40°C bath temperature), and the residue was partitioned between water (50mL) and MTBE (300mL). Water layer was separated and additionally extracted (2x300mL of MTBE). Combined organic extracts were washed with saturated aq. $NaHCO_3$ (200mL). MTBE layer was dried over $Na_2SO_4$ with stirring for 1.5h, filtered and evaporated to give 5-methoxy-4-(2-methylprop-1-en-1-yl)furan-2(5H)-one as an orange oil (120g, 98%).

Distilled at 0.2-0.3 mbar vacuum with bp 100-105°C. GC/MS: m/z = 168; $^1$H NMR (400 MHz, $CDCl_3$) $\delta$ 5.59 (1 H, s) 5.88-5.86 (1 H, m) 5.75 (1H, d, J=0.6) 3.53 (3H, s) 2.01-2.00 (3H, m) 1.97-1.96 (3H, m). $^{13}$C NMR ($CDCl_3$): 171.4 (C), 159.2 (C), 151.9 (C), 115.8 (CH), 115.2 (CH), 104.4 (CH), 56.1 ($CH_3$), 28.2 ($CH_3$), 21.4 ($CH_3$).

The following compounds were prepared from 4-(2-methyl-1-propenyl)-5-morpholino-2(5*H*)-furanone according to the first of the above methods by replacing methanol with 3-methylbutanol (isoamyl alcohol), *n*-pentanol (amyl alcohol) or n-butanol:

## Example 12A

### 5-(3-Methylbutoxy)-4-(2-methylprop-1-en-1-yl)furan-2(5H)-one

Distilled at 0.4 mbar vacuum with bp 139-140°C; GC/MS: m/z = 224; $^1$H-NMR (400 MHz, $CDCl_3$) $\delta$ 5.94 (1 H, s) 5.87-5.84 (1 H, m) 5.80 (1 H, s) 3.86-3.80 (1H, m) 3.70-3.64 (1 H, m) 2.00 (3H, s) 1.96 (3H, s) 1.77-1.66 (1 H, m) 1.57-1.50 (2H, m) 0.93-0.91 (3H, m) 0.91-0.89 (3H, m)

## Example 12B

### 4-(2-Methylprop-1-en-1-yl)-5-pentyloxyfuran-2(5H)-one

Distilled at 0.08 mbar vacuum with bp 123-134°C; GC/MS: m/z = 224; $^1$H-NMR (400 MHz, $CDCl_3$) $\delta$ 5.94 (s, 1 H); 5.86 (s, 1 H); 5.80 (s, 1 H); 3.84-3.75 (m, 1 H); 3.68-3.59 (m, 1H); 2.01 (s, 3H); 1.95 (s, 3H); 1.69-1.59 (m, 2H); 1.38-1.29 (m, 4H); 0.95-0.85 (m, 3H)

**Example 12C**

**5-Butoxy-4-(2-methylprop-1-en-1-yl)furan-2(5H)-one**

Distilled at 0.05 mbar vacuum with bp 136-146°C; GC/MS: m/z = 210; $^1$H-NMR (400 MHz, $CDCl_3$) δ 5.94 (s, 1 H); 5.86 (s, 1 H); 5.80 (s, 1 H); 3.85-3.76 (m, 1 H); 3.69-3.60 (m, 1H); 2.00 (s, 3H); 1.95 (s, 3H); 1.68-1.58 (m, 2H); 1.45-1.32 (m, 2H); 0.93 (t, 3H, J=7.4 Hz)

**Example 13**

**Preparation of 5-methoxy-4-(2-methylpropyl)-dihydrofuran-2(3H)-one**

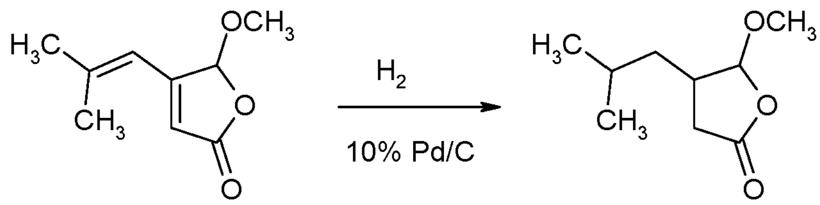


A solution of 5-methoxy-4-(2-methylprop-1-en-1-yl)furan-2(5H)-one (100 g, see example 12) in MTBE (700mL), 5g (5 wt%) of 10% Pd/C was charged to a hydrogenation vessel. 1 atm of hydrogen gas was introduced and pressure was kept constant during the reaction. After 7h at 20°C, the reaction was filtered through a celite pad (ø60mm, H=30mm) and rinsed with MTBE (3x50mL). The filtrate was washed with 1 M $NaHCO_3$ solution (200mL), water, brine and dried on $Na_2SO_4$ . After solvent removal 5-methoxy-4-(2-methylpropyl)-dihydrofuran-2(3H)-one was obtained as colourless liquid (98g, 96%).

GC/MS: m/z = 172
$^1$H NMR (400 MHz, $CDCl_3$) δ 5.25 (d, *J* = 5.0 Hz, 0.53 H, major isomer), 5.04 (d, *J* = 2.4 Hz, 0.26H, minor isomer), 3.48 (s, 0.92H, minor isomer), 3.46 (s, 1.66H, major isomer), 2.78 (dd, *J* = 17.7, 8.6 Hz, 0.33H), 2.59 - 2.42 (m, 1.27H, major isomer), 2.42-2.34 (m, 0.33H, minor isomer), 2.29 (dd, J = 16.7, 11.8 Hz, 0.63H, major isomer), 2.15 (dd, J = 17.7, 4.6 Hz, 0.33H, minor isomer), 1.67 - 1.31 (m, 2.63H both isomers), 1.28 - 1.19 (m, 0.33H, minor isomer), 0.95 - 0.86 (m, 6H both isomers). $^{13}$C NMR ($CDCl_3$): 177.7 (C, major isomer), 176.04 (C, minor isomer), 110.1 (CH, minor isomer), 106.1 (CH, major isomer), 57.0 ($CH_3$, minor isomer), 56.6 ($CH_3$, major isomer), 41.2 ($CH_2$, minor isomer), 39.1 ($CH_2$, minor isomer), 38.3 ($CH_2$, major isomer), 37.1 ($CH_2$, major isomer), 33.9 (CH, minor isomer), 32.8 (CH, major isomer), 26.0 (CH, major isomer), 25.8 (CH, minor isomer), 23.0 ($CH_3$, major isomer), 22.9 (minor isomer), 22.7 (major isomer), 22.6 (minor isomer).

Using the same general method and starting from the compounds of examples 12A, 12B and 12C respectively, the following compounds were also obtained:

**Example 13A**

**5-(3-Methylbutoxy)-4-(2-methylpropyl)-dihydrofuran-2(3H)-one**

GC/MS: m/z = 228; $^1$H-NMR (400 MHz, $CDCl_3$) δ 5.35 (0.86H, d, J=5.0 Hz) 5.13 (0.14H, d, J=2.7 Hz) 3.87-3.79 (m, 1H) 3.57-3.45 (m, 1H) 2.58-2.37 (2H, m) 2.35-2.27 (0.79H, m) 2.20-2.11 (0.21H, m) 1.74-1.62 (1H, m) 1.61-1.44 (4H, m) 1.42-1.31 (1 H, m) 0.95-0.86 (12H, m)

**Example 13B**

**4-(2-Methylpropyl)-5-pentyloxydihydrofuran-2(3H)-one**

GC/MS: m/z = 228; $^1$H-NMR (400 MHz, $CDCl_3$) δ 5.35 (0.77H, d, J=5.0 Hz) 5.13 (0.23H, d, J=2.6 Hz,) 3.83-3.75 (1 H, m) 3.55-3.40 (1 H, m) 2.59-2.37 (2H, m) 2.32 (0.73H, dd, J=16.6, 11.8 Hz) 2.15 (0.27H, dd, J=17.7, 5.0 Hz) 1.67-1.45 (4H, m) 1.41-1.25 (5H, m) 0.96-0.86 (9H, m)

**Example 13C**

**5-Butoxy-4-(2-methylpropyl)-dihydrofuran-2(3H)-one**

GC/MS: m/z = 214; $^1$H-NMR (400 MHz, $CDCl_3$) δ 5.35 (0.8H, d, J=5.0 Hz) 5.13 (0.2H, d, J=2.6 Hz,) 3.85-3.75 (1 H, m) 3.56-3.41 (1 H, m) 2.59-2.38 (2H, m) 2.31 (0.76H, dd, J=16.5, 11.7 Hz) 2.15 (0.24H, dd, J=17.6, 5.0 Hz) 1.66-1.45 (4H, m) 1.43-1.30 (3H, m) 0.95-0.87 (9H, m)

**Example 14**

**Preparation and hydrogenation of 5-(L-Menthyloxy)-4-(2-methyl-1-propenyl)-2(5*H*)-furanone**

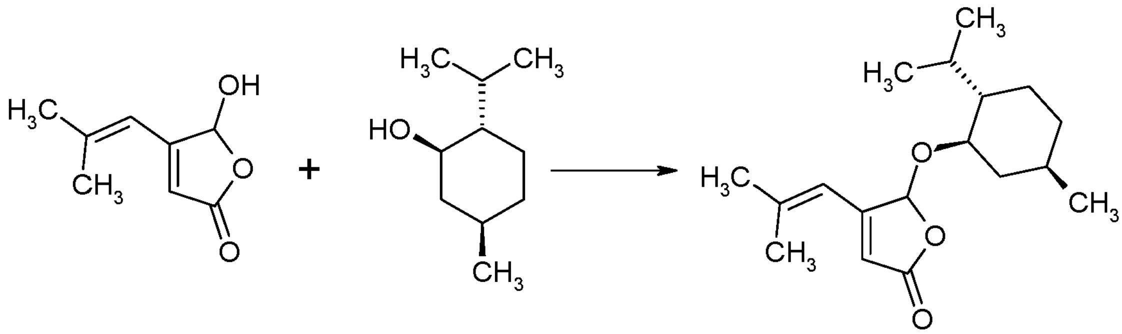

A mixture of 5-hydroxy-4-(2-methyl-1-propenyl)-2-furanone (59.5g, 0.386 mol), L-menthol (89.5g, 1.5 equivalents) and methanesulfonic acid (1.5g) was stirred at 70-80°C under vacuum (to remove water) for 100h. The liquid mass was poured (hot) into acetonitrile (450mL) and the title compound isolated by cooling, filtration and washing. The yield was 76.2g (67%). Suitable crystals for XRD were grown by slow evaporation of an acetone solution. The configuration at the acetal carbon was (R).

Hydrogenation of 5-(L-menthyloxy)-4-(2-methyl-1-propenyl)-2-furanone in ethyl acetate using the conditions of Example 13, gave the saturated derivative in quantitative yield. By $^1$H NMR, the compound was a 1:1 mixture of diastereomers.

**Example 15**

**Preparation of 5-acetoxy-4-(2-methylprop-1-en-1-yl)furan-2(5*H*)-one**

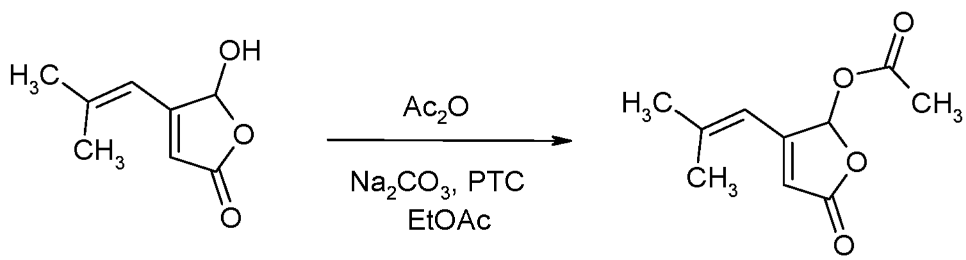


A suspension of 5-hydroxy-4-(2-methylprop-1-en-1-yl)furan-2(5*H*)-one (19g, 0.123mol) in ethyl acetate (100mL) was treated with solid sodium carbonate (13.1g, 0.123mol) and tetrabutylammonium hydrogensulfate (0.5g). Acetic anhydride (18.8g, 1.5eq.) was added in one portion. A mildly exothermic reaction ensued. The mixture was stirred overnight, water (100mL) added and the organic phase separated (pH of aqueous phase was 6.0). The ethyl acetate phase was water washed and concentrated to leave a solid (24.6g, 100%). This was pure by TLC (100:3 CPME: acetic acid). If this reaction is carried out in isopropyl acetate, the pure compound can be isolated by cooling the isopropyl acetate solution after the water washing in about 70% yield.

m/z: 196

**Example 16**

**Hydrogenation of 5-acetoxy-4-(2-methylprop-1-en-1-yl)furan-2(5*H*)-one**

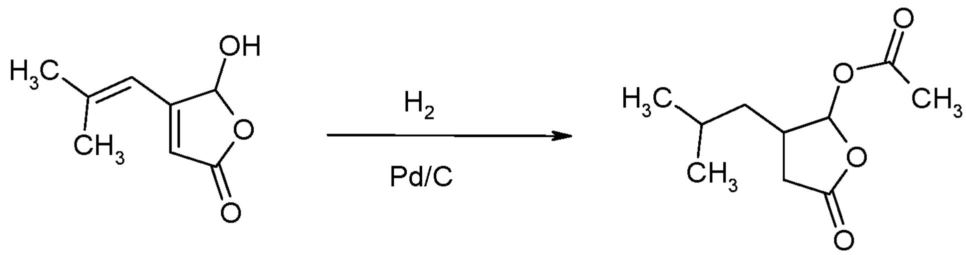


The material of example 34 was hydrogenated under similar conditions to those outlined in Example 13 to give a >90% yield of 5-acetoxy-4-(2-methylpropyl)-3,4-dihydro-2(5*H*)-furanone as a 1:1 mixture of diastereomers. The product is accompanied by ca. 5% of 4-(2-methylpropyl)-3,4-dihydrofuran-2(5*H*)-one. The product can be hydrolysed as in Example 30 to give yields of 5-hydroxy-4-(2-methylpropyl)-3,4-dihydro-5*H*-2-furanone/ 3-formyl-5-methylhexanoic acid.

**Example 17**

**Preparation of 5-hydroxy-4-(2-methylpropyl)dihydrofuran-2(3H)-one ($I^A$) by hydrogenation of 5-hydroxy-4-(2-methylprop-1-en-1-yl)furan-2(5H)-one in alkaline solution**

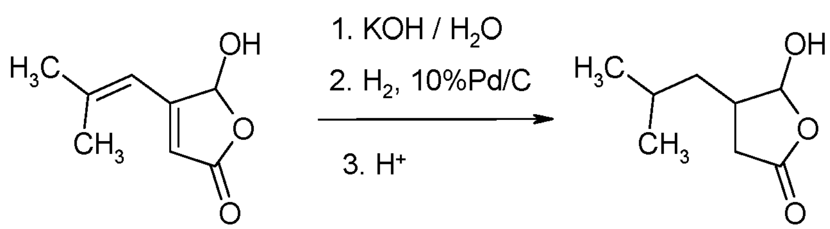


5-Hydroxy-4-(2-methylpropen-1-yl)-2-furanone (3.35g, 21.7mmol) and water (20mL) were charged to a hydrogenator. Potassium hydroxide (1.21 g, 1 eq) was then charged to the vessel and the contents were heated to 40°C until dissolution had occurred. 10% Pd/carbon catalyst (0.67g) was charged to the vessel and the reactor contents were then hydrogenated at 40°C and 5barg hydrogen pressure. On completion of reaction the reaction was cooled and the catalyst removed by filtration. The pH was adjusted to pH 2 by addition of 36% hydrochloric acid and the aqueous layer was washed with toluene to extract the desired product. The combined toluene extracts were concentrated to give the title compound (3.17g, 92%).

**Example 18**

**Hydrogenation of 5-hydroxy-4-(2-methylprop-1-en-1-yl)furan-2(5H)-one in neutral solution,**

5-Hydroxy-4-(2-methylpropen-1-yl)-2-furanone was hydrogenated in 6mL of water per gram starting material with 10% w/w of Pd/C catalyst. (22h, 40°C, 10 bar). On filtration, an oily phase separated from the water. This was a 1:1 mixture of 5-hydroxy-4-(2-methylpropyl)dihydrofuran-2(3H)-one ($I^A$) and 4-(2-methylpropyl)-dihydrofuran-2-one. Compound ($I^A$) can easily be separated pure by an acid base extraction. This reaction was repeated in organic solvents with 2-propanol giving relatively pure ($I^A$).

**Example 19**

**Preparation of 5-hydroxy-4-(2-methylpropyl)dihydrofuran-2(3H)-one ($I^A$) from 5,5'-(piperazine-1,4-diyl)bis(4-(2-methylprop-1-en-1-yl)furan-2(5*H*)-one) in one pot**

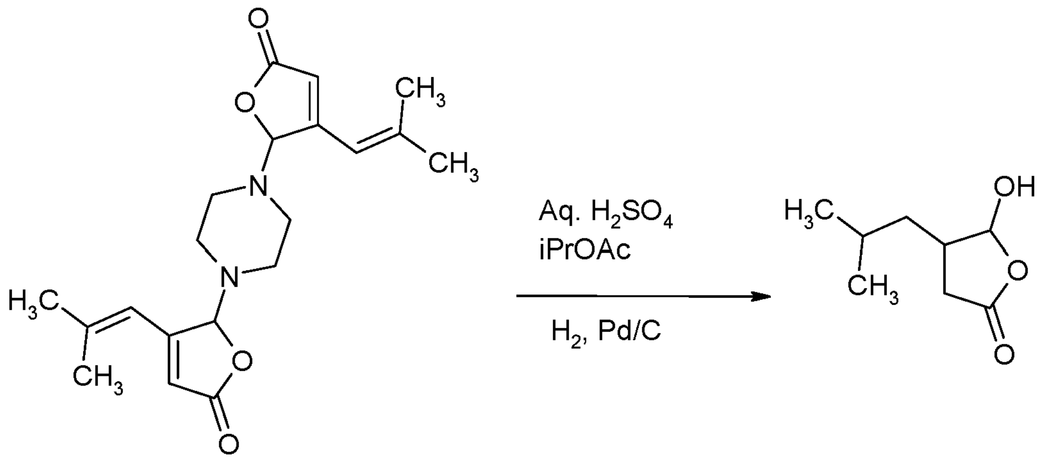


5,5'-(Piperazine-1,4-diyl)bis(4-(2-methylprop-1-en-1-yl)furan-2(5*H*)-one) (50.0g, 0.14mol) was charged to water (300mL) containing sulphuric acid (16.0g, 0.163mol). Isopropyl acetate (200mL) was added. 50% water wet 5% palladium on carbon (3.0g) was added and the mixture was hydrogenated (5bar hydrogen) at ambient temperature for 15h. The reaction mixture was filtered from catalyst, washed with isopropyl acetate (300mL) - this was also used to wash out the vessel. The organic phase was separated, washed with water (100mL) and concentrated on a rotary evaporator to afford 38.8g of clear oil. This was a mixture of desired compound (**$I^A$**) and the over-reduced lactone (4-(2-methylpropyl)-dihydrofuran-2-one). It was readily purified by extraction with aqueous potassium carbonate solution and wash with toluene. Acidification of the potassium carbonate extract with formic acid gave the desired product (**$I^A$**) (25.6 g, 58%). $^1H$ NMR ($D_2O$ with added $K_2CO_3$). δ 9.5 (1 H), 2.8 (m, 2H), 2.40 (m,2H), 1.55 (m, 2H), 1.30 (m, 2H), 0.95 (m, 6H).

**Example 20**

**Biotransformation of 3-formyl-5-methylhexanoic acid to pregabalin with recombinant ω-transaminases.**

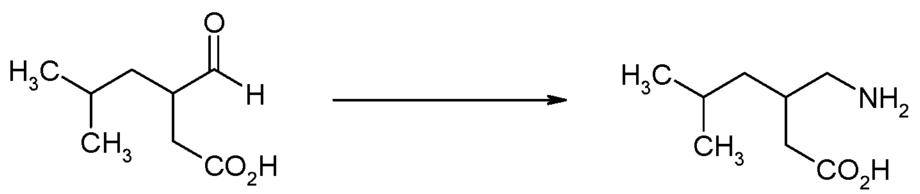

Transamination of 3-formyl-5-methylhexanoic acid to form Pregabalin was evaluated with various recombinant transaminases. Recombinant ω-transaminases from *Vibrio fluvialis, Rhodobacter sphaeroides,* and *Paracoccus denitrificans* were expressed in *E. coli* as follows: The pET28b ω-transaminase expression constructs were transformed into BL21(DE3) E. *coli* (Stratagene, Agilent Technologies, Santa Clara, CA, USA) as directed and overnight cultures were grown in LB + kanamycin media. The LB cultures were used to inoculate expression cultures grown in Overnight Express Instant TB + kanamycin Medium (Novagen, EMD Chemicals, Gibbstown, NJ, USA). Cultures were incubated for 20 h at 30°C, and the cells were harvested by centrifugation (4000 x g, 30 min, 4°C) and stored at -20°C.

Reactions (0.5mL) were carried out at 30°C in potassium phosphate buffer (100mM, pH 7.0), pyridoxal phosphate (2mM), isopropylamine (150mM), 3-formyl-5-methylhexanoic acid (50mM) and ω-transaminase (40mg wet cells/mL) from *Vibrio fluvialis, Rhodobacter sphaeroides,* or *Paracoccus denitrificans.* After 24h, reaction samples (0.1mL) were diluted with 0.4mL acetonitrile:water (1:1, v/v). Aliquots (0.05mL) of the diluted reaction samples were treated with

saturated aqueous sodium bicarbonate (0.01mL) and Marfey's reagent (*N*-α-(2,4-dinitro-5-fluorophenyl)alaninamide, 0.2mL of 5g/L solution in acetonitrile) at 40°C. After 1 h, the derivatization reactions were quenched with 0.01mL 1 N aqueous hydrochloric acid and diluted with 0.23mL of acetonitirle. The derivatized reaction samples were analyzed by UPLC (column: BEH C18, 50mm x 2.1mm id, gradient elution: 70% A:30% B to 55% A:45% B in 5 min (A = 1% triethylamine (pH 3 with phosphoric acid); B = acetonitrile), flow rate: 0.8mL/min, column temperature: 30°C, detection: 210-400nm) to give the results shown in Table 5.

**Table** 5

| Entry | Enzyme | Accession Number | %Yield Pregabalin | %ee (*S*)-Pregabalin |
|---|---|---|---|---|
| 1 | *Vibrio fluvialis* ω-transaminase | AEA39183 | 37.5 | 60.6 |
| 2 | *Rhodobacter sphaeroides* ω-transaminase | YP_001043908 | 24 | 84.4 |
| 3 | *Paracoccus denitrificans* ω-transaminase | YP_917746 | 44 | 60.6 |

**Example 21**

**Biotransformation of 3-formyl-5-methylhexanoic acid to pregabalin with recombinant ω-transaminases.**

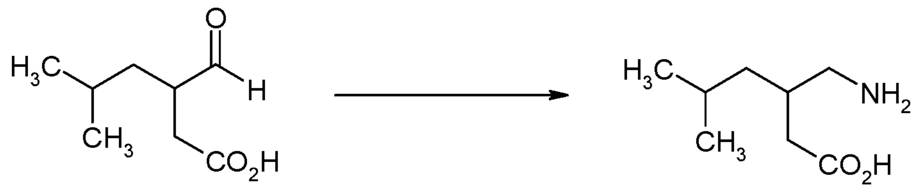

Recombinant variants of *Vibrio fluvialis* were tested for the reductive amination of 3-formyl-5-methylhexanoic acid to form Pregabalin. Variants of *V. fluvialis* ω-transaminase (Accession number AEA39183) were expressed in E. *coli* as follows: QuikChange Site-directed Mutagenesis kits from Stratagene (La Jolla, CA,USA) was used to create *V. fluvialis* aminotransferase variants as directed. Primers were ordered from Integrated DNA Technologies (Coralville, IA,USA). The pET28b ω-transaminase expression constructs were transformed into BL21(DE3) E. *coli* (Stratagene, Agilent Technologies, Santa Clara, CA, USA) as directed and overnight cultures were grown in LB + kanamycin media. The LB cultures were used to inoculate expression cultures grown in Overnight Express Instant TB + kanamycin Medium (Novagen, EMD Chemicals, Gibbstown, NJ, USA). Cultures were incubated for 20h at 30°C, and the cells were harvested by centrifugation (4000 x g, 30min, 4°C) and stored at -20°C.

Reactions (0.5mL) were carried out at 30°C in potassium phosphate buffer (100mM, pH 7.0) with pyridoxal phosphate (2mM), isopropylamine (300mM), 3-formyl-5-methylhexanoic acid (100mM) and *V. fluvialis* ω-transaminase wild-type or variants (40mg wet cells/mL). After 28h, reaction samples (0.1 mL) were diluted with 0.4mL acetonitrile:water (1:1, v/v). Aliquots (0.05mL) of the diluted reaction samples were treated with saturated aqueous sodium bicarbonate (0.01 mL) and Marfey's reagent (N-α-(2,4-dinitro-5-fluorophenyl)alaninamide, 0.2mL of 5g/L solution in acetonitrile) at 40°C. After 1 h, the derivatization reactions were quenched with 0.01 mL 1N aqueous hydrochloric acid and diluted 0.23mL of acetonitrile. The derivatized reaction samples were analyzed by UPLC (column: BEH C18, 50mm x 2.1mm id, gradient elution: 70% A:30% B to 55% A:45% B in 5min (A = 1% triethylamine (pH 3 with phosphoric acid); B = acetonitrile), flow rate: 0.8mL/min, column temperature: 30°C, detection: 210-400nm) to give the results shown in Table 6.

**Table 6**

| Entry | *V. fluvialis* ω-transaminase variant | % Conversion | % ee |
|---|---|---|---|
| 1 | W57F/K163L/R415F | 6.1 | 81.1 |
| 2 | Y165F | 9.5 | 75.3 |

(continued)

| Entry | *V. fluvialis* ω-transaminase variant | % Conversion | % ee |
|---|---|---|---|
| 3 | W147N | 10.0 | 75.0 |
| 4 | A228G | 32.3 | 74.9 |
| 5 | N166V | 16.6 | 74.5 |
| 6 | W57F/A228G | 33.3 | 73.9 |
| 7 | V156M | 13.2 | 70.3 |
| 8 | S159A | 7.9 | 70.2 |
| 9 | W57F/R415F | 10.7 | 68.5 |
| 10 | R415F | 11.8 | 68.1 |
| 11 | M59N | 10.1 | 68.0 |
| 12 | P301K | 19.1 | 67.6 |
| 13 | Y113F | 7.9 | 65.2 |
| 14 | F86G | 32.1 | 64.1 |
| 15 | 1254V | 32.1 | 64.0 |
| 16 | H326N | 15.9 | 63.8 |
| 17 | C414I | 10.1 | 63.0 |
| 18 | W57F/K163L/V153A | 46.1 | 41.2 |
| 19 | W57F/K163L | 43.5 | 36.1 |
| 20 | D21Y | 45.1 | 34.2 |
| 21 | W57F/D21Y | 46.3 | 34.0 |
| 22 | M294V | 42.4 | 33.2 |
| 23 | W57F/M294V | 41.2 | 32.5 |
| 24 | W57F/V177I | 39.9 | 29.0 |
| 25[1] | Vat 565 | 20.0 | 90.5 |
| 26[1] | Vfat665 | 15.0 | 93.1 |
| 27[1] | Vfat701 | 9.0 | 94.4 |
| 28[1] | Vfat707 | 7.5 | 95.7 |
| 29[1] | Vfat747 | 50.0 | 97.5 |
| 30[1] | Vfat825 | 75.0 | 95.7 |
| 31[1] | Vfat 850 | 81.3 | 94.6 |
| 32[1] | Vfat 875 | 80.5 | 95.1 |
| 33[1] | Vfat888[2] | 95.0 | 95.5 |
| Note - 1: Entry 25 thru 33 - reactions were performed using 400mM of 3-formyl-5-methylhexanoic acid , 3mM PLP, 800mM IPM at 45°C.<br>2: Vfat 888: | | | |

DNA SEQUENCE

ATGAATAAACCACAAAGCTGGGAAGCGCGTGCTGAAACTTACTCTCTGTAC
GGCTTCACTGATATGCCATCTCTGCACCAGCGTGGTACCGTGGTTGTCACC
CACGGCGAGGGCCCATACATCGTGGACGTCAACGGTCGCCGTTACCTGGA
CGCAAACTCCGGCCTGTACAATATGGTTGCCGGCTTCGACCACAAGGGTCT
GATCGACGCAGCAAAGGCCCAGTACGAACGCTTCCCGGGTTACCATAGCTT
CTTCGGTCGTATGTCTGATCAAACTGTTATGCTGAGCGAGAAACTGGTAGA
GGTGTCTCCATTCGACAGCGGTCGCGTGTTCTATACTAACTCCGGCTCCGA
GGCTAACGATACTATGGTGAAAATGCTGTGGTTTCTGCACGCCGCAGAGGG
CAAGCCGCAAAAACGCAAAATCCTGACTCGTAACAACGCATACCACGGTGT
AACTGCTGTTTCCGCTTCCATGACGGGTCTGCCGTACAACTCTGTATTCGG
CCTGCCGCTGCCGGGTTTCGTTCACCTGACCTGTCCGCACTATTGGCGTTA
CGGCGAAGAAGGTGAAACCGAAGAGCAGTTTGTTGCTCGTCTGGCCCGCG
AGCTGGAGGAAACTATCCAACGTGAAGGCGCGGACACGATTGCGGGCTTC
TTTGCTGAGCCGGTCATGGGCGCGGGCGGCGTAATCCCGCCGGCGAAAG
GTTACTTCCAGGCGATCCTGCCGATTCTGCGTAAGTACGACATCCCGGTTA
TCTCTGATGAAGTTATCTGCGGCTTTGGTCGTACCGGTAATACTTGGGGTTG
CGTTACCTATGACTTCACCCCGGATGCGATCATCTCCAGCAAAAATCTGACC
GCCGGTTTCTTTCCGGTTGGTGCTGTGATTCTGGGTCCGGAACTGGCGAAA
CGCCTGGAAACGGCGATCGAAGCTATCGAAGAGTTCCCGCACGGCTTTAC
GGCCAGCGGTCACCCGGTGGGTTGCGCTATCGCTCTGAAAGCAATCGATG
TTGTGATGAATGAGGGTCTGGCAGAGAACGTGCGCCGCCTGGCACCGCGT
TTTGAGGAGCGTCTGAAACACATTGCCGAACGTCCGAACATCGGTGAATAT
CGTGGCATCGGTTTTATGTGGGCACTGGAGGCTGTGAAAGACAAAGCATCT
AAAACCCCATTCGATGGTAATCTGTCTGTGAGCGAGCGTATCGCTAACACCT
GTACCGACCTGGGCCTGATCTGTAGCCCGATGGGTCAGTCCGTTATCCTGT
GCCCGCCGTTCATCCTGACCGAGGCGCAAATGGATGAGATGTTTGACAAAC
TGGAGAAGGCTCTGGACAAAGTCTTTGCGGAGGTGGCGTAA (SEQ ID NO. 10)

**AMINO ACID SEQUENCE**

MNKPQSWEARAETYSLYGFTDMPSLHQRGTVVVTHGEGPYIVDVNGRRYLDA
NSGLYNMVAGFDHKGLIDAAKAQYERFPGYHSFFGRMSDQTVMLSEKLVEVS
PFDSGRVFYTNSGSEANDTMVKMLWFLHAAEGKPQKRKILTRNNAYHGVTAV
SASMTGLPYNSVFGLPLPGFVHLTCPHYWRYGEEGETEEQFVARLARELEETI
QREGADTIAGFFAEPVMGAGGVIPPAKGYFQAILPILRKYDIPVISDEVICGFGRT
GNTWGCVTYDFTPDAIISSKNLTAGFFPVGAVILGPELAKRLETAIEAIEEFPHGF
TASGHPVGCAIALKAIDVVMNEGLAENVRRLAPRFEERLKHIAERPNIGEYRGIG
FMWALEAVKDKASKTPFDGNLSVSERIANTCTDLGLICSPMGQSVILCPPFILTE
AQMDEMFDKLEKALDKVFAEVA (SEQ ID NO. 2)

**Example 22**

**Alternative variants of *Vibrio fluvalis* ω-transaminase**

The following further recombinant variants of *Vibrio fluvialis* ω-Transaminase were expressed in *E. coli* as follows: The pET28b ω-transaminase expression constructs were transformed into BL21(DE3) *E. coli* (Stratagene, Agilent Technologies, Santa Clara, CA, USA) as directed and overnight cultures were grown in LB + kanamycin media. The LB cultures were used to inoculate expression cultures grown in Overnight Express Instant TB + kanamycin Medium (Novagen, EMD Chemicals, Gibbstown, NJ, USA). Cultures were incubated for 20 h at 30°C, and the cells were harvested by centrifugation (4000 x g, 30 min, 4°C) and stored at -20°C.

**Example 22a: Vfat906**

DNA Sequence:

ATGAATAAACCACAAAGCTGGGAAGCGCGTGCTGAAACTTACTCTCTGTACGGCTT
CACTGATATGCCATCTCTGCACgAGCGTGGTACCGTGGTTGTCACCCACGGCGAG
GGCCCATACATCGTGGACGTCAACGGTCGCCGTTACCTGGACGCAAACTCCGGCC
TGTACAATATGGTTGCCGGCTTCGACCACAAGGGTCTGATCGACGCAGCAAAGGC
CCAGTACGAACGCTTCCCGGGTTACCATAGCTTCTTCGGTCGTATGTCTGATCAAA
CTGTTATGCTGAGCGAGAAACTGGTAGAGGTGTCTCCATTCGACAGCGGTCGCGT
GTTCTATACTAACTCCGGCTCCGAGGCTAACGATACTATGGTGAAAATGCTGTGGT
TTCTGCACGCCGCAGAGGGCAAGCCGCAAAAACGCAAAATCCTGACTCGTaacAAC
GCATACCACGGTGTAACTGCTGTTTCCGCTTCCATGACGGGTCTGCCGTACAACTC
TGTATTCGGCCTGCCGCTGCCGGGTTTCGTTCACCTGACCTGTCCGCACTATTGG
CGTTACGGCGAAGAAGGTGAAACCGAAGAGCAGTTTGTTGCTCGTCTGGCCCGCG
AGCTGGAGGAAACTATCCAACGTGAAGGCGCGGACACGATTGCGGGCTTCTTTGC
TGAGCCGGTCATGGGCGCGGGCGGCGTAATCCCGCCGGCGAAAGGTTACTTCCA

GGCGATCCTGCCGATTCTGCGTAAGTACGACATCCCGGTTATCTCTGATGAAGTTATCTGCGGCTTTGGTCGTACCGGTAATACTTGGGGTTGCGTTACCTATGACTTCACCCCGGATGCGATCATCTCCAGCAAAAATCTGACCGCCGGTTTCTTTCCGGTTGGTGCTGTGATTCTGGGTCCGGAACTGAGCAAACGCCTGGAAACGGCGATCGAAGCTATCGAAGAGTTCCCGCACGGCTTTACGGCCggcGGTCACCCGGTGGGTTGCGCTATCGCTCTGAAAGCAATCGATGTTGTGATGAATGAGGGTCTGGCAGAGAACGTGCGCCGCCTGGCACCGCGTTTTGAGGAGCGTCTGAAACACATTGCCGAACGTCCGAACATCGGTGAATATCGTGGCATCGGTTTTATGTGGGCACTGGAGGCTGTGAAAGACAAAGCATCTAAAACCCCATTCGATGGTAATCTGTCTGTGAGCaaaCGTATCGCTAACACCTGTcagGACCTGGGCCTGATCTGTAGCgCGCTGGGTCAGTCCGTTATCCTGTGCCCGCCGTTCATCCTGACCGAGGCGCAAATGGATGAGATGTTTGACAAACTGGAGAAGGCTCTGGACAAAGTCTTTGCGGAGGTGGCGTAA (SEQ ID NO. 11)

Amino acid sequence:

MNKPQSWEARAETYSLYGFTDMPSLHERGTVVVTHGEGPYIVDVNGRRYLDANSGLYNMVAGFDHKGLIDAAKAQYERFPGYHSFFGRMSDQTVMLSEKLVEVSPFDSGRVFYTNSGSEANDTMVKMLWFLHAAEGKPQKRKILTRNNAYHGVTAVSASMTGLPYNSVFGLPLPGFVHLTCPHYWRYGEEGETEEQFVARLARELEETIQREGADTIAGFFAEPVMGAGGVIPPAKGYFQAILPILRKYDIPVISDEVICGFGRTGNTWGCVTYDFTPDAIISSKNLTAGFFPVGAVILGPELSKRLETAIEAIEEFPHGFTAGGHPVGCAIALKAIDVVMNEGLAENVRRLAPRFEERLKHIAERPNIGEYRGIGFMWALEAVKDKASKTPFDGNLSVSKRIANTCQDLGLICSALGQSVILCPPFILTEAQMDEMFDKLEKALDKVFAEVA (SEQ ID NO. 3)

**Example 22b: Vfat999**

DNA Sequence:

ATGAATAAACCACAAAGCTGGGAAGCGCGTGCTGAAACTTACTCTCTGTACGGCTT
CACTGATATGCCATCTCTGCACGAGCGTGGTACCGTGGTTGTCACCCACGGCGAG
GGCCCATACGTGGTGGACGTCAACGGTCGCCGTTACCTGGACGCAAACTCCGGC
CTGTACAATATGGTTGCCGGCTTCGACCACAAGGGTCTGATCGACGCAGCAAAGG
CCCAGTACGAACGCTTCCCGGGTTACCATAGCTTCTTCGGTCGTATGTCTGATCAA
ACTGTTATGCTGAGCGAGAAACTGGTAGAGGTGTCTCCATTCGACAGCGGTCGCG
TGTTCTATACTAACTCCGGCTCCGAGGCTAACGATACTATGGTGAAAATGCTGTGG
TTTCTGCACGCCGCAGAGGGCAAGCCGCAAAAACGCAAAATCCTGACTCGTCAAA
ACGCATACCACGGTGTAACTGCTGTTTCCGCTTCCATGACGGGTCTGCCGCACAA
CTCTGTATTCGGCCTGCCGCTGCCGGGTTTCGTTCACCTGACCTGTCCGCACTATT
GGCGTTACGGCGAAGAAGGTGAAACCGAAGAGCAGTTTGTTGCTCGTCTGGCCCG
CGAGCTGGAGGAAACTATCCAACGTGAAGGCGCGGACACGATTGCGGGCTTCTTT
GCTGAGCCGGTCATGGGCGCGGGCGGCGTAATCCCGCCGGCGAAAGGTTACTTC
CAGGCGATCCTGCCGATTCTGCGTAAGTACGACATCCCGGTTATCTCTGATGAAGT
TATCTGCGGCTTTGGTCGTACCGGTAATACTTGGGGTTGCGTTACCTATGACTTCA
CCCCGGATGCGATCATCTCCAGCAAAAATCTGACCGCCGGTTTCTTTCCGGTTGGT
GCTGTGATTCTGGGTCCGGAACTGAGCAAACGCCTGGAAACGGCGATCGAAGCTA
TCGAAGAGTTCCCGCACGGCTTTACGGCCGGCGGTCACCCGGTGGGTTGCGCTA
TCGCTCTGAAAGCAATCGATGTTGTGATGAATGAGGGTCTGGCAGAGAACGTGCG
CCGCCTGGCACCGCGTTTTGAGGAGCGTCTGAAACACATTGCCGAACGTCCGAAC
ATCGGTGAATATCGTGGCATCGGTTTTATGTGGGCACTGGAGGCTGTGAAAGACA
AAGCATCTAAAACCCCATTCGATGGTAATCTGTCTGTGAGCAAACGTATCGCTAAC
ACCTGTCAGGACCTGGGCCTGATCTGTAGCGCGCTGGGTCAGTCCGTTATCCTGA
GCCCGCCGTTCATCCTGACCGAGGCGCAAATGGATGAGATGTTTGACAAACTGGA
GAAGGCTCTGGACAAAGTCTTTGCGGAGGTGGCGTAA (SEQ ID NO. 12)

Amino acid sequence:

MNKPQSWEARAETYSLYGFTDMPSLHERGTVVVTHGEGPYVVDVNGRRYLDANSGL YNMVAGFDHKGLIDAAKAQYERFPGYHSFFGRMSDQTVMLSEKLVEVSPFDSGRVFY TNSGSEANDTMVKMLWFLHAAEGKPQKRKILTRQNAYHGVTAVSASMTGLPHNSVFG LPLPGFVHLTCPHYWRYGEEGETEEQFVARLARELEETIQREGADTIAGFFAEPVMGA GGVIPPAKGYFQAILPILRKYDIPVISDEVICGFGRTGNTWGCVTYDFTPDAIISSKNLTA GFFPVGAVILGPELSKRLETAIEAIEEFPHGFTAGGHPVGCAIALKAIDVVMNEGLAENV RRLAPRFEERLKHIAERPNIGEYRGIGFMWALEAVKDKASKTPFDGNLSVSKRIANTCQ DLGLICSALGQSVILSPPFILTEAQMDEMFDKLEKALDKVFAEVA (SEQ ID NO. 4)

**Example 22c: Vfat1010**

DNA Sequence:

ATGAATAAACCACAAAGCTGGGAAGCGCGTGCTGAAACTTACTCTCTGTACGGCTT CACTGATATGCCATCTCTGCACGAGCGTGGTACCGTGGTTGTCACCCACGGCGAG GGCCCATACATCGTGGACGTCAACGGTCGCCGTTACCTGGACGCAAACTCCGGCC TGTACAATATGGTTGCCGGCTTCGACCACAAGGGTCTGATCGACGCAGCAAAGGC CCAGTACGAACGCTTCCCGGGTTACCATAGCTTCTTCGGTCGTATGTCTGATCAAA CTGTTATGCTGAGCGAGAAACTGGTAGAGGTGTCTCCATTCGACAGCGGTCGCGT GTTCTATACTAACTCCGGCTCCGAGGCTAACGATACTATGGTGAAAATGCTGTGGT

TTCTGCACGCCGCAGAGGGCAAGCCGCAAAAACGCAAAATCCTGACTCGTCAAAA
CGCATACCACGGTGTAACTGCTGTTTCCGCTTCCATGACGGGTATGCCGCACAAC
TCTGTATTCGGCCTGCCGCTGCCGGGTTTCGTTCACCTGACCTGTCCGCACTATTG
GCGTTACGGCGAAGAAGGTGAAACCGAAGAGCAGTTTGTTGCTCGTCTGGCCCGC
GAGCTGGAGGAAACTATCCAACGTGAAGGCGCGGACACGATTGCGGGCTTCTTTG
CTGAGCCGGTCATGGGCGCGGGCGGCGTAATCCCGCCGGCGAAAGGTTACTTCC
AGGCGATCCTGCCGATTCTGCGTAAGTACGACATCCCGGTTATCTCTGATGAAGTT
ATCTGCGGCTTTGGTCGTACCGGTAATACTTGGGGTTGCGTTACCTATGACTTCAC
CCCGGATGCGATCATCTCCAGCAAAAATCTGACCGCCGGTTTCTTTCCGGTTGGT
GCTGTGATTCTGGGTCCGGCACTGAGCAAACGCCTGGAAACGGCGATCGAAGCTA
TCGAAGAGTTCCCGCACGGCTTTACGGCCGGCGGTCACCCGGTGGGTTGCGCTA
TCGCTCTGAAAGCAATCGATGTTGTGATGAATGAGGGTCTGGCAGAGAACGTGCG
CCGCCTGGCACCGCGTTTTGAGGAGCGTCTGAAACACATTGCCGAACGTCCGAAC
ATCGGTGAATATCGTGGCATCGGTTTTATGTGGGCACTGGAGGCTGTGAAAGACA
AAGCATCTAAAACCCCATTCGATGGTAATCTGTCTGTGAGCAAACGTATCGCTAAC
ACCTGTCAGGACCTGGGCCTGATCTGTAGCGCGATGGGTCAGTCCGTTATCCTGA
GCCCGCCGTTCATCCTGACCGAGGCGCAAATGGATGAGATGTTTGACAAACTGGA
GAAGGCTCTGGACAAAGTCTTTGCGGAGGTGGCGTAA (SEQ ID NO. 13)

Amino acid sequence:

MNKPQSWEARAETYSLYGFTDMPSLHERGTVVVTHGEGPYIVDVNGRRYLDANSGLY
NMVAGFDHKGLIDAAKAQYERFPGYHSFFGRMSDQTVMLSEKLVEVSPFDSGRVFYT
NSGSEANDTMVKMLWFLHAAEGKPQKRKILTRQNAYHGVTAVSASMTGMPHNSVFG
LPLPGFVHLTCPHYWRYGEEGETEEQFVARLARELEETIQREGADTIAGFFAEPVMGA
GGVIPPAKGYFQAILPILRKYDIPVISDEVICGFGRTGNTWGCVTYDFTPDAIISSKNLTA
GFFPVGAVILGPALSKRLETAIEAIEEFPHGFTAGGHPVGCAIALKAIDVVMNEGLAENV
RRLAPRFEERLKHIAERPNIGEYRGIGFMWALEAVKDKASKTPFDGNLSVSKRIANTCQ
DLGLICSAMGQSVILSPPFILTEAQMDEMFDKLEKALDKVFAEVA (SEQ ID NO. 5)

**Example 22d: Vfat1020**

DNA Sequence:

ATGAATAAACCACAAAGCTGGGAAGCGCGTGCTGAAACTTACTCTCTGTACGGCTT
CACTGATATGCCATCTCTGCACGAGCGTGGTACCGTGGTTGTCACCCACGGCGAG
GGCCCATACGTGGTGGACGTCAACGGTCGCCGTTACCTGGACGCAAACTCCGGC
CTGTACAATATGGTTGCCGGCTTCGACCACAAGGGTCTGATCGACGCAGCAAAGG
CCCAGTACGAACGCTTCCCGGGTTACCATAGCTTCTTCGGTCGTATGTCTGATCAA
ACTGTTATGCTGAGCGAGAAACTGGTAGAGGTGTCTCCATTCGACAGCGGTCGCG
TGTTCTATACTAACTCCGGCTCCGAGGCTAACGATACTATGGTGAAAATGCTGTGG
TTTCTGCACGCCGCAGAGGGCAAGCCGCAAAAACGCAAAATCCTGACTCGTCAAA
ACGCATACCACGGTGTAACTGCTGTTTCCGCTTCCATGACGGGTCTGCCGCACAA
CTCTGTATTCGGCCTGCCGCTGCCGGGTTTCGTTCACCTGGGTTGTCCGCACTATT
GGCGTTACGGCGAAGAAGGTGAAACCGAAGAGCAGTTTGTTGCTCGTCTGGCCCG
CGAGCTGGAGGAAACTATCCAACGTGAAGGCGCGGACACGATTGCGGGCTTCTTT
GCTGAGCCGGTCATGGGCGCGGGCGGCGTAATCCCGCCGGCGAAAGGTTACTTC
CAGGCGATCCTGCCGATTCTGCGTAAGTACGACATCCCGGTTATCTCTGATGAAGT
TATCTGCGGCTTTGGTCGTACCGGTAATACTTGGGGTTGCGTTACCTATGACTTCA
CCCCGGATGCGATCATCTCCAGCAAAAATCTGACCGCCGGTTTCTTTCCGGTTGGT
GCTGTGATTCTGGGTCCGGAACTGAGCAAACGCCTGGAAACGGCGATCGAAGCTA
TCGAAGAGTTCCCGCACGGCTTTACGGCCGGCGGTCACCCGGTGGGTTGCGCTA
TCGCTCTGAAAGCAATCGATGTTGTGATGAATGAGGGTCTGGCAGAGAACGTGCG
CCGCCTGGCACCGCGTTTTGAGGAGCGTCTGAAACACATTGCCGAACGTCCGAAC
ATCGGTGAATATCGTGGCATCGGTTTTATGTGGGCACTGGAGGCTGTGAAAGACA
AAGCATCTAAAACCCCATTCGATGGTAATCTGTCTGTGAGCAAACGTATCGCTAAC
ACCTGTCAGGACCTGGGCCTGATCTGTAGCGCGATGGGTCAGTCCGTTATCCTGA
GCCCGCCGTTCATCCTGACCGAGGCGCAAATGGATGAGATGTTTGACAAACTGGA
GAAGGCTCTGGACAAAGTCTTTGCGGAGGTGGCGTAA (SEQ ID NO. 14)

Amino acid sequence:

MNKPQSWEARAETYSLYGFTDMPSLHERGTVVVTHGEGPYVVDVNGRRYLDANSGL YNMVAGFDHKGLIDAAKAQYERFPGYHSFFGRMSDQTVMLSEKLVEVSPFDSGRVFY TNSGSEANDTMVKMLWFLHAAEGKPQKRKILTRQNAYHGVTAVSASMTGLPHNSVFG LPLPGFVHLGCPHYWRYGEEGETEEQFVARLARELEETIQREGADTIAGFFAEPVMGA GGVIPPAKGYFQAILPILRKYDIPVISDEVICGFGRTGNTWGCVTYDFTPDAIISSKNLTA GFFPVGAVILGPELSKRLETAIEAIEEFPHGFTAGGHPVGCAIALKAIDVVMNEGLAENV RRLAPRFEERLKHIAERPNIGEYRGIGFMWALEAVKDKASKTPFDGNLSVSKRIANTCQ DLGLICAAMGQSVILSPPFILTEAQMDEMFDKLEKALDKVFAEVA (SEQ ID NO. 6)

**Example 22e: Vfat1030**

DNA Sequence:

ATGAATAAACCACAAAGCTGGGAAGCGCGTGCTGAAACTTACTCTCTGTACGGCTTCACTGATATGCCATCTCTGCACGAGCGTGGTACCGTGGTTGTCACCCACGGCGAGGGCCCATACATCGTGGACGTCCACGGTCGCCGTTACCTGGACGCAAACTCCGGCCTGTACAATATGGTTGCCGGCTTCGACCACAAGGGTCTGATCGACGCAGCAAAGGCCCAGTACGAACGCTTCCCGGGTTACCATAGCTTCTTCGGTCGTATGTCTGATCAAACTGTTATGCTGAGCGAGAAACTGGTAGAGGTGTCTCCATTCGACAGCGGTCGCGTGTTCTATACTAACTCCGGCTCCGAGGCTAACGATACTATGGTGAAAATGCTGTGGTTTCTGCACGCCGCAGAGGGCAAGCCGCAAAAACGCAAAATCCTGACTCGTCAAAACGCATACCACGGTGTAACTGCTGTTTCCGCTTCCATGACGGGTCTGCCGCACAACTCTGTATTCGGCCTGCCGCTGCCGGGTTTCGTTCACCTGAGCTGTCCGCACTATTGGCGTTACGGCGAAGAAGGTGAAACCGAAGAGCAGTTTGTTGCTCGTCTGGCCCGCGAGCTGGAGGAAACTATCCAACGTGAAGGCGCGGACACGATTGCGGGCTTCTTTGCTGAGCCGGTCATGGGCGCGGGCGGCGTAATCCCGCCGGCGAAAGGTTACTTCCAGGCGATCCTGCCGATTCTGCGTAAGTACGACATCCCGGTTATCTCTGATGAAGTTATCTGCGGCTTTGGTCGTACCGGTAATACTTGGGGTTGCGTTACCTATGACTTCACCCCGGATGCGATCATCTCCAGCAAAAATCTGACCGCCGGTTTCTTTCCGGTTGGTGCTGTGATTCTGGGTCCGGAACTGAGCAAACGCCTGGAAACGGCGATCGAAGCTATCGAAGAGTTCCCGCACGGCTTTACGGCCGGCGGTCACCCGGTGGGTTGCGCTATCGCTCTGAAAGCAATCGATGTTGTGATGAATGAGGGTCTGGCAGAGAACGTGCGCCGCCTGGCACCGCGTTTTGAGGAGCGTCTGAAACACATTGCCGAACGTCCGAACATCGGTGAATATCGTGGCATCGGTTTTATGTGGGCACTGGAGGCTGTGAAAGACAAAGCATCTAAAACCCCATTCGATGGTAATCTGTCTGTGAGCAAACGTATCGCTAACACCTGTCAGGACCTGGGCCTGATCTGTAGCGCGATGGGTCAGTCCGTTATCCTGAGCCCGCCGTTCATCCTGACCGAGGCGCAAATGGATGAGATGTTTGACAAACTGGAGAAGGCTCTGGACAAAGTCTTTGCGGAGGTGGCGTAA (SEQ ID NO. 15)

Amino acid sequence:

MNKPQSWEARAETYSLYGFTDMPSLHERGTVVVTHGEGPYIVDVHGRRYLDANSGLY NMVAGFDHKGLIDAAKAQYERFPGYHSFFGRMSDQTVMLSEKLVEVSPFDSGRVFYT NSGSEANDTMVKMLWFLHAAEGKPQKRKILTRQNAYHGVTAVSASMTGLPHNSVFGL PLPGFVHLSCPHYWRYGEEGETEEQFVARLARELEETIQREGADTIAGFFAEPVMGAG GVIPPAKGYFQAILPILRKYDIPVISDEVICGFGRTGNTWGCVTYDFTPDAIISSKNLTAG FFPVGAVILGPELSKRLETAIEAIEEFPHGFTAGGHPVGCAIALKAIDVVMNEGLAENVR RLAPRFEERLKHIAERPNIGEYRGIGFMWALEAVKDKASKTPFDGNLSVSKRIANTCQD LGLICSAMGQSVILSPPFILTEAQMDEMFDKLEKALDKVFAEVA (SEQ ID NO. 7)

**Example 23**

**Preparation of (S)-Pregabalin via hydrolysis of 5-methoxy-4-(2-methylpropyl)-dihydrofuran-2(3H)-one and enzymatic transamination**

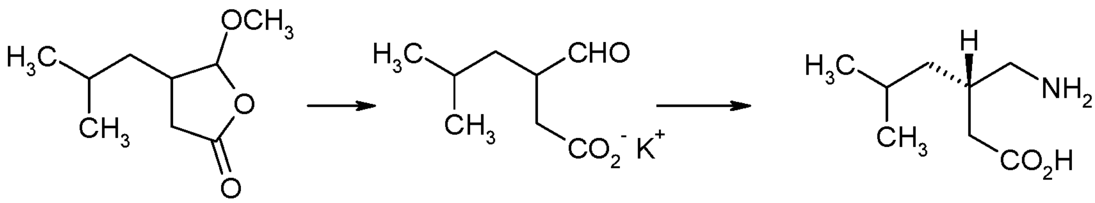

5-Methoxy-4-(2-methylpropyl)-dihydrofuran-2(3H)-one (2.58g, 15mmol, see example 13) was suspended in DIW (5.2g) and cooled in an ice/water bath. Aq KOH (50% w/w, 1.77g, 1.05eq) was added dropwise *via* syringe over 5mins. The reaction was removed from the ice/water bath and stirred at rt for 90mins. The pH was adjusted to 7.0 using formic acid. The reaction mixture was then used as feedstock in the subsequent transaminase reaction.

Transaminase enzyme solution (12.5g), PLP (35mg), DIW (15mL) and 4.OM isopropylamine.HCl aq soln (7.5mL, 30mmol) were charged to a 100mL flask and warmed to 45°C. The hydrolysis reaction was added in one portion followed by DIW (6 mL, used as a vessel rinse). The pH of the reaction was adjusted to 7.25 using neat isopropylamine and the reaction was stirred at 45°C until reaction completion. The reaction mixture was then heated to an internal temperature of 55°C and the pH adjusted to 4.0 using 95% formic acid. Darco carbon (125mg) was added and the mixture was allowed to cool to room temperature before cooling on ice/water for 20 minutes. The mixture was then filtered through Whatman paper no. 3. The filtrate was concentrated to 1/3 of its weight and then heated to 55°C. The pH of the solution was then adjusted to pH 7.5 using 50% KOH after which the solution was cooled to ambient and then to 0-5°C in an ice/water bath. Precipitation of product was observed in the cooldown. The slurry was filtered and washed with DIW/EtOH (10mL, 1:1, 0°C). The white precipitate was dried for 12 hours in a vacuum oven (45°C) to yield (S)-Pregabalin in 61% yield, 98.6% w/w purity and 99. 8% ee (preferred S-isomer).

**Example 24**

**Preparation of (S)-Pregabalin via hydrolysis of 5-butoxy-4-(2-methylpropyl)-dihydrofuran-2(3H)-one and enzymatic transamination.**

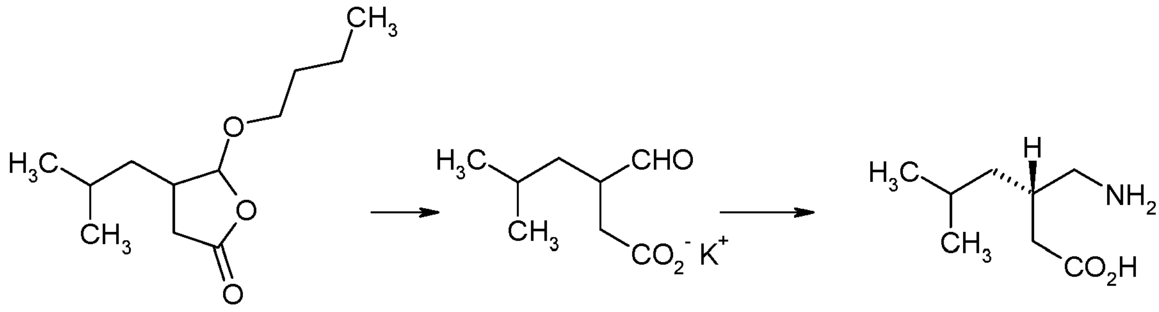

5-Butoxy-4-(2-methylpropyl)-dihydrofuran-2(3H)-one (3.21 g, 15mmol, see example 13C) was suspended in DIW (8.5g) and cooled in an ice/water bath. Aq KOH (50% w/w, 2.02g, 1.2eq) was added dropwise via syringe over 5mins. The reaction was removed from the ice/water bath and stirred at rt for 90mins. The reaction pH was then adjusted to pH 7.0 using formic acid, and the reaction mixture was then used as feedstock in the subsequent transaminase reaction.

Transaminase enzyme solution (12.5g), PLP (35mg), DIW (15mL) and 4.0M isopropylamine.HCl solution (7.5mL, 30mmol) were charged to a 100mL flask and warmed to 45°C. The hydrolysis reaction (see above) was added in one portion followed by DIW (6mL, used as a vessel rinse). The pH of the reaction was adjusted to 7.25 using neat isopropylamine and the reaction was stirred at 45°C.

The reaction mixture was heated to an internal temperature of 55°C and pH adjusted to 4.0 using 95% formic acid. Darco carbon (125mg) was added and the mixture was allowed to cool to room temperature before being cooled on ice/water for 20 minutes. The mixture was then filtered through Whatman paper no 3. The filtrate was concentrated to 1/3 of its weight then heated to 55°C. The pH of the solution was then adjusted to pH 7.5 using 50% KOH after which the solution was cooled to ambient and then to 0-5°C in an ice/water bath. Precipitation of product was observed in the cooldown. The slurry was filtered and washed with DIW/EtOH (10mL, 1:1, 0°C). The white precipitate was dried for 12 hours in a vacuum oven (45°C) to yield (S)-Pregabalin in 51% yield, 98.4% w/w purity and 99.9% e.e preferred S-isomer.

## Example 25

## Preparation of (S)-Pregabalin from 5-hydroxy-4-(2-methylpropyl)-dihydrofuran-2(3H)-one via enzymatic transamination

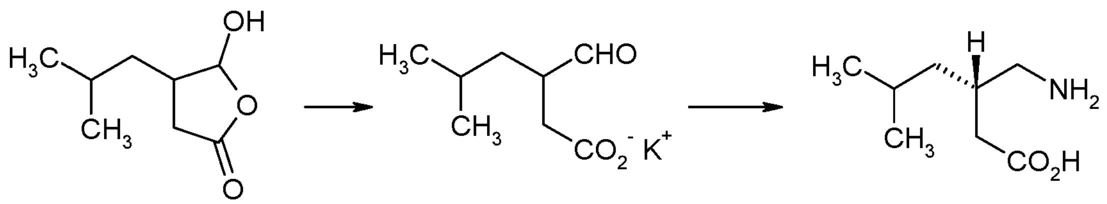

5-Hydroxy-4-(2-methylpropyl)-dihydrofuran-2(3H)-one (2.0g, 12.5mmol) was suspended in DIW (8.5g) and cooled in an ice/water bath. Potassium carbonate (0.863g, 6.3mmol) was added portion wise over the course of 5mins. The reaction was removed from the ice/water bath and stirred at rt for 90mins. The pH was adjusted to 7.0 using formic acid and the reaction mixture was then used as feedstock in the subsequent transaminase reaction.

Transaminase enzyme solution (10.4g), PLP (30mg), DIW (12.5mL) and aq 4.0M isopropylamine.HCl aq soln (6.3mL, 30mmol) were charged to a 100mL flask and warmed to 45°C. The hydrolysis reaction was added in one portion followed by DIW (5mL, used as a vessel rinse). The pH of the reaction was adjusted to 7.25 using neat isopropylamine and the reaction was stirred at 45°C. The reaction mixture was heated to an internal temperature of 55°C and adjusted to pH 4.0 using 95% formic acid. Darco carbon (125mg) was added and the mixture was allowed to cool to room temperature before being cooled on ice/water for 20 minutes. The mixture was then filtered through Whatman paper no 3. The filtrate was concentrated to 1/3 of its weight then heated to 55°C. The pH of the solution was then adjusted to pH 7.5 using 50% KOH after which the solution was cooled to ambient and then to 0-5°C in an ice/water bath. Precipitation of product was observed in the cooldown. The slurry was filtered and washed with DIW/EtOH (10mL, 1:1, 0°C). The white precipitate was dried for 12 hours in a vacuum oven (45°C) to yield (S)-Pregabalin in 61% yield, 98.3% w/w purity and 99.9% e.e of the preferred S-isomer.

### Example 26

**Preparation of (R/S)-3-aminomethyl-5-methylhexanoic acid via Reductive amination of 5-hydroxy-4-(2-methylprop-1-en-1-yl)furan-2(*5H*)-one**

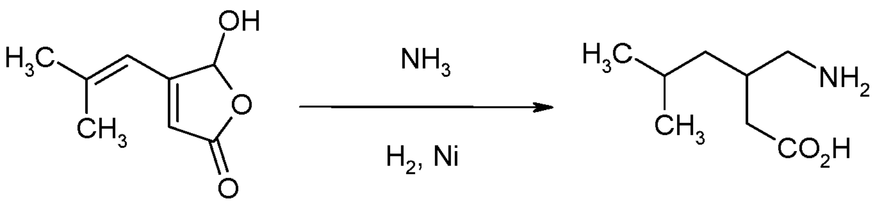


A 0.01M solution of 5-hydroxy-4-(2-methylprop-1-en-1-yl)furan-2(5H)-one in 30% aqueous ammonia solution was hydrogenated (10 bar, ambient temperature) for 48h in the presence of 10mol% Raney Nickel catalyst. The catalyst was filtered and the solution concentrated to leave a solid. The product was isolated by addition of hydrochloric acid to a methanol suspension and found to be pure 3-aminomethyl-5-methylhexanoic acid hydrochloride.

The use of palladium as a catalyst gave mixtures of 3-aminomethyl-5-methylhexanoic acid and the corresponding secondary amine, 3-[(2-carboxymethyl-4-methyl-pentylamino)-methyl]-5-methyl-hexanoic acid.

**SEQUENCE LISTING**

**SEQ ID NO. 1**

Generic Vfat aa sequence

MNKPQSWEARAETYSLYGFTDMPSLH$\mathbf{X}^{27}$RGTVVVTHGEGPY$\mathbf{X}^{41}$VDV$\mathbf{X}^{45}$GRRYLDAN
SGLYNMVAGFDHKGLIDAAKAQYERFPGYHSFFGRMSDQTVMLSEKLVEVSPFDSGR
VFYTNSGSEANDTMVKMLWFLHAAEGKPQKRKILTR$\mathbf{X}^{147}$NAYHGVTAVSASMTG$\mathbf{X}^{163}$P
$\mathbf{X}^{165}$NSVFGLPLPGFVHL$\mathbf{X}^{180}$CPHYWRYGEEGETEEQFVARLARELEETIQREGADTIAG
FFAEPVMGAGGVIPPAKGYFQAILPILRKYDIPVISDEVICGFGRTGNTWGCVTYDFTPD
AIISSKNLTAGFFPVGAVILGPEL$\mathbf{X}^{304}$KRLETAIEAIEEFPHGFTA$\mathbf{X}^{324}$GHPVGCAIALKAID
VVMNEGLAENVRRLAPRFEERLKHIAERPNIGEYRGIGFMWALEAVKDKASKTPFDGN
LSVS$\mathbf{X}^{401}$RIANTC$\mathbf{X}^{408}$DLGLIC$\mathbf{X}^{415}\mathbf{X}^{416}\mathbf{X}^{417}$GQSVIL$\mathbf{X}^{424}$PPFILTEAQMDEMFDKLEKALD
KVFAEVA

$\mathbf{X}^{27}$ is selected from glutamine (Q) and glutamic acid (E); $\mathbf{X}^{41}$ is selected from isoleucine (I) and valine (V); $\mathbf{X}^{45}$ is selected from asparigine (N) and histidine (H); $\mathbf{X}^{147}$ is selected from asparigine (N) and glutamine (Q); $\mathbf{X}^{163}$ is selected from leucine (L) and methionine (M); $\mathbf{X}^{165}$ is selected from tyrosine (Y) and histidine (H); $\mathbf{X}^{180}$ is selected from threonine (T); glycine (G) and serine (S); $\mathbf{X}^{304}$ is selected from alanine (A) and serine (S); $\mathbf{X}^{324}$ is selected from glycine (G) and serine (S); $\mathbf{X}^{401}$ is selected from lysine (K) and glutamic acid (E); $\mathbf{X}^{408}$ is selected from threonine (T) and glutamine (Q); $\mathbf{X}^{415}$ is selected from serine (S) and alanine (A); $\mathbf{X}^{416}$ is selected from proline (P) and alanine (A); $\mathbf{X}^{417}$ is selected from leucine (L) and methionine (M); and $\mathbf{X}^{424}$ is selected from cysteine (C) and serine (S).

**SEQ ID NO. 2**

Vfat888 aa sequence

MNKPQSWEARAETYSLYGFTDMPSLHQRGTVVVTHGEGPYIVDVNGRRYLDANSGL
YNMVAGFDHKGLIDAAKAQYERFPGYHSFFGRMSDQTVMLSEKLVEVSPFDSGRVFY
TNSGSEANDTMVKMLWFLHAAEGKPQKRKILTRNNAYHGVTAVSASMTGLPYNSVFG
LPLPGFVHLTCPHYWRYGEEGETEEQFVARLARELEETIQREGADTIAGFFAEPVMGA
GGVIPPAKGYFQAILPILRKYDIPVISDEVICGFGRTGNTWGCVTYDFTPDAIISSKNLTA
GFFPVGAVILGPELAKRLETAIEAIEEFPHGFTASGHPVGCAIALKAIDVVMNEGLAENV
RRLAPRFEERLKHIAERPNIGEYRGIGFMWALEAVKDKASKTPFDGNLSVSERIANTCT
DLGLICSPMGQSVILCPPFILTEAQMDEMFDKLEKALDKVFAEVA

**SEQ ID NO. 3**

Vfat906 aa sequence

MNKPQSWEARAETYSLYGFTDMPSLHERGTVVVTHGEGPYIVDVNGRRYLDANSGLY
NMVAGFDHKGLIDAAKAQYERFPGYHSFFGRMSDQTVMLSEKLVEVSPFDSGRVFYT
NSGSEANDTMVKMLWFLHAAEGKPQKRKILTRNNAYHGVTAVSASMTGLPYNSVFGL
PLPGFVHLTCPHYWRYGEEGETEEQFVARLARELEETIQREGADTIAGFFAEPVMGAG
GVIPPAKGYFQAILPILRKYDIPVISDEVICGFGRTGNTWGCVTYDFTPDAIISSKNLTAG
FFPVGAVILGPELSKRLETAIEAIEEFPHGFTAGGHPVGCAIALKAIDVVMNEGLAENVR
RLAPRFEERLKHIAERPNIGEYRGIGFMWALEAVKDKASKTPFDGNLSVSKRIANTCQD
LGLICSALGQSVILCPPFILTEAQMDEMFDKLEKALDKVFAEVA

**SEQ ID NO. 4**

Vfat999 aa sequence

MNKPQSWEARAETYSLYGFTDMPSLHERGTVVVTHGEGPYVVDVNGRRYLDANSGL YNMVAGFDHKGLIDAAKAQYERFPGYHSFFGRMSDQTVMLSEKLVEVSPFDSGRVFY TNSGSEANDTMVKMLWFLHAAEGKPQKRKILTRQNAYHGVTAVSASMTGLPHNSVFG LPLPGFVHLTCPHYWRYGEEGETEEQFVARLARELEETIQREGADTIAGFFAEPVMGA GGVIPPAKGYFQAILPILRKYDIPVISDEVICGFGRTGNTWGCVTYDFTPDAIISSKNLTA GFFPVGAVILGPELSKRLETAIEAIEEFPHGFTAGGHPVGCAIALKAIDVVMNEGLAENV RRLAPRFEERLKHIAERPNIGEYRGIGFMWALEAVKDKASKTPFDGNLSVSKRIANTCQ DLGLICSALGQSVILSPPFILTEAQMDEMFDKLEKALDKVFAEVA

**SEQ ID NO. 5**

Vfat1010 aa sequence

MNKPQSWEARAETYSLYGFTDMPSLHERGTVVVTHGEGPYIVDVNGRRYLDANSGLY NMVAGFDHKGLIDAAKAQYERFPGYHSFFGRMSDQTVMLSEKLVEVSPFDSGRVFYT NSGSEANDTMVKMLWFLHAAEGKPQKRKILTRQNAYHGVTAVSASMTGMPHNSVFG LPLPGFVHLTCPHYWRYGEEGETEEQFVARLARELEETIQREGADTIAGFFAEPVMGA GGVIPPAKGYFQAILPILRKYDIPVISDEVICGFGRTGNTWGCVTYDFTPDAIISSKNLTA GFFPVGAVILGPALSKRLETAIEAIEEFPHGFTAGGHPVGCAIALKAIDVVMNEGLAENV RRLAPRFEERLKHIAERPNIGEYRGIGFMWALEAVKDKASKTPFDGNLSVSKRIANTCQ DLGLICSAMGQSVILSPPFILTEAQMDEMFDKLEKALDKVFAEVA

**SEQ ID NO. 6**

Vfat1020 aa sequence

MNKPQSWEARAETYSLYGFTDMPSLHERGTVVVTHGEGPYVVDVNGRRYLDANSGL YNMVAGFDHKGLIDAAKAQYERFPGYHSFFGRMSDQTVMLSEKLVEVSPFDSGRVFY TNSGSEANDTMVKMLWFLHAAEGKPQKRKILTRQNAYHGVTAVSASMTGLPHNSVFG LPLPGFVHLGCPHYWRYGEEGETEEQFVARLARELEETIQREGADTIAGFFAEPVMGA GGVIPPAKGYFQAILPILRKYDIPVISDEVICGFGRTGNTWGCVTYDFTPDAIISSKNLTA GFFPVGAVILGPELSKRLETAIEAIEEFPHGFTAGGHPVGCAIALKAIDVVMNEGLAENV RRLAPRFEERLKHIAERPNIGEYRGIGFMWALEAVKDKASKTPFDGNLSVSKRIANTCQ DLGLICAAMGQSVILSPPFILTEAQMDEMFDKLEKALDKVFAEVA

**SEQ ID NO. 7**

Vfat1030 aa sequence

MNKPQSWEARAETYSLYGFTDMPSLHERGTVVVTHGEGPYIVDVHGRRYLDANSGLY
NMVAGFDHKGLIDAAKAQYERFPGYHSFFGRMSDQTVMLSEKLVEVSPFDSGRVFYT
NSGSEANDTMVKMLWFLHAAEGKPQKRKILTRQNAYHGVTAVSASMTGLPHNSVFGL
PLPGFVHLSCPHYWRYGEEGETEEQFVARLARELEETIQREGADTIAGFFAEPVMGAG
GVIPPAKGYFQAILPILRKYDIPVISDEVICGFGRTGNTWGCVTYDFTPDAIISSKNLTAG
FFPVGAVILGPELSKRLETAIEAIEEFPHGFTAGGHPVGCAIALKAIDVVMNEGLAENVR
RLAPRFEERLKHIAERPNIGEYRGIGFMWALEAVKDKASKTPFDGNLSVSKRIANTCQD
LGLICSAMGQSVILSPPFILTEAQMDEMFDKLEKALDKVFAEVA

**SEQ ID NO. 10**

Vfat 888 DNA SEQUENCE

ATGAATAAACCACAAAGCTGGGAAGCGCGTGCTGAAACTTACTCTCTGTACGGCTT
CACTGATATGCCATCTCTGCACCAGCGTGGTACCGTGGTTGTCACCCACGGCGAG
GGCCCATACATCGTGGACGTCAACGGTCGCCGTTACCTGGACGCAAACTCCGGCC
TGTACAATATGGTTGCCGGCTTCGACCACAAGGGTCTGATCGACGCAGCAAAGGC
CCAGTACGAACGCTTCCCGGGTTACCATAGCTTCTTCGGTCGTATGTCTGATCAAA
CTGTTATGCTGAGCGAGAAACTGGTAGAGGTGTCTCCATTCGACAGCGGTCGCGT
GTTCTATACTAACTCCGGCTCCGAGGCTAACGATACTATGGTGAAAATGCTGTGGT

TTCTGCACGCCGCAGAGGGCAAGCCGCAAAAACGCAAAATCCTGACTCGTAACAA
CGCATACCACGGTGTAACTGCTGTTTCCGCTTCCATGACGGGTCTGCCGTACAACT
CTGTATTCGGCCTGCCGCTGCCGGGTTTCGTTCACCTGACCTGTCCGCACTATTG
GCGTTACGGCGAAGAAGGTGAAACCGAAGAGCAGTTTGTTGCTCGTCTGGCCCGC
GAGCTGGAGGAAACTATCCAACGTGAAGGCGCGGACACGATTGCGGGCTTCTTTG
CTGAGCCGGTCATGGGCGCGGGCGGCGTAATCCCGCCGGCGAAAGGTTACTTCC
AGGCGATCCTGCCGATTCTGCGTAAGTACGACATCCCGGTTATCTCTGATGAAGTT
ATCTGCGGCTTTGGTCGTACCGGTAATACTTGGGGTTGCGTTACCTATGACTTCAC
CCCGGATGCGATCATCTCCAGCAAAAATCTGACCGCCGGTTTCTTTCCGGTTGGT
GCTGTGATTCTGGGTCCGGAACTGGCGAAACGCCTGGAAACGGCGATCGAAGCTA
TCGAAGAGTTCCCGCACGGCTTTACGGCCAGCGGTCACCCGGTGGGTTGCGCTAT
CGCTCTGAAAGCAATCGATGTTGTGATGAATGAGGGTCTGGCAGAGAACGTGCGC
CGCCTGGCACCGCGTTTTGAGGAGCGTCTGAAACACATTGCCGAACGTCCGAACA
TCGGTGAATATCGTGGCATCGGTTTTATGTGGGCACTGGAGGCTGTGAAAGACAA
AGCATCTAAAACCCCATTCGATGGTAATCTGTCTGTGAGCGAGCGTATCGCTAACA
CCTGTACCGACCTGGGCCTGATCTGTAGCCCGATGGGTCAGTCCGTTATCCTGTG
CCCGCCGTTCATCCTGACCGAGGCGCAAATGGATGAGATGTTTGACAAACTGGAG
AAGGCTCTGGACAAAGTCTTTGCGGAGGTGGCGTAA

**SEQ ID NO. 11**

Vfat 906 DNA SEQUENCE

ATGAATAAACCACAAAGCTGGGAAGCGCGTGCTGAAACTTACTCTCTGTACGGCTT
CACTGATATGCCATCTCTGCACgAGCGTGGTACCGTGGTTGTCACCCACGGCGAG
GGCCCATACATCGTGGACGTCAACGGTCGCCGTTACCTGGACGCAAACTCCGGCC
TGTACAATATGGTTGCCGGCTTCGACCACAAGGGTCTGATCGACGCAGCAAAGGC
CCAGTACGAACGCTTCCCGGGTTACCATAGCTTCTTCGGTCGTATGTCTGATCAAA
CTGTTATGCTGAGCGAGAAACTGGTAGAGGTGTCTCCATTCGACAGCGGTCGCGT
GTTCTATACTAACTCCGGCTCCGAGGCTAACGATACTATGGTGAAAATGCTGTGGT
TTCTGCACGCCGCAGAGGGCAAGCCGCAAAAACGCAAAATCCTGACTCGTaacAAC
GCATACCACGGTGTAACTGCTGTTTCCGCTTCCATGACGGGTCTGCCGTACAACTC
TGTATTCGGCCTGCCGCTGCCGGGTTTCGTTCACCTGACCTGTCCGCACTATTGG
CGTTACGGCGAAGAAGGTGAAACCGAAGAGCAGTTTGTTGCTCGTCTGGCCCGCG
AGCTGGAGGAAACTATCCAACGTGAAGGCGCGGACACGATTGCGGGCTTCTTTGC
TGAGCCGGTCATGGGCGCGGGCGGCGTAATCCCGCCGGCGAAAGGTTACTTCCA
GGCGATCCTGCCGATTCTGCGTAAGTACGACATCCCGGTTATCTCTGATGAAGTTA
TCTGCGGCTTTGGTCGTACCGGTAATACTTGGGGTTGCGTTACCTATGACTTCACC
CCGGATGCGATCATCTCCAGCAAAAATCTGACCGCCGGTTTCTTTCCGGTTGGTG
CTGTGATTCTGGGTCCGGAACTGAGCAAACGCCTGGAAACGGCGATCGAAGCTAT
CGAAGAGTTCCCGCACGGCTTTACGGCCggcGGTCACCCGGTGGGTTGCGCTATC
GCTCTGAAAGCAATCGATGTTGTGATGAATGAGGGTCTGGCAGAGAACGTGCGCC
GCCTGGCACCGCGTTTTGAGGAGCGTCTGAAACACATTGCCGAACGTCCGAACAT
CGGTGAATATCGTGGCATCGGTTTTATGTGGGCACTGGAGGCTGTGAAAGACAAA
GCATCTAAAACCCCATTCGATGGTAATCTGTCTGTGAGCaaaCGTATCGCTAACACC
TGTcagGACCTGGGCCTGATCTGTAGCgCGCTGGGTCAGTCCGTTATCCTGTGCCC
GCCGTTCATCCTGACCGAGGCGCAAATGGATGAGATGTTTGACAAACTGGAGAAG
GCTCTGGACAAAGTCTTTGCGGAGGTGGCGTAA

**SEQ ID NO. 12**

Vfat 999 DNA SEQUENCE

ATGAATAAACCACAAAGCTGGGAAGCGCGTGCTGAAACTTACTCTCTGTACGGCTT
CACTGATATGCCATCTCTGCACGAGCGTGGTACCGTGGTTGTCACCCACGGCGAG
GGCCCATACGTGGTGGACGTCAACGGTCGCCGTTACCTGGACGCAAACTCCGGC
CTGTACAATATGGTTGCCGGCTTCGACCACAAGGGTCTGATCGACGCAGCAAAGG
CCCAGTACGAACGCTTCCCGGGTTACCATAGCTTCTTCGGTCGTATGTCTGATCAA
ACTGTTATGCTGAGCGAGAAACTGGTAGAGGTGTCTCCATTCGACAGCGGTCGCG
TGTTCTATACTAACTCCGGCTCCGAGGCTAACGATACTATGGTGAAAATGCTGTGG
TTTCTGCACGCCGCAGAGGGCAAGCCGCAAAAACGCAAAATCCTGACTCGTCAAA
ACGCATACCACGGTGTAACTGCTGTTTCCGCTTCCATGACGGGTCTGCCGCACAA
CTCTGTATTCGGCCTGCCGCTGCCGGGTTTCGTTCACCTGACCTGTCCGCACTATT
GGCGTTACGGCGAAGAAGGTGAAACCGAAGAGCAGTTTGTTGCTCGTCTGGCCCG
CGAGCTGGAGGAAACTATCCAACGTGAAGGCGCGGACACGATTGCGGGCTTCTTT
GCTGAGCCGGTCATGGGCGCGGGCGGCGTAATCCCGCCGGCGAAAGGTTACTTC
CAGGCGATCCTGCCGATTCTGCGTAAGTACGACATCCCGGTTATCTCTGATGAAGT
TATCTGCGGCTTTGGTCGTACCGGTAATACTTGGGGTTGCGTTACCTATGACTTCA
CCCCGGATGCGATCATCTCCAGCAAAAATCTGACCGCCGGTTTCTTTCCGGTTGGT
GCTGTGATTCTGGGTCCGGAACTGAGCAAACGCCTGGAAACGGCGATCGAAGCTA
TCGAAGAGTTCCCGCACGGCTTTACGGCCGGCGGTCACCCGGTGGGTTGCGCTA
TCGCTCTGAAAGCAATCGATGTTGTGATGAATGAGGGTCTGGCAGAGAACGTGCG
CCGCCTGGCACCGCGTTTTGAGGAGCGTCTGAAACACATTGCCGAACGTCCGAAC
ATCGGTGAATATCGTGGCATCGGTTTTATGTGGGCACTGGAGGCTGTGAAAGACA

AAGCATCTAAAACCCCATTCGATGGTAATCTGTCTGTGAGCAAACGTATCGCTAAC
ACCTGTCAGGACCTGGGCCTGATCTGTAGCGCGCTGGGTCAGTCCGTTATCCTGA
GCCCGCCGTTCATCCTGACCGAGGCGCAAATGGATGAGATGTTTGACAAACTGGA
GAAGGCTCTGGACAAAGTCTTTGCGGAGGTGGCGTAA

**SEQ ID NO. 13**

Vfat 1010 DNA SEQUENCE

ATGAATAAACCACAAAGCTGGGAAGCGCGTGCTGAAACTTACTCTCTGTACGGCTT
CACTGATATGCCATCTCTGCACGAGCGTGGTACCGTGGTTGTCACCCACGGCGAG
GGCCCATACATCGTGGACGTCAACGGTCGCCGTTACCTGGACGCAAACTCCGGCC
TGTACAATATGGTTGCCGGCTTCGACCACAAGGGTCTGATCGACGCAGCAAAGGC
CCAGTACGAACGCTTCCCGGGTTACCATAGCTTCTTCGGTCGTATGTCTGATCAAA
CTGTTATGCTGAGCGAGAAACTGGTAGAGGTGTCTCCATTCGACAGCGGTCGCGT
GTTCTATACTAACTCCGGCTCCGAGGCTAACGATACTATGGTGAAAATGCTGTGGT
TTCTGCACGCCGCAGAGGGCAAGCCGCAAAAACGCAAAATCCTGACTCGTCAAAA
CGCATACCACGGTGTAACTGCTGTTTCCGCTTCCATGACGGGTATGCCGCACAAC
TCTGTATTCGGCCTGCCGCTGCCGGGTTTCGTTCACCTGACCTGTCCGCACTATTG
GCGTTACGGCGAAGAAGGTGAAACCGAAGAGCAGTTTGTTGCTCGTCTGGCCCGC
GAGCTGGAGGAAACTATCCAACGTGAAGGCGCGGACACGATTGCGGGCTTCTTTG
CTGAGCCGGTCATGGGCGCGGGCGGCGTAATCCCGCCGGCGAAAGGTTACTTCC
AGGCGATCCTGCCGATTCTGCGTAAGTACGACATCCCGGTTATCTCTGATGAAGTT
ATCTGCGGCTTTGGTCGTACCGGTAATACTTGGGGTTGCGTTACCTATGACTTCAC
CCCGGATGCGATCATCTCCAGCAAAAATCTGACCGCCGGTTTCTTTCCGGTTGGT
GCTGTGATTCTGGGTCCGGCACTGAGCAAACGCCTGGAAACGGCGATCGAAGCTA
TCGAAGAGTTCCCGCACGGCTTTACGGCCGGCGGTCACCCGGTGGGTTGCGCTA
TCGCTCTGAAAGCAATCGATGTTGTGATGAATGAGGGTCTGGCAGAGAACGTGCG
CCGCCTGGCACCGCGTTTTGAGGAGCGTCTGAAACACATTGCCGAACGTCCGAAC
ATCGGTGAATATCGTGGCATCGGTTTTATGTGGGCACTGGAGGCTGTGAAAGACA
AAGCATCTAAAACCCCATTCGATGGTAATCTGTCTGTGAGCAAACGTATCGCTAAC
ACCTGTCAGGACCTGGGCCTGATCTGTAGCGCGATGGGTCAGTCCGTTATCCTGA
GCCCGCCGTTCATCCTGACCGAGGCGCAAATGGATGAGATGTTTGACAAACTGGA
GAAGGCTCTGGACAAAGTCTTTGCGGAGGTGGCGTAA

**SEQ ID NO. 14**

Vfat 1020 DNA SEQUENCE

ATGAATAAACCACAAAGCTGGGAAGCGCGTGCTGAAACTTACTCTCTGTACGGCTT
CACTGATATGCCATCTCTGCACGAGCGTGGTACCGTGGTTGTCACCCACGGCGAG
GGCCCATACGTGGTGGACGTCAACGGTCGCCGTTACCTGGACGCAAACTCCGGC
CTGTACAATATGGTTGCCGGCTTCGACCACAAGGGTCTGATCGACGCAGCAAAGG
CCCAGTACGAACGCTTCCCGGGTTACCATAGCTTCTTCGGTCGTATGTCTGATCAA
ACTGTTATGCTGAGCGAGAAACTGGTAGAGGTGTCTCCATTCGACAGCGGTCGCG
TGTTCTATACTAACTCCGGCTCCGAGGCTAACGATACTATGGTGAAAATGCTGTGG
TTTCTGCACGCCGCAGAGGGCAAGCCGCAAAAACGCAAAATCCTGACTCGTCAAA
ACGCATACCACGGTGTAACTGCTGTTTCCGCTTCCATGACGGGTCTGCCGCACAA
CTCTGTATTCGGCCTGCCGCTGCCGGGTTTCGTTCACCTGGGTTGTCCGCACTATT
GGCGTTACGGCGAAGAAGGTGAAACCGAAGAGCAGTTTGTTGCTCGTCTGGCCCG
CGAGCTGGAGGAAACTATCCAACGTGAAGGCGCGGACACGATTGCGGGCTTCTTT
GCTGAGCCGGTCATGGGCGCGGGCGGCGTAATCCCGCCGGCGAAAGGTTACTTC
CAGGCGATCCTGCCGATTCTGCGTAAGTACGACATCCCGGTTATCTCTGATGAAGT
TATCTGCGGCTTTGGTCGTACCGGTAATACTTGGGGTTGCGTTACCTATGACTTCA
CCCCGGATGCGATCATCTCCAGCAAAAATCTGACCGCCGGTTTCTTTCCGGTTGGT
GCTGTGATTCTGGGTCCGGAACTGAGCAAACGCCTGGAAACGGCGATCGAAGCTA
TCGAAGAGTTCCCGCACGGCTTTACGGCCGGCGGTCACCCGGTGGGTTGCGCTA
TCGCTCTGAAAGCAATCGATGTTGTGATGAATGAGGGTCTGGCAGAGAACGTGCG
CCGCCTGGCACCGCGTTTTGAGGAGCGTCTGAAACACATTGCCGAACGTCCGAAC
ATCGGTGAATATCGTGGCATCGGTTTTATGTGGGCACTGGAGGCTGTGAAAGACA
AAGCATCTAAAACCCCATTCGATGGTAATCTGTCTGTGAGCAAACGTATCGCTAAC
ACCTGTCAGGACCTGGGCCTGATCTGTAGCGCGATGGGTCAGTCCGTTATCCTGA
GCCCGCCGTTCATCCTGACCGAGGCGCAAATGGATGAGATGTTTGACAAACTGGA
GAAGGCTCTGGACAAAGTCTTTGCGGAGGTGGCGTAA

**SEQ ID NO. 15**

Vfat 1030 DNA SEQUENCE

ATGAATAAACCACAAAGCTGGGAAGCGCGTGCTGAAACTTACTCTCTGTACGGCTT
CACTGATATGCCATCTCTGCACGAGCGTGGTACCGTGGTTGTCACCCACGGCGAG
GGCCCATACATCGTGGACGTCCACGGTCGCCGTTACCTGGACGCAAACTCCGGC
CTGTACAATATGGTTGCCGGCTTCGACCACAAGGGTCTGATCGACGCAGCAAAGG

CCCAGTACGAACGCTTCCCGGGTTACCATAGCTTCTTCGGTCGTATGTCTGATCAA
ACTGTTATGCTGAGCGAGAAACTGGTAGAGGTGTCTCCATTCGACAGCGGTCGCG
TGTTCTATACTAACTCCGGCTCCGAGGCTAACGATACTATGGTGAAAATGCTGTGG
TTTCTGCACGCCGCAGAGGGCAAGCCGCAAAAACGCAAAATCCTGACTCGTCAAA
ACGCATACCACGGTGTAACTGCTGTTTCCGCTTCCATGACGGGTCTGCCGCACAA
CTCTGTATTCGGCCTGCCGCTGCCGGGTTTCGTTCACCTGAGCTGTCCGCACTATT
GGCGTTACGGCGAAGAAGGTGAAACCGAAGAGCAGTTTGTTGCTCGTCTGGCCCG
CGAGCTGGAGGAAACTATCCAACGTGAAGGCGCGGACACGATTGCGGGCTTCTTT
GCTGAGCCGGTCATGGGCGCGGGCGGCGTAATCCCGCCGGCGAAAGGTTACTTC
CAGGCGATCCTGCCGATTCTGCGTAAGTACGACATCCCGGTTATCTCTGATGAAGT
TATCTGCGGCTTTGGTCGTACCGGTAATACTTGGGGTTGCGTTACCTATGACTTCA
CCCCGGATGCGATCATCTCCAGCAAAAATCTGACCGCCGGTTTCTTTCCGGTTGGT
GCTGTGATTCTGGGTCCGGAACTGAGCAAACGCCTGGAAACGGCGATCGAAGCTA
TCGAAGAGTTCCCGCACGGCTTTACGGCCGGCGGTCACCCGGTGGGTTGCGCTA
TCGCTCTGAAAGCAATCGATGTTGTGATGAATGAGGGTCTGGCAGAGAACGTGCG
CCGCCTGGCACCGCGTTTTGAGGAGCGTCTGAAACACATTGCCGAACGTCCGAAC
ATCGGTGAATATCGTGGCATCGGTTTTATGTGGGCACTGGAGGCTGTGAAAGACA
AAGCATCTAAAACCCCATTCGATGGTAATCTGTCTGTGAGCAAACGTATCGCTAAC
ACCTGTCAGGACCTGGGCCTGATCTGTAGCGCGATGGGTCAGTCCGTTATCCTGA
GCCCGCCGTTCATCCTGACCGAGGCGCAAATGGATGAGATGTTTGACAAACTGGA
GAAGGCTCTGGACAAAGTCTTTGCGGAGGTGGCGTAA

## Claims

**1.** A compound according to formula (**VI**)

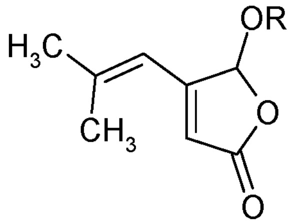

(**VI**)

wherein R is selected from:

hydrogen,
($C_1$-$C_6$)alkyl,
($C_1$-$C_6$)haloalkyl,

$(C_1-C_3)$alkoxy$(C_2-C_6)$alkyl,
$(C_2-C_6)$alkenyl,
$(C_3-C_{10})$cycloalkyl, which may optionally be substituted with 1, 2 or 3 groups independently selected from halo, $(C_1-C_3)$alkyl, and $(C_1-C_3)$alkyloxy,
$(C_3-C_{10})$cycloalkyl$(C_1-C_6)$alkyl, wherein the cycloalkyl group may optionally be substituted with 1, 2 or 3 groups independently selected from halo, $(C_1-C_3)$alkyl, and $(C_1-C_3)$alkyloxy,
aryl, which may optionally be substituted with 1, 2 or 3 groups independently selected from halo, $(C_1-C_3)$alkyl, and $(C_1-C_3)$alkyloxy,
aryl$(C_1-C_6)$alkyl, wherein the aryl group may optionally be substituted with 1, 2 or 3 groups independently selected from halo, $(C_1-C_3)$alkyl, and $(C_1-C_3)$alkyloxy,
$R^1$-C(O)-, and
$R^2$-$SO_2$-;

$R^1$ is selected from:

hydrogen,
$(C_1-C_6)$alkyl,
$(C_1-C_6)$haloalkyl,
$(C_1-C_3)$alkoxy$(C_2-C_6)$alkyl,
$(C_2-C_6)$alkenyl,
$(C_3-C_{10})$cycloalkyl, which may optionally be substituted with 1, 2 or 3 groups independently selected from halo, $(C_1-C_3)$alkyl, and $(C_1-C_3)$alkyloxy,
$(C_3-C_{10})$cycloalkyl$(C_1-C_6)$alkyl, wherein the cycloalkyl group may optionally be substituted with 1, 2 or 3 groups independently selected from halo, $(C_1-C_3)$alkyl, and $(C_1-C_3)$alkyloxy,
aryl, which may optionally be substituted with 1, 2 or 3 groups independently selected from halo, $(C_1-C_3)$alkyl, and $(C_1-C_3)$alkyloxy, and
aryl$(C_1-C_6)$alkyl, wherein the aryl group may optionally be substituted with 1, 2 or 3 groups independently selected from halo, $(C_1-C_3)$alkyl, and $(C_1-C_3)$alkyloxy; and

$R^2$ is selected from:

$(C_1-C_6)$alkyl,
$(C_1-C_6)$haloalkyl,
$(C_1-C_3)$alkoxy$(C_2-C_6)$alkyl,
$(C_2-C_6)$alkenyl,
$(C_3-C_{10})$cycloalkyl, which may optionally be substituted with 1, 2 or 3 groups independently selected from halo, $(C_1-C_3)$alkyl, and $(C_1-C_3)$alkyloxy,
$(C_3-C_{10})$cycloalkyl$(C_1-C_6)$alkyl, wherein the cycloalkyl group may optionally be substituted with 1, 2 or 3 groups independently selected from halo, $(C_1-C_3)$alkyl, and $(C_1-C_3)$alkyloxy,
aryl, which may optionally be substituted with 1, 2 or 3 groups independently selected from halo, $(C_1-C_3)$alkyl, and $(C_1-C_3)$alkyloxy, and
aryl$(C_1-C_6)$alkyl, wherein the aryl group may optionally be substituted with 1, 2 or 3 groups independently selected from halo, $(C_1-C_3)$alkyl, and $(C_1-C_3)$alkyloxy.

**2.** The compound of claim 1 which is 5-hydroxy-4-(2-methyl-1-propenyl)-5*H*-2-furanone according to formula (**VI$^A$**)

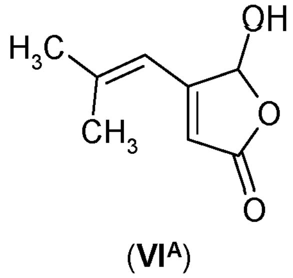

(**VI$^A$**)

**3.** The compound of formula (**VI$^B$**)

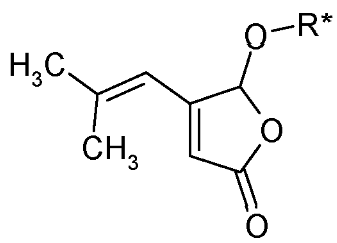

(**VI^B**)

wherein R* is a chiral ($C_5$-$C_{15}$)hydrocarbon group.

**4.** The compound according to claim 3 wherein R* is selected from (*R*)- or (*S*)-α-methylbenzyl, (*R*)- or (*S*)-1-(1-naphthyl)ethyl, (*R*)- or (*S*)-1-(2-naphthyl)ethyl, menthyl and bornyl.

**5.** The compound according to claim 1 wherein R is $R^1$-C(O)- or $R^2$-$SO_2$- and $R^1$ and $R^2$ are chiral ($C_5$-$C_{15}$) hydrocarbon groups.

**6.** A compound of formula (IX)

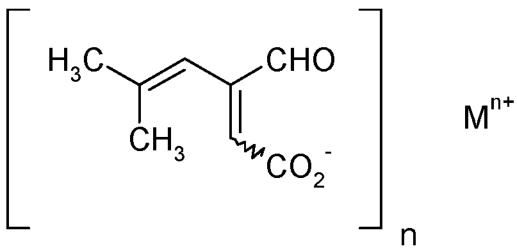

(**IX**)

wherein:

n is 1 and $M^+$ is selected from $Li^+$, $Na^+$, $K^+$, $Rb^+$, $NH_4^+$, (($C_1$-$C_3$)alkyl)$NH_3^+$, (($C_1$-$C_3$)alkyl)$_2NH_2^+$, (($C_1$-$C_3$)alkyl)$_3NH^+$ and (($C_1$-$C_3$)alkyl)$_4N^+$; or
n is 2 and $M^{2+}$ is selected from $Mg^{2+}$, $Ca^{2+}$ and $Zn^{2+}$.

**7.** The compound according to claim 6 wherein n is 1 and $M^+$ is selected from $NH_4^+$ and (($C_1$-$C_3$)alkyl)$NH_3^+$.

**8.** The compound according to claim 6 wherein n is 1 and $M^+$ is selected from $Li^+$, $Na^+$ and $K^+$.

**9.** A compound of formula (**VII**)

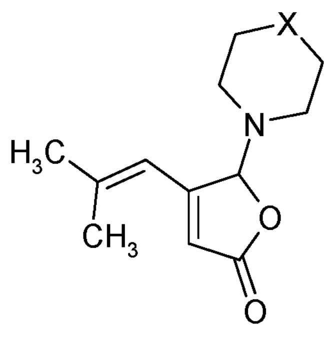

(**VII**)

wherein -X- represents a single bond, $-CH_2-$, -O-; -NH-, $-N((C_1-C_3)alkyl)-$, -N(benzyl)-, or

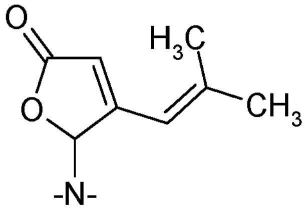

.

**10.** The compound according to claim 9 selected from:

4-(2-methylpropenyl)-5-pyrrolidin-1-yl-5*H*-furan-2-one;
4-(2-methylpropenyl)-5-piperidin-1-yl-5*H*-furan-2-one;
4-(2-methylpropenyl)-5-morpholin-4-yl-5*H*-furan-2-one; and
1,4-bis-(4-(2-methylpropenyl)-5*H*-furan-2-on-5-yl)piperazine.

**11.** A process for the manufacture of the compound of formula (**VI^A**) according to claim 2 comprising the step of treating a compound of formula (**VII**)

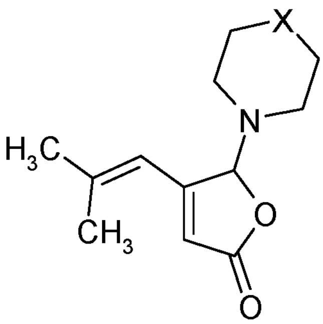

(**VII**)

wherein -X- represents a single bond, $-CH_2-$, -O-, -NH-, $-N((C_1-C_3)alkyl)-$, -N(benzyl)-, or

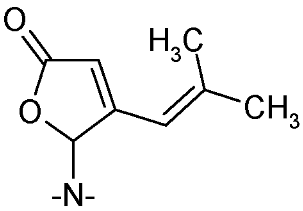

with water in the presence of an acid catalyst.

**12.** A process for the manufacture of a compound of formula (**VI**) according to claim 1 wherein R is other than hydrogen, $R^1$-C(O)-, and $R^2$-$SO_2$-, comprising the step of treating a compound of formula (**VI^A**) with an alcohol R-OH in the presence of an acid catalyst.

**13.** A process for the manufacture of a compound of formula (**VI**) according to claim 1 wherein R is other than hydrogen, $R^1$-C(O)-, and $R^2$-$SO_2$-, comprising the step of treating a compound of formula (**VII**) with an alcohol R-OH in the presence of stoichiometric acid.

**14.** A process for the manufacture of a compound of formula (**VI**) according to claim 1 wherein R is $R^1$-C(O)-, comprising the steps of treating a compound of formula (**VI^A**) with an acid chloride $R^1$-C(O)-Cl or acid anhydride $(R^1-C(O))_2O$.

**15.** A process for the manufacture of a compound of formula (**VI**) according to claim 1 wherein R is $R^2$-$SO_2$-; comprising the step of treating a compound of formula (**VI$^A$**) with a sulfonyl chloride $R^2$-$SO_2$-Cl.

**16.** A process for the manufacture of 3-aminomethyl-5-methylhexanoic acid (**II**)

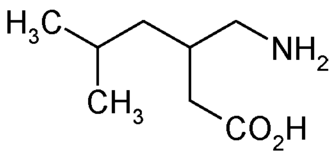

(**II**)

or a pharmaceutically acceptable salt thereof, comprising the steps:

(a) preparing 5-hydroxy-4-(2-methyl-1-propenyl)-5*H*-2-furanone (VIA)

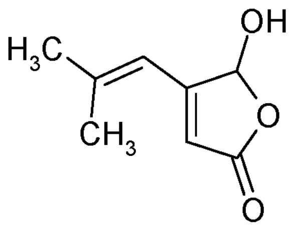

(**VI$^A$**)

(b) converting said 5-hydroxy-4-(2-methyl-1-propenyl)-5*H*-2-furanone into 5-hydroxy-4-(2-methylpropyl)-3,4-dihydro-5*H*-2-furanone (**I$^A$**)

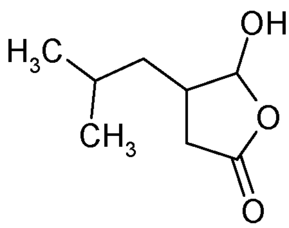

(**I$^A$**)

and
(c) converting said 5-hydroxy-4-(2-methylpropyl)-3,4-dihydro-5*H*-2-furanone into 3-aminomethyl-5-methylhexanoic acid (**II**).

**17.** The process according to claim 16 wherein the 3-aminomethyl-5-methylhexanoic acid (**II**) is (*S*)-3-aminomethyl-5-methylhexanoic acid ((*S*)-**II**)

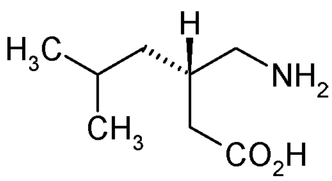

((*S*)-**II**)

wherein said (*S*)-3-aminomethyl-5-methylhexanoic acid has an enantiomeric excess of at least 80%.

**18.** The process according to claim 16 or 17 wherein step (a) comprises a process according to claim 11.

**19.** The process according to any one of claims 16 to 18 wherein step (b) comprises the steps:

(b1) treating the 5-hydroxy-4-(2-methyl-1-propenyl)-5*H*-2-furanone (VIA) with a metal oxide, hydroxide, carbonate or bicarbonate, ammonia, a mono- di- or tri-($C_1$-$C_3$)alkylamine, or a tetra-($C_1$-$C_3$)alkylammonium hydroxide to form a salt of formula (**IX**)

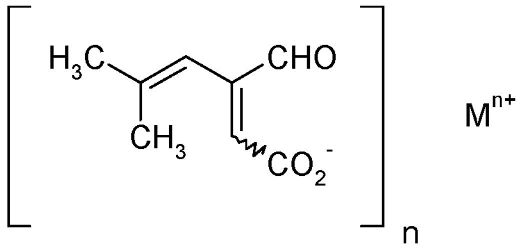

(**IX**)

wherein:

n is 1 and $M^+$ is selected from $Li^+$, $Na^+$, $K^+$, $Rb^+$, $NH_4^+$, (($C_1$-$C_3$)alkyl)$NH_3^+$, (($C_1$-$C_3$)alkyl)$_2NH_2^+$, (($C_1$-$C_3$)alkyl)$_3NH^+$ and (($C_1$-$C_3$)alkyl)$_4N^+$; or
n is 2 and $M^{2+}$ is selected from $Mg^{2+}$, $Ca^{2+}$ and $Z_n^{2+}$;

(b2) hydrogenating the salt of formula (**IX**) to obtain a salt of formula (X)

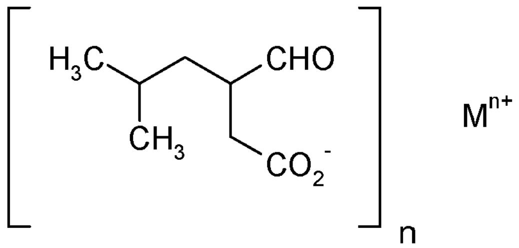

(**X**) ;

and
(b3) treating the salt of formula (**X**) with an acid.

**20.** The process according to any one of claims 16 to 18 wherein step (b) comprises the steps:

(b1) converting the 5-hydroxy-4-(2-methyl-1-propenyl)-5*H*-2-furanone (**VI^A^**) to a compound of formula (**VI**) as defined in claim 3 wherein R is a chiral ($C_5$-$C_{15}$)hydrocarbon group;
(b2) hydrogenating the compound of formula (**VI**) to obtain a compound of formula (**XI**)

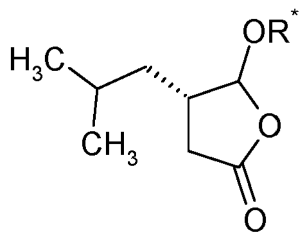

(**XI**) ;

wherein R* is a chiral ($C_5$-$C_{15}$)hydrocarbon group; and
(b3) treating the compound of formula (**XI**) with an acid to give ((*S*)-**I^A^**).

**21.** A process for the manufacture of 3-aminomethyl-5-methylhexanoic acid (**II**)

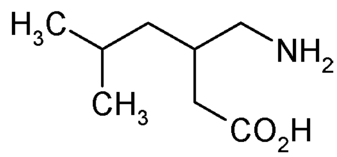

(**II**)

or a pharmaceutically acceptable salt thereof, comprising the steps:

(a) preparing 5-hydroxy-4-(2-methyl-1-propenyl)-5*H*-2-furanone (**VI$^A$**)

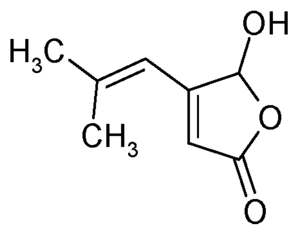

(**VI$^A$**)

(b) treating the 5-hydroxy-4-(2-methyl-1-propenyl)-5*H*-2-furanone (VIA) with ammonia or a mono-($C_1$-$C_3$)alkylamine to form a salt of formula (**IX$^A$**)

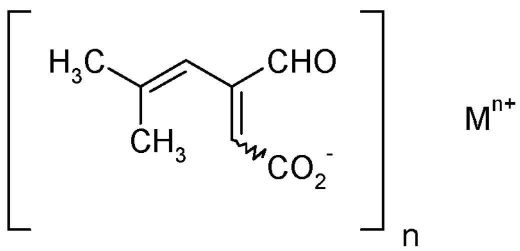

(**IX$^A$**)

wherein:

n is 1 and $M^+$ is selected from $NH_4^+$ and (($C_1$-$C_3$)alkyl)$NH_3^+$;

(c) hydrogenating the salt of formula (**IX^A**) to obtain a salt of formula (**X^A**)

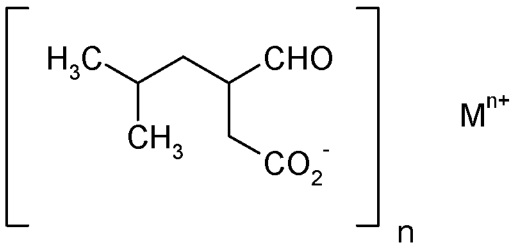

(**X^A**) ;

and

(d) treating the salt of formula (**X^A**) with a transaminase or an amine oxidase/imine reductase enzyme to provide 3-aminomethyl-5-methylhexanoic acid (**II**).

**22.** The process according to claim 21 wherein the 3-aminomethyl-5-methylhexanoic acid (**II**) is (S)-3-aminomethyl-5-methylhexanoic acid ((*S*)-**II**)

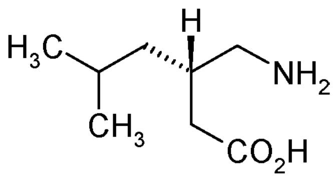

((*S*)-**II**)

wherein said (S)-3-aminomethyl-5-methylhexanoic acid has an enantiomeric excess of at least 80%.

**23.** The process according to claim 21 or 22 wherein step (a) comprises a process according to claim 11.

**Patentansprüche**

**1.** Verbindung der Formel (VI)

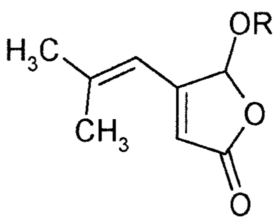

(**VI**)

worin R ausgewählt ist aus:

Wasserstoff,
($C_1$-$C_6$)-Alkyl,
($C_1$-$C_6$)-Halogenalkyl,
($C_1$-$C_3$) -Alkoxy- ($C_2$-$C_6$) -alkyl,
($C_2$-$C_6$)-Alkenyl,

$(C_3-C_{10})$ -Cycloalkyl, das gegebenenfalls substituiert ist mit 1, 2 oder 3 Gruppe(n), unabhängig ausgewählt aus Halogen, $(C_1-C_3)$-Alkyl und $(C_1-C_3)$-Alkyloxy,
$(C_3-C_{10})$-Cycloalkyl-$(C_1-C_6)$-alkyl, wobei die Cyclo-alkylgruppe gegebenenfalls substituiert ist mit 1, 2 oder 3 Gruppe(n), unabhängig ausgewählt aus Halogen, $(C_1-C_3)$-Alkyl und $(C_1-C_3)$-Alkyloxy,
Aryl, das gegebenenfalls substituiert ist mit 1, 2 oder 3 Gruppe(n), unabhängig ausgewählt aus Halogen, $(C_1-C_3)$-Alkyl und $(C_1-C_3)$-Alkyloxy,
Aryl-$(C_1-C_6)$-alkyl, wobei die Arylgruppe gegebenenfalls substituiert sein kann mit 1, 2 oder 3 Gruppe(n), unabhängig ausgewählt aus Halogen, $(C_1-C_3)$-Alkyl und $(C_1-C_3)$-Alkyloxy,
$R^1$-C(O)-, und
$R^2$-$SO_2$-;

$R^1$ ausgewählt ist aus:

Wasserstoff,
$(C_1-C_6)$-Alkyl,
$(C_1-C_6)$-Halogenalkyl,
$(C_1-C_3)$-Alkoxy-$(C_2-C_6)$-alkyl,
$(C_2-C_6)$-Alkenyl,
$(C_3-C_{10})$-Cycloalkyl, das gegebenenfalls substituiert sein kann mit 1, 2 oder 3 Gruppe(n), unabhängig ausgewählt aus Ha-logen, $(C_1-C_3)$-Alkyl und $(C_1-C_3)$-Alkyloxy,
$(C_3-C_{10})$-Cycloalkyl-$(C_1-C_6)$-alkyl, wobei die Cyclo-alkylgruppe gegebenenfalls substituiert sein kann mit 1, 2 oder 3 Gruppe(n), unabhängig ausgewählt aus Halogen, $(C_1-C_3)$-Alkyl und $(C_1-C_3)$-Alkyloxy,
Aryl, das gegebenenfalls substituiert sein kann mit 1, 2 oder 3 Gruppe(n), unabhängig ausgewählt aus Halogen, $(C_1-C_3)$-Alkyl und $(C1-C_3)$-Alkyloxy und
Aryl-$(C_1-C_6)$-alkyl, wobei die Arylgruppe gegebenenfalls substituiert sein kann mit 1, 2 oder 3 Gruppe(n), unabhängig ausgewählt aus Halogen, $(C_1-C_3)$-Alkyl und $(C_1-C_3)$)-Alkyloxy; und

$R^2$ ausgewählt ist aus:

$(C_1-C_6)$-Alkyl,
$(C_1-C_6)$-Halogenalkyl,
$(C_1-C_3)$-Alkoxy-$(C_2-C_6)$-alkyl,
$(C_2-C_6)$-Alkenyl,
$(C_3-C_{10})$-Cycloalkyl, das gegebenenfalls substituiert sein kann mit 1, 2 oder 3 Gruppe(n), unabhängig ausgewählt aus Ha-logen, $(C_1-C_3)$-Alkyl und $(C_1-C_3)$-Alkyloxy,
$(C_3-C_{10})$-Cycloalkyl-$(C_1-C_6)$-alkyl, wobei die Cyclo-alkylgruppe gegebenenfalls substituiert sein kann mit 1, 2 oder 3 Gruppe(n), unabhängig ausgewählt aus Halogen, $(C_1-C_3)$-Alkyl und $(C_1-C_3)$-Alkyloxy,
Aryl, das gegebenenfalls substituiert sein kann mit 1, 2 oder 3 Gruppe(n), unabhängig ausgewählt aus Halogen, $(C_1-C_3)$-Alkyl und $(C_1-C_3)$-Alkyloxy und
Aryl-$(C_1-C_6)$-alkyl, wobei die Arylgruppe gegebenenfalls substituiert sein kann mit 1, 2 oder 3 Gruppe(n), unabhängig ausgewählt aus Halogen, $(C_1-C_3)$-Alkyl und $(C_1-C_3)$-Alkyloxy.

**2.** Verbindung nach Anspruch 1, welche 5-Hydroxy-4-(2-methyl-1-propenyl)-5H-2-furanon ist, gemäß der Formel (**VI^A^**)

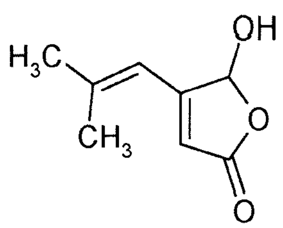

(**$VI^A$**)

.

**3.** Verbindung der Formel (**$VI^B$**)

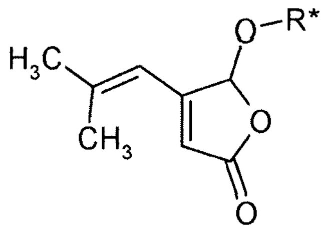

($VI^B$)

worin R* eine chirale ($C_5$-$C_{15}$)-Kohlenwasserstoffgruppe ist.

4. Verbindung nach Anspruch 3, worin R* ausgewählt ist aus (*R*) - oder (*S*)-$\alpha$-Methylbenzyl, (*R*) - oder (*S*)-1-(1-Naphthyl)ethyl, (*R*)- oder (*S*)-1-(2-Naphthyl)ethyl, Menthyl und Bornyl.

5. Verbindung nach Anspruch 1, worin R gleich $R^1$-C(O)- oder $R^2$-$SO_2$- ist und $R^1$ und $R^2$ chirale ($C_5$-$C_{15}$)-Kohlenwasserstoff-gruppen sind.

6. Verbindung der Formel (**IX**)

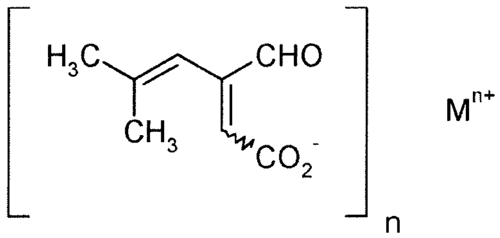

(**IX**)

worin:

n gleich 1 ist und $M^+$ ausgewählt ist aus $Li^+$, $Na^+$, $K^+$, $Rb^+$, $Nah_4^+$, (($C_1$-$C_3$)-Alkyl)-$NH_3^+$, (($C_1$-$C_3$)-Alkyl)$_2$-$NH_2^+$, (($C_1$-$C_3$)-Alkyl)$_3$$NH^+$und (($C_1$-$C_3$)-Alkyl)$_4$$N^+$; oder
n gleich 2 ist und $M^{2+}$ ausgewählt ist aus $Mg^{2+}$, $Ca^{2+}$ und $Zn^{2+}$.

7. Verbindung nach Anspruch 6, worin n gleich 1 ist und $M^+$ ausgewählt ist aus $NH_4^+$ und (($C_1$-$C_3$)-Alkyl)-$NH_3^+$.

8. Verbindung nach Anspruch 6, worin n gleich 1 ist und $M^+$ ausgewählt ist aus $Li^+$, $Na^+$ und $K^+$.

9. Verbindung der Formel (**VII**)

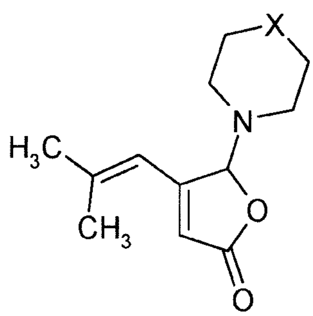

(**VII**)

worin -X- eine Einfachbindung, -$CH_2$-, -O-, -NH-, -N(($C_1$-$C_3$)-Alkyl)-, -N(Benzyl)- oder

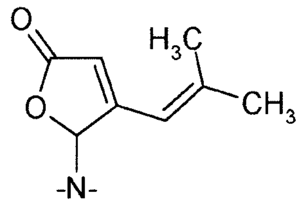

ist.

**10.** Verbindung nach Anspruch 9, ausgewählt aus:

4-(2-Methylpropenyl)-5-pyrrolidin-1-yl-5*H*-furan-2-on;
4-(2-Methylpropenyl)-5-piperidin-1-yl-5*H*-furan-2-on;
4-(2-Methylpropenyl)-5-morpholin-4-yl-5*H*-furan-2-on und
1,4-Bis-(4-(2-Methylpropenyl)-5H-furan-2-on-5-yl)piperazin.

**11.** Verfahren zur Herstellung der Verbindung der Formel (**VI^A^**) gemäß Anspruch 2, umfassend den Schritt der Behandlung einer Verbindung der Formel (**VII**)

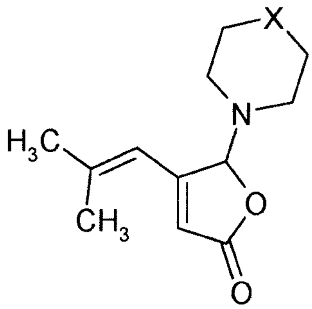

(**VII**)

worin -X- eine Einfachbindung, $-CH_2-$, -O-, -NH-, $-N((C_1-C_3)$-Alkyl)-, -N(Benzyl)- oder

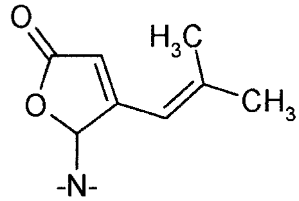

ist, mit Wasser in Gegenwart eines Säurekatalysators.

**12.** Verfahren zur Herstellung einer Verbindung der Formel (**VI**) gemäß Anspruch 1, worin R verschieden ist von Wasserstoff, $R^1$-C(O)- und $R^2-SO_2-$, umfassend den Schritt der Behandlung einer Verbindung der Formel (**$VI^A$**) mit einem Alkohol R-OH in Gegenwart eines Säurekatalysators.

**13.** Verfahren zur Herstellung einer Verbindung der Formel (**VI**) gemäß Anspruch 1, worin R verschieden ist von Wasserstoff, $R^1$-C(O) - und $R^2-SO_2^-$, umfassend den Schritt der Behandlung einer Verbindung der Formel (**VII**) mit einem Alkohol R-OH in Gegenwart von stöchiometrischer Säure.

**14.** Verfahren zur Herstellung einer Verbindung der Formel (**VI**) gemäß Anspruch 1, worin R $R^1$-C(O)- ist, umfassend die Schritte der Behandlung einer Verbindung der Formel (**$VI^A$**) mit einem Säurechlorid $R^1$-C(O)-Cl oder Säureanhydrid $(R^1-C(O))_2O$.

15. Verfahren zur Herstellung einer Verbindung der Formel (**VI**) gemäß Anspruch 1, worin R $R^2$-$SO_2$- ist, umfassend den Schritt der Behandlung einer Verbindung der Formel (**VI**$^A$) mit einem Sulfonylchlorid $R^2$-$SO_2$-Cl.

16. Verfahren zur Herstellung von 3-Aminomethyl-5-methylhexansäure (**II**)

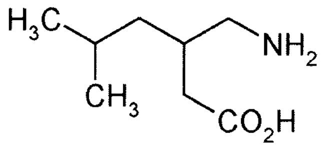

(**II**)

oder ein pharmazeutisch annehmbares Salz davon, umfassend die Schritte:

(a) Herstellen von 5-Hydroxy-4-(2-methyl-1-propenyl)-5*H*-2-furanon (**VI**$^A$)

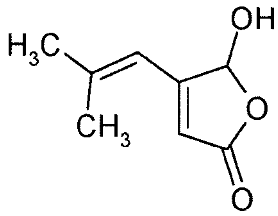

(**VI**$^A$)

(b) Überführen des 5-Hydroxy-4-(2-methyl-1-propenyl)-5*H*-2-furanons in 5-Hydroxy-4-(2-methylpropyl)-3,4-dihydro-5*H*-2-furanon (**I**$^A$)

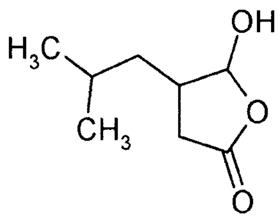

(**I**$^A$)

und

(c) Überführen des 5-Hydroxy-4-(2-methylpropyl)-3,4-dihydro-5*H*-2-furanons in 3-Aminomethyl-5-methylhexansäure (**II**).

17. Verfahren gemäß Anspruch 16, wobei die 3-Aminomethyl-5-methylhexansäure (**II**) (S)-3-Aminomethyl-5-methylhexansäure ((*S*)-**II**) ist

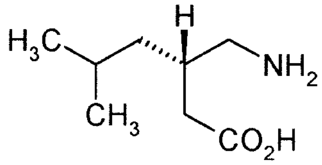

((*S*)-**II**)

wobei die (*S*)-3-Aminomethyl-5-methylhexansäure einen enantiomeren Überschuss von mindestens 80% aufweist.

**18.** Verfahren gemäß Anspruch 16 oder 17, wobei Schritt (a) ein Verfahren gemäß Anspruch 11 umfasst.

**19.** Verfahren gemäß einem der Ansprüche 16 bis 18, wobei Schritt (b) die Schritte umfasst:

(b1) Behandeln des 5-Hydroxy-4-(2-methyl-1-propenyl)-5*H*-2-furanons (**VI$^{A}$**) mit einem Metalloxid, -hydroxid, -carbonat oder -bicarbonat, Ammoniak, einem Mono-, Di-oder Tri- ($C_1$-$C_3$)-alkylamin, oder Tetra- ($C_1$-$C_3$)-alkylammoniumhydroxid, um ein Salz der Formel (**IX**) zu bilden

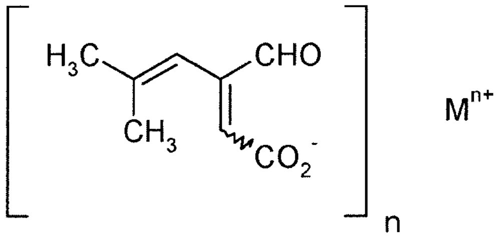

(**IX**)

worin

n gleich 1 ist und $M^+$ ausgewählt ist aus $Li^+$, $Na^+$, $K^+$, $Rb^+$, $Nah^+$, $((C_1\text{-}C_3)\text{-Alkyl})\text{-}NH_3^+$, $((C_1\text{-}C_3)\text{-Alkyl})_2\text{-}NH_2^+$, $((C_1\text{-}C_3)\text{-Alkyl})_3NH^+$und $((C_{1\text{-}3})\text{-Alkyl})_4N^+$; oder
n gleich 2 ist und $M^{2+}$ ausgewählt ist aus $Mg^{2+}$, $Ca^{2+}$ und $Zn^{2+}$ ;

(b2) Hydrieren des Salzes der Formel (**IX**), um ein Salz der Formel (**X**) zu erhalten

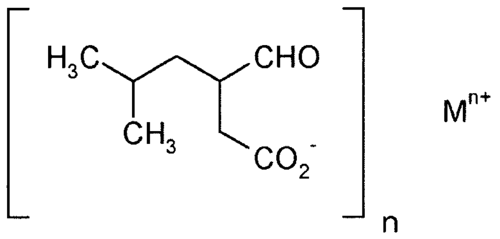

(**X**) ;

und
(b3) Behandeln des Salzes der Formel (X) mit einer Säure.

**20.** Verfahren nach einem der Ansprüche 16 bis 18, wobei der Schritt (b) die Schritte umfasst:

(b1) Überführen des 5-Hydroxy-4-(2-methyl-1-propenyl)-5*H*-2-furanons (**VI$^{A}$**) in eine Verbindung der Formel (**VI**), wie in Anspruch 3 definiert, worin R eine chirale ($C_5$-$C_{15}$)-Kohlenwasserstoffgruppe ist;
(b2) Hydrieren der Verbindung der Formel (**VI**), um eine Verbindung der Formel (**XI**) zu erhalten

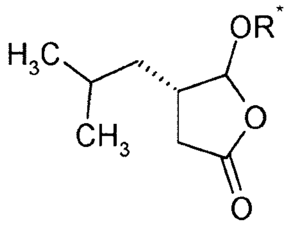

(**XI**) ;

worin R* eine chirale ($C_5$-$C_{15}$)-Kohlenwasserstoffgruppe ist; und
(b3) Behandeln der Verbindung der Formel (**XI**) mit einer Säure, um ((*S*)-**I^A^**) zu erhalten.

**21.** Verfahren zur Herstellung von 3-Aminomethyl-5-methylhexansäure (**II**)

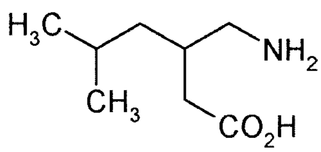

(**II**)

oder ein pharmazeutisch annehmbares Salz davon, umfassend die Schritte:

(a) Herstellen von 5-Hydroxy-4-(2-methyl-1-propenyl)-5*H*-2-furanon (**$VI^A$**)

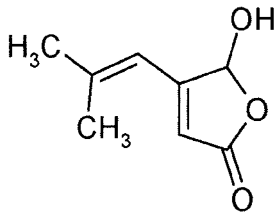

(**$VI^A$**)

(b) Behandeln des 5-Hydroxy-4-(2-methyl-1-propenyl)-5*H*-2-furanons (**$VI^A$**) mit Ammoniak oder einem Mono-($C_1$-$C_3$) - alkylamin, um ein Salz der Formel (**$IX^A$**) zu bilden

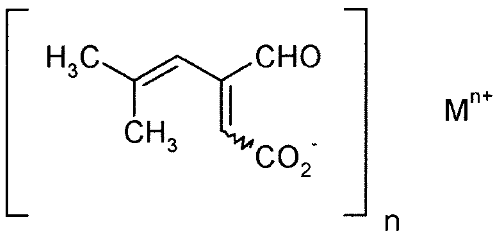

(**$IX^A$**)

worin:

n gleich 1 und $M^+$ ausgewählt ist aus $NH_4^+$ und (($C_1$-$C_3$)-Alkyl)-$NH_3^+$;

(c) Hydrieren des Salzes der Formel (**$IX^A$**), um ein Salz der Formel (**$X^A$**) zu erhalten

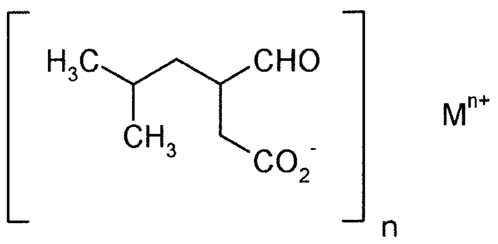

($X^A$)

;

und

(d) Behandeln des Salzes der Formel ($X^A$) mit einer Transaminase oder einem Aminoxidase/Iminreduktase-Enzym, um 3-Aminomethyl-5-methylhexansäure (**II**) zu erhalten.

**22.** Verfahren gemäß Anspruch 21, wobei die 3-Aminomethyl-5-methylhexansäure (**II**) (S)-3-Aminomethyl-5-methylhexansäure ((*S*)-**II**) ist

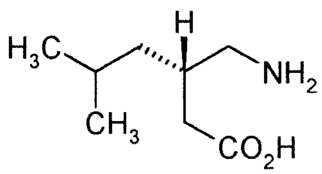

((*S*)-**II**)

wobei die (*S*)-3-Aminomethyl-5-methylhexansäure einen enantiomeren Überschuss von mindestens 80% aufweist.

**23.** Verfahren gemäß Anspruch 21 oder 22, wobei Schritt (a) ein Verfahren gemäß Anspruch 11 umfasst.

## Revendications

**1.** Composé selon la formule (**VI**)

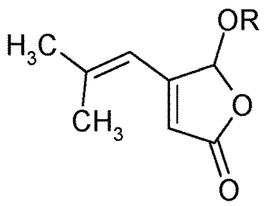

(**VI**)

dans lequel R est choisi parmi :

un atome d'hydrogène,
un groupe alkyle en $C_1$ à $C_6$,
halogénoalkyle en $C_1$ à $C_6$,
alcoxy en $C_1$ à $C_3$-alkyle en $C_2$ à $C_6$,
alcényle en $C_2$ à $C_6$,
cycloalkyle en $C_3$ à $C_{10}$, qui peut être optionnellement substitué par 1, 2 ou 3 groupes choisis indépendamment parmi les groupes halogéno, alkyle en $C_1$ à $C_3$ et alkyloxy en $C_1$ à $C_3$,
cycloalkyle en $C_3$ à $C_{10}$-alkyle en $C_1$ à $C_6$, dans lequel le groupe cycloalkyle peut être optionnellement substitué par 1, 2 ou 3 groupes choisis indépendamment parmi les groupes halogéno, alkyle en $C_1$ à $C_3$

et alkyloxy en $C_1$ à $C_3$,
aryle, qui peut être optionnellement substitué par 1, 2 ou 3 groupes choisis indépendamment parmi les groupes halogéno, alkyle en $C_1$ à $C_3$ et alkyloxy en $C_1$ à $C_3$,
arylalkyle en $C_1$ à $C_6$, dans lequel le groupe aryle peut être optionnellement substitué par 1, 2 ou 3 groupes choisis indépendamment parmi les groupes halogéno, alkyle en $C_1$ à $C_3$ et alkyloxy en $C_1$ à $C_3$,
$R^1$-C(O)-, et
$R^2$-$SO_2$- ;
$R^1$ est choisi parmi :

un atome d'hydrogène,

un groupe alkyle en $C_1$ à $C_6$,
halogénoalkyle en $C_1$ à $C_6$,
alcoxy en $C_1$ à $C_3$-alkyle en $C_2$ à $C_6$,
alcényle en $C_2$ à $C_6$,
cycloalkyle en $C_3$ à $C_{10}$, qui peut être optionnellement substitué par 1, 2 ou 3 groupes choisis indépendamment parmi les groupes halogéno, alkyle en $C_1$ à $C_3$ et alkyloxy en $C_1$ à $C_3$,
cycloalkyle en $C_3$ à $C_{10}$-alkyle en $C_1$ à $C_6$, dans lequel le groupe cycloalkyle peut être optionnellement substitué par 1, 2 ou 3 groupes choisis indépendamment parmi les groupes halogéno, alkyle en $C_1$ à $C_3$ et alkyloxy en $C_1$ à $C_3$,
aryle, qui peut être optionnellement substitué par 1, 2 ou 3 groupes choisis indépendamment parmi les groupes halogéno, alkyle en $C_1$ à $C_3$ et alkyloxy en $C_1$ à $C_3$, et
arylalkyle en $C_1$ à $C_6$, dans lequel le groupe aryle peut être optionnellement substitué par 1, 2 ou 3 groupes choisis indépendamment parmi les groupes halogéno, alkyle en $C_1$ à $C_3$ et alkyloxy en $C_1$ à $C_3$ ; et

$R^2$ est choisi parmi :

un groupe alkyle en $C_1$ à $C_6$,
halogénoalkyle en $C_1$ à $C_6$,
alcoxy en $C_1$ à $C_3$-alkyle en $C_2$ à $C_6$,
alcényle en $C_2$ à $C_6$,
cycloalkyle en $C_3$ à $C_{10}$, qui peut être optionnellement substitué par 1, 2 ou 3 groupes choisis indépendamment parmi les groupes halogéno, alkyle en $C_1$ à $C_3$ et alkyloxy en $C_1$ à $C_3$,
cycloalkyle en $C_3$ à $C_{10}$-alkyle en $C_1$ à $C_6$, dans lequel le groupe cycloalkyle peut être optionnellement substitué par 1, 2 ou 3 groupes choisis indépendamment parmi les groupes halogéno, alkyle en $C_1$ à $C_3$ et alkyloxy en $C_1$ à $C_3$,
aryle, qui peut être optionnellement substitué par 1, 2 ou 3 groupes choisis indépendamment parmi les groupes halogéno, alkyle en $C_1$ à $C_3$ et alkyloxy en $C_1$ à $C_3$, et
arylalkyle en $C_1$ à $C_6$, dans lequel le groupe aryle peut être optionnellement substitué par 1, 2 ou 3 groupes choisis indépendamment parmi les groupes halogéno, alkyle en $C_1$ à $C_3$ et alkyloxy en $C_1$ à $C_3$.

**2.** Composé selon la revendication 1 qui est la 5-hydroxy-4-(2-méthyl-1-propényl)-5*H*-2-furanone selon la formule (**VI$^A$**)

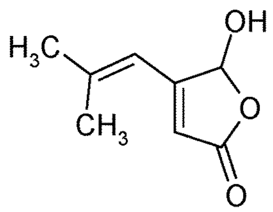

(**VI$^A$**)

**3.** Composé de formule (**VI$^B$**)

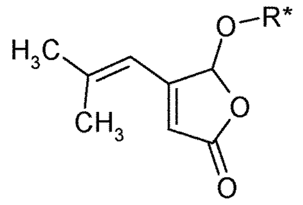

($VI^B$)

dans lequel R* représente un groupe hydrocarboné en $C_5$ à $C_{15}$ chiral.

**4.** Composé selon la revendication 3, dans lequel R* est choisi parmi les groupes (*R*)- ou (*S*)-α-méthyl-benzyle, (*R*) - ou (*S*)-1-(1-naphtyl)éthyle, (*R*)- ou (*S*)-1-(2-naphtyl)éthyle, menthyle et bornyle.

**5.** Composé selon la revendication 1, dans lequel R représente $R^1$-C(O)- ou $R^2$-$SO_2$- et $R^1$ et $R^2$ représentent des groupes hydrocarbonés en $C_5$ à $C_{15}$ chiraux.

**6.** Composé de formule (**IX**)

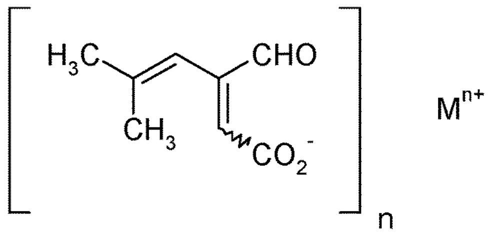

(**IX**)

dans lequel :

n vaut 1 et $M^+$ est choisi parmi $Li^+$, $Na^+$, $K^+$, $Rb^+$, $NH_4^+$, (alkyle en $C_1$ à $C_3$)$NH_3^+$, (alkyle en $C_1$ à $C_3$)$_2NH_2^+$, (alkyle en $C_1$ à $C_3$)$_3NH^+$ et (alkyle en $C_1$ à $C_3$)$_4N^+$ ; ou
n vaut 2 et $M^{2+}$ est choisi parmi $Mg^{2+}$, $Ca^{2+}$ et $Zn^{2+}$.

**7.** Composé selon la revendication 6 dans lequel n vaut 1 et $M^+$ est choisi parmi $NH_4^+$ et (alkyle en $C_1$ à $C_3$)$NH_3^+$.

**8.** Composé selon la revendication 6 dans lequel n vaut 1 et $M^+$ est choisi parmi $Li^+$, $Na^+$ et $K^+$.

**9.** Composé de formule (**VII**)

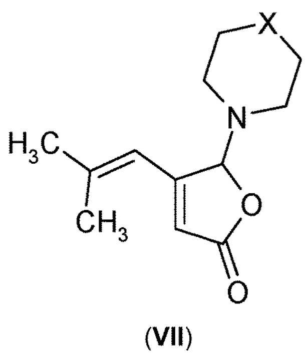

(**VII**)

dans lequel -X- représente une liaison simple, -$CH_2$-, -O- ; -NH-, -N (alkyle en $C_1$ à $C_3$)-, -N(benzyl)-, ou

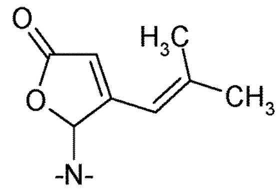

.

10. Composé selon la revendication 9 choisi parmi :

la 4-(2-méthylpropényl)-5-pyrrolidin-1-yl-5*H*-furan-2-one ;
la 4-(2-méthylpropényl)-5-pipéridin-1-yl-5*H*-furan-2-one ;
la 4-(2-méthylpropényl)-5-morpholin-4-yl-5H-furan-2-one ; et
la 1,4-bis-(4-(2-méthylpropényl)-5*H*-furan-2-on-5-yl)pipérazine.

11. Procédé de fabrication du composé de formule (**VI^A^**) selon la revendication 2 comprenant l'étape de traitement d'un composé de formule (**VII**)

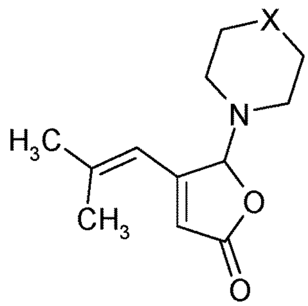

(**VII**)

dans lequel -X- représente une liaison simple, $-CH_2-$, -O-, -NH-, -N (alkyle en $C_1$ à $C_3$)-, -N(benzyl)-,

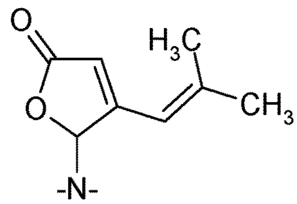

avec de l'eau en présence d'un catalyseur acide.

12. Procédé de fabrication d'un composé de formule (**VI**) selon la revendication 1 dans lequel R est autre qu'un atome d'hydrogène, $R^1$-C(O)- et $R^2$-$SO_2$-, comprenant l'étape de traitement d'un composé de formule (**VI$^A$**) avec un alcool R-OH en présence d'un catalyseur acide.

13. Procédé de fabrication d'un composé de formule (**VI**) selon la revendication 1 dans lequel R est autre qu'un atome d'hydrogène, $R^1$-C(O)- et $R^2$-$SO_2$-, comprenant l'étape de traitement d'un composé de formule (**VII**) avec un alcool R-OH en présence d'acide stoechiométrique.

14. Procédé de fabrication d'un composé de formule (**VI**) selon la revendication 1 dans lequel R représente $R^1$-C(O)-, comprenant les étapes de traitement d'un composé de formule (**VI$^A$**) avec un chlorure d'acide $R^1$-C(O)-Cl ou un anhydride d'acide $(R^1\text{-C(O)})_2O$.

15. Procédé de fabrication d'un composé de formule (**VI**) selon la revendication 1 dans lequel R représente $R^2$-$SO_2$- ; comprenant l'étape de traitement d'un composé de formule (**VI$^A$**) avec un chlorure de sulfonyle $R^2$-$SO_2$-Cl.

**16.** Procédé de fabrication de l'acide 3-amino-méthyl-5-méthylhexanoïque (**II**)

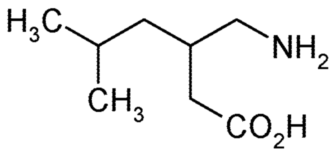

(**II**)

ou de l'un de ses sels pharmaceutiquement acceptables, comprenant les étapes suivantes :

(a) la préparation de 5-hydroxy-4-(2-méthyl-1-propényl)-5H-2-furanone (**VI^A^**)

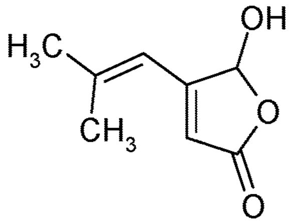

(**VI**A)

(b) la conversion de ladite 5-hydroxy-4-(2-méthyl-1-propényl)-5*H*-2-furanone en 5-hydroxy-4-(2-méthyl-propyl)-3,4-dihydro-5*H*-2-furanone (**I**A)

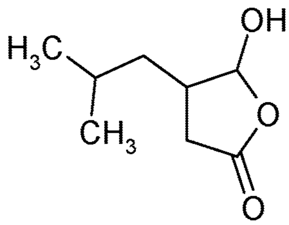

(**I**A)

et

(c) la conversion de ladite 5-hydroxy-4-(2-méthyl-propyl)-3,4-dihydro-5*H*-2-furanone en acide 3-amino-méthyl-5-méthylhexanoïque (**II**).

**17.** Procédé selon la revendication 16 dans lequel l'acide 3-aminométhyl-5-méthylhexanoïque (**II**) est l'acide (*S*)-3-aminométhyl-5-méthylhexanoïque ((*S*)-**II**)

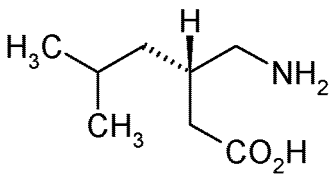

((*S*)-**II**)

dans lequel ledit acide (*S*)-3-aminométhyl-5-méthylhexanoïque est en excès énantiomérique d'au moins 80 %.

**18.** Procédé selon la revendication 16 ou 17 dans lequel l'étape (a) comprend un procédé selon la revendication 11.

**19.** Procédé selon l'une quelconque des revendications 16 à 18 dans lequel l'étape (b) comprend les étapes suivantes :

(b1) le traitement de la 5-hydroxy-4-(2-méthyl-1-propényl)-5*H*-2-furanone (**VI$^{A}$**) avec un oxyde, un hydroxyde, un carbonate ou un bicarbonate de métal, de l'ammoniac, une mono-, di- ou tri(alkyle en $C_1$ à $C_3$)amine, ou un hydroxyde de tétra-alkyle en $C_1$ à $C_3$-ammonium pour former un sel de formule (**IX**)

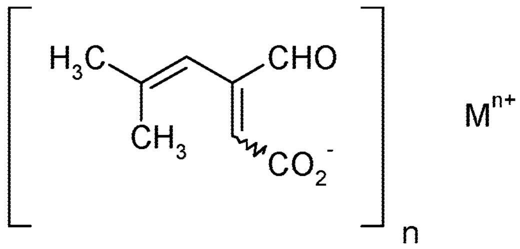

(**IX**)

dans lequel :

n vaut 1 et $M^+$ est choisi parmi $Li^+$, $Na^+$, $K^+$, $Rb^+$, $NH_4^+$, (alkyle en $C_1$ à $C_3$) $N_{H}^{3}+$, (alkyle en $C_1$ à $C_3$)$_2NH_2^+$, (alkyle en $C_1$ à $C_3$)$_3NH^+$ et (alkyle en $C_1$ à $C_3$)$_4N^+$ ; ou
n vaut 2 et $M^{2+}$ est choisi parmi $Mg^{2+}$, $Ca^{2+}$ et $Zn^{2+}$;

(b2) l'hydrogénation du sel de formule (**IX**) pour obtenir un sel de formule (**X**)

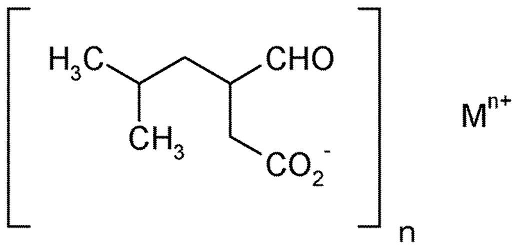

(**X**) ;

et
(b3) le traitement du sel de formule (**X**) avec un acide.

**20.** Procédé selon l'une quelconque des revendications 16 à 18 dans lequel l'étape (b) comprend les étapes suivantes :

(b1) la conversion de la 5-hydroxy-4-(2-méthyl-1-propényl)-5*H*-2-furanone (**VI$^{A}$**) en un composé de formule (**VI**) tel que défini dans la revendication 3 dans lequel R représente un groupe hydrocarboné en $C_5$ à $C_{15}$ chiral ;
(b2) l'hydrogénation du composé de formule (**VI**) pour obtenir un composé de formule (**XI**)

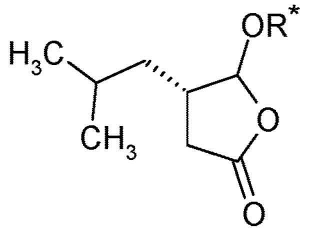

(**XI**)

dans lequel R* représente un groupe hydrocarboné en $C_5$ à $C_{15}$ chiral ; et
(b3) le traitement du composé de formule (**XI**) avec un acide pour donner ((*S*)-**I$^{A}$**).

**21.** Procédé de fabrication de l'acide 3-amino-méthyl-5-méthylhexanoïque (**II**)

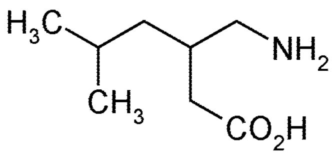

(**II**)

ou de l'un de ses sels pharmaceutiquement acceptables, comprenant les étapes suivantes :

(a) la préparation de la 5-hydroxy-4-(2-méthyl-1-propényl)-5*H*-2-furanone (**VI$^A$**)

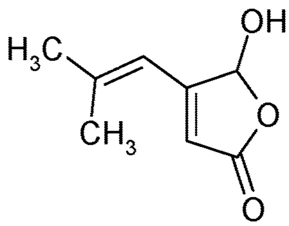

(**VI$^A$**)

(b) le traitement de la 5-hydroxy-4-(2-méthyl-1-propényl)-5*H*-2-furanone (**VI$^A$**) avec de l'ammoniac ou une mono-(alkyle en $C_1$ à $C_3$)amine pour former un sel de formule (**IX$^A$**)

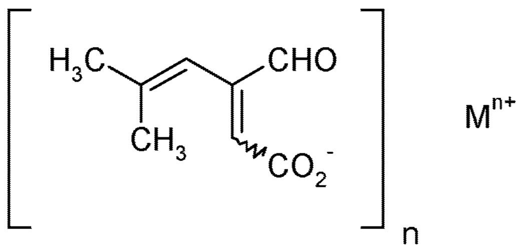

(**IX$^A$**)

dans lequel :

n vaut 1 et $M^+$ est choisi parmi $NH_4^+$ et (alkyle en $C_1$ à $C_3$)$NH_3^+$ ;

(c) l'hydrogénation du sel de formule (**IX$^A$**) pour obtenir un sel de formule (**X$^A$**)

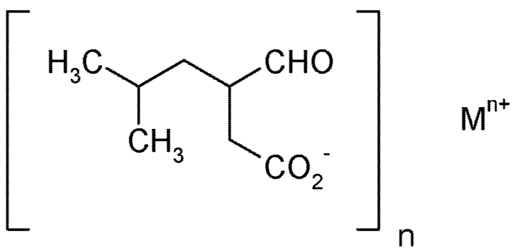

(**X$^A$**) ;

et

(d) le traitement du sel de formule (**X$^A$**) avec une transaminase ou une enzyme amine oxydase/imine réductase pour fournir l'acide 3-aminométhyl-5-méthyl-hexanoïque (**II**).

**22.** Procédé selon la revendication 21 dans lequel l'acide 3-aminométhyl-5-méthylhexanoïque (II) est l'acide (*S*)-3-aminométhyl-5-méthylhexanoïque ((*S*)-**II**)

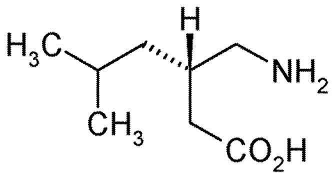

((*S*)-**II**)

dans lequel ledit acide (*S*)-3-aminométhyl-5-méthylhexanoïque est en excès énantiomérique d'au moins 80 %.

**23.** Procédé selon la revendication 21 ou 22 dans lequel l'étape (a) comprend un procédé selon la revendication 11.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5563175 A
- US 6001876 A
- WO 03093220 A
- US 2008004699 W
- WO 2008127646 A2
- IN 2010000140 W
- WO 2011086565 A
- GB 834100 A


### Non-patent literature cited in the description


- **KIENZLE, F. et al.** *Helv. Chim. Acta,* 1985, vol. 68 (5), 1133-39